# NEW EUROPEAN PATENT SPECIFICATION

(11) **EP 0 503 785 B3**
(45) Date of publication of this specification: **29.02.2012**
(45) Mention of the grant of the patent: 25.04.2001
(21) Application number: 92301449.2
(22) Date of filing: 21.02.1992
(51) Int. Cl.: C07D 233/90, A61K 31/415, C07D 405/12, C07D 405/14, C07D 403/10, C07F 7/18

(54) **1-Biphenylimidazole derivatives, their preparation and their therapeutic use**
1-Biphenylimidazolderivate, Verfahren zu deren Herstellung und deren therapeutische Anwendung
Dérivés de 1-biphénylimidazole, leur préparation et leur utilisation thérapeutique

(30) Priority: 21.02.1991 JP 2709891; 26.04.1991 JP 9658891; 06.06.1991 JP 13488991; 08.07.1991 JP 16713891; 24.07.1991 JP 18484191; 15.07.1991 JP 17397291
(43) Date of publication of application: 16.09.1992
(60) Divisional application: 93200195.1
(73) Proprietor: Daiichi Sankyo Company, Limited, Tokyo 103-8426 (JP)
(72) Inventor: Yanagisawa, Hiroaki, Shinagawa-ku Tokyo 140 (JP); Shimoji, Yasuo, Shinagawa-ku Tokyo 140 (JP); Fujimoto, Koichi, Shinagawa-ku Tokyo 140 (JP); Kanazaki, Takuro, Shinagawa-ku Tokyo 140 (JP); Anemiya, Yoshiya, Shinagawa-ku Tokyo 140 (JP); Koike, Hiroyuki, Shinagawa-ku Tokyo 140 (JP); Sada, Toshio, Shinagawa-ku Tokyo 140 (JP)
(74) Representative: Gibson, Christian John Robert

(56) References cited:
- EP-A- 0 323 841
- EP-A- 0 401 030
- EP-A1- 0 028 834
- EP-A2- 0 028 833
- EP-A2- 0 253 310
- EP-A2- 0 324 377
- WO-A-91/12002
- WO-A-91/14367
- J. MED. CEHM. vol. 34, 1991, WASHINGTON, page 2410
- J. MED. CHEM. vol. 33, 1990, WASHINGTON, page 1330
- J. MED. CHEM. vol. 34, 1991, WASHINGTON, page 2525

## Description

The present invention provides a series of novel 1-(biphenylmethyl)imidazole derivatives which have valuable hypotensive activities, and which may, therefore, be used in the treatment and prophylaxis of hypertension, including diseases of the heart and circulatory system. We also provide methods and compositions using these compounds, as well as processes for their preparation.

It is known that the renin-angiotension system provides one of the important mechanisms for maintaining the homeostasis of blood pressure in living animals. When blood pressure is reduced or the sodium ion concentration of the body fluids falls, this system is activated. As a result, the enzyme renin and angiotensin converting enzyme (hereinafter abbreviated, as is conventional, to "ACE") are activated and act on angiotensinogen, which is first decomposed by the renin to produce angiotensin I (hereinafter abbreviated to "Al"). This Al is then converted by ACE to angiotensin II (hereinafter abbreviated to "All"). Since All induces strong contractions of blood vessels and accelerates the secretion of aldosterone, the activation of the system results in an elevation of blood pressure. Inhibitors or suppressors of the renin-angiotension system, such as renin inhbitors, ACE inhibitors and All antagonists, dilate blood vessels, cause lower blood pressure and improve the circulatory function, which is the basis for the use of these agents in the treatment of heart diseases.

At present only ACE inhibitors are used clinically, although renin inhibitors and All antagonists are under investigation for such use. Of these, some peptide type All antagonists, such as saralasin, have been known for many years, whilst certain non-peptide type antagonists have recently been discovered (for example, those compounds disclosed in European Patent Publications No. 28 833, 28 834, 245 637, 253 310, 323 841, 324 377, 380 959, 399 732, 399 731, 400 835 and 401030, in J. Med.Chem. 1990, 33, 1330-1336, J.Med.Chem. 1991, 34, 2410-2414 and J. Med.Chem. 1991,34,2525-2547, in international Patent Publications WO 91/12002 and WO 91/14367 and in Japanese Patent Application Kokai No. Sho 57-98270). Of these, the closest prior art is believed to be European Patent Publications No. 253 310 and 324 377.

European Patent Publication No. 253 310 discloses a series of 1-phenol-, 1-phenethyl-or 1-benzyl-imidazole derivatives which are said to have the ability to inhibit the activity of All. Included in the scope of these prior art compounds are a number of 1-biphenyl-methylimidazole derivatives, which, however, differ from the compounds of the present invention in the nature of the substituent at the 4- or 5- position of the imidazole ring.

European Patent Publication No. 324 377 also discloses a series of such compounds. The activities of all of these prior art compounds, however, including those of European Patent Publications No. 253 310 and 324 377, are not thought to be sufficient and more potent All antagonists are sought for better clinical results.

We have now discovered a limited series of 1-(biphenylmethyl)imidazole-5-carboxylic acid derivatives having an excellent All receptor antagonist activity, and which are therefore useful as anti-hypertensive drugs and for the therapy and prophylaxis of heart diseases.

Thus, the present invention provides a compound of formula (I): 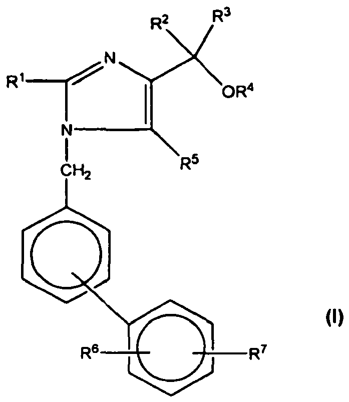 in which:
R¹ represents a propyl group;
R² and R³ each represent a methyl group;
R⁴ represents a hydrogen atom;
R⁵ represents a carboxy group;
R⁶ represents a hydrogen atom;
R⁷ represents a tetrazol-5-yl group at the 2- position of the benzene ring; and
the benzene ring which bears the substituents represented by R⁶ and R⁷ is at the 4-position of the benzyl group to which it is attached;
and pharmaceutically acceptable salts and esters thereof.

The present application also describes compounds of formula (I) : 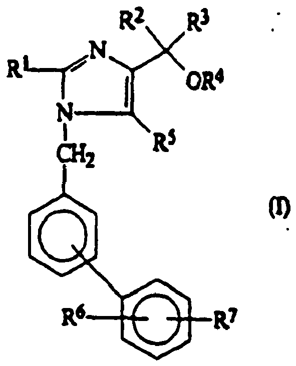 in which:
R¹ represents an alkyl group having from 2 to 5 carbon atoms or an alkenyl group having from 3 to 5 carbon atoms;
R² and R³ are the same or different and each represents an alkyl group having from 1 to 4 carbon atoms;
R⁴ represents:
   a hydrogen atom,
   a methyl or ethyl group,
   an alkanoyl group having from 1 to 5 carbon atoms,
   an alkenoyl group having from 3 to 5 carbon atoms,
   a benzoyl group, or
   an alkoxycarbonyl group having from 2 to 5 carbon atoms;
R⁵ represents a group of formula -COOR^{5a} or a group of formula -CONR⁸R⁹, in which:
R^{5a} represents
   a hydrogen atom,
   an alkyl group having from 1 to 4 carbon atoms,
   a benzyl group,
   an alkanoyloxyalkyl group, in which the alkanoyl part has from 1 to 5 carbon atoms, and the alkyl part is a methyl or ethyl group,
   a cycloalkanoyloxyalkyl group, in which the cycloalkanoyl part has 6 or 7 carbon atoms, and the alkyl part is a methyl or ethyl group,
   an alkoxycarbonyloxyalkyl group, in which the alkoxy part has from 1 to 4 carbon atoms, and the alkyl part is a methyl or ethyl group,
   a cycloalkoxycarbonyloxyalkyl group, in which the cycloalkoxy part has 5 or 6 carbon atoms, and the alkyl part is a methyl or ethyl group,
   a [5-(phenyl-, methyl-or ethyl-)-2-oxo-1,3- dioxolen-4-yl]methyl group, or
   a phthalidyl group;
R⁸ and R⁹ are the same or different and each represents:
   a hydrogen atom,
   a methyl group,
   an ethyl group, or
   a substituted methyl or ethyl group which is substituted by at least one substituent selected from carboxy groups, methoxycarbonyl groups and
   ethoxycarbonyl groups;
   or R⁸ and R⁹ together represent an unsubstituted alkylene group which has 4 or 5 carbon atoms or a substituted alkylene group which has 4 or 5 carbon atoms and which is substituted by at least one substituent selected from carboxy groups, methoxycarbonyl groups and ethoxycarbonyl groups;
   R⁶ represents a hydrogen atom, or it represents a methyl group, an ethyl group; a methoxy group, an ethoxy group, a fluorine atom or a chlorine atom on the 6-position of the benzene ring;
   R⁷ represents a carboxy group or a tetrazol-5-yl group at the 2- or 3- position of the benzene ring; and
the benzene ring which bears the substituents represented by R⁶ and R⁷ is at the 4-position of the benzyl group to which it is attached;
and pharmaceutically acceptable salts and esters thereof.

The invention also provides a pharmaceutical composition for the treatment or prophylaxis of hypertension, which comprises an effective amount of an anti-hypertensive agent in admixture with a pharmaceutically acceptable carrier or diluent, in which the anti-hypertensive agent is at least one compound of formula (I) or a pharmaceutically acceptable salt or ester thereof.

The invention further provides compounds of formula (I) and pharmaceutically acceptable-salts and esters thereof for use in a method of therapy, and still further provides the use of compounds of formula (I) and pharmaceutically acceptable salts and esters thereof for the manufacture of a medicament for the treatment or prophylaxis of hypertension in a mammal, e.g. a human being.

The invention still further provides processes for the preparation of compounds of formula (I) and pharmaceutically acceptable salts and esters thereof, which are described in more detail hereafter.

In the compounds of the present invention, where R² or R³ is an alkyl group, this is an alkyl group having from 1 to 4 carbon atoms, and may be a straight or branched chain group having from 1 to 4 carbon atoms while where R¹ is an alkyl group, this is an alkyl group having from 2 to 5 carbon atoms, and may be a straight or branched chain group; examples include the methyl, ethyl, propyl, isopropyl, butyl, isobutyl, sec-butyl, t-butyl, pentyl, t-pentyl, 2-methylbutyl, 3-methylbutyl and 1-ethylpropyl groups. A¹ more preferably represents a.straight chain group, i.e. most preferably an ethyl, propyl orbutyl group. Each of R² and R³, which may be the same or different, more preferably represents a methyl, ethyl, propyl, isopropyl or t-butyl group, and most preferably a methyl or ethyl group when R⁵ represents a carboxy group, or an isopropyl or t-butyl group when R⁵ represents a group of formula -CONR⁸R⁹.

Where R¹ represents an alkenyl group, this may be a straight or branched chain alkenyl group containing from 3 to 5 carbon atoms. Examples of such groups include: the 1-propenyl, 2-propenyl, 1-methyl-2-propenyl, 2-methyl-1-propenyl, 2-methyl-2-propenyl, 2-ethyl-2-propenyl, 1-butenyl, 2-butenyl, 1-methyl-2-butenyl, 2-methyl-2-butenyl, 3-methyl-2-butenyl, 3-butenyl, 1-methyl-3-butenyl, 2-methyl-3-butenyl, 1-pentenyl, 2-pentenyl, 3-pentenyl and 4-pentenyl groups. R¹ preferably represents a straight or branched chain alkenyl group containing 3 or 4 carbon atoms, and more preferably a 1-propenyl or 1-butenyl group.

R⁴ can represent an alkanoyl group; such a group may be a straight or branched chain group and has from 1 to 5 carbon atoms. Examples of such groups include the formyl, acetyl, propionyl, butyryl, isobutyryl, pivaloyl, valeryl and isovaleryl groups, of which the formyl and acetyl groups are preferred.

Where R⁴ represents an alkenoyl group, this may have from 3 to 5 carbon atoms, and examples include the acryloyl, methacryloyl, crotonoyl, 3-methyl-2-butenoyl and 2-methyl-2-butenoyl, especially (E)-2-methyl-2-butenoyl, groups.

Where R⁴ represents an alkoxycarbonyl group, the alkoxy part has from 1 to 4 carbon atoms, i.e. the group as a whole has from 2 to 5 carbon atoms, and examples of such groups include the methoxycarbonyl, ethoxycarbonyl, propoxycarbonyl, isopropoxycarbonyl, butoxycarbonyl, isobutoxycarbonyl, t-butoxycarbonyl and pentyloxycarbonyl groups, of which the methoxycarbonyl and ethoxycarbonyl groups are preferred.

R⁵ represents a group of formula -COOR^{5a} or a group of formula -CONR⁸R⁹. Where it represents a group of formula - CONR⁸R⁹, and R⁸ or R⁹ represents an alkyl group, this may be an unsubstituted methyl or ethyl group or a substituted methyl or ethyl group, which is substituted by at least one substituent selected from carboxy groups, methoxycarbonyl groups and ethoxycarbonyl groups.

Where R⁸ and R⁹ together represent an alkylene group, this has from 4 to 5 carbon atoms and may be substituted or unsubstituted; it may also be a straight or branched chain group. Examples of the unsubstituted groups include the ethylethylene, tetramethylene and pentamethylene groups. In such cases, the group of formula -NR⁸R⁹ is a nitrogen-containing heterocyclic group having 5 or 6 ring atoms (one being the nitrogen atom), for example, it is a 1-pyrrolidinyl or piperidino group, respectively. Where the group is substituted, there may be one or more substituents selected from carboxy, methoxycarbonyl and ethoxycarbonyl groups.

Where R⁵ represents a carboxy group (i.e. R^{5a} represents a hydrogen atom), the compound is a carboxylic acid and can, therefore, form esters, in which the carboxy group is replaced by a group of formula -COOR^{5a}, in which R^{5a} represents an ester residue as defined above (i.e. R^{5a} represents a group other than a hydrogen atom). It can also form salts, examples of which are as exemplified below in relation to R⁷.
The benzene ring which bears the substituents represented by R⁶ and R⁷ is at the 4- position of the benzyl group to which it attaches, i.e. the compounds have the formula (Ia):

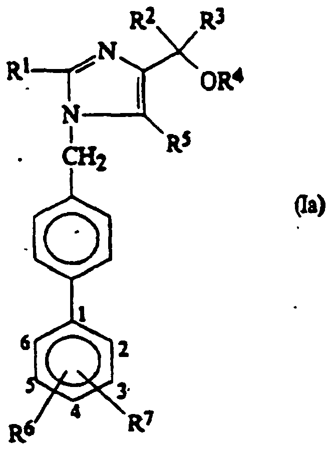

R⁶ represents a hydrogen atom, or a methyl, ethyl, methoxy or ethoxy group or fluorine or chlorine atom at the 6-position of the benzene ring.

R⁷ may represent a carboxy group or a tetrazol-5-yl group. When it represents a carboxy group, the resulting compounds may form salts or esters. There is no particular restriction on the nature of these salts or esters, provided that, where they are intended for therapeutic use, they are pharmaceutically acceptable i.e. it should not have increased, or unacceptably increased, toxicity or reduced, or unacceptably reduced, activity, as compared with the parent acid. Where they are intended for non-therapeutic uses, e.g. as intermediates in the preparation of other, and possibly more active, compounds, even this restriction does not apply. Examples of such salts include: salts with an alkali metal, such as sodium, potassium or lithium; salts with an alkaline earth metal, such as barium or calcium; salts with another metal, such as magnesium and aluminium; organic base salts, such as a salt with guanidine, triethylamine, dicyclohexylamine; and salts with a basic amino acid, such as lysine or arginine. Examples of ester groups may be as follows. It is well known in the art that certain ester residues confer advantages on compounds incorporating them, for example easier or better absorption in vivo, and, if desired, such ester residues may be used in the present invention.

Examples of such ester residues include:
alkyl groups having from 1 to 6 carbon atoms, such as those exemplified above in relation to R¹ and 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups.
haloalkyl groups having from 1 to 6, preferably from 1 to 4, carbon atoms, in which the alkyl part may be as exemplified-above in relation to R¹, for example the trifluoromethyl, 2,2,2-trichloroethyl, 2,2,2-trifluoroethyl, 2-chloroethyl, 2-fluoroethyl, 2-iodoethyl, 4-fluorobutyl, 3-chloropropyl and 6-iodohexyl groups, of which the 2,2,2-trichloroethyl and 2-chloroethyl groups are preferred;
hydroxyalkyl groups having from 1 to 6, preferably from 1 to 4, carbon atoms, in which the alkyl part may be as exemplified above in relation to the above alkyl groups having from 1 to 6 alkyl groups, for example the 2-hydroxyethyl, 2,3-dihydroxypropyl, 3-hydroxypropyl, 3,4-dihydroxybutyl and 4-hydroxybutyl groups, of which the 2-hydroxyethyl group is preferred;
alkoxyalkyl and alkoxyalkoxyalkyl groups in which the alkoxy and the alkyl parts each have from 1 to 6, preferably from 1 to 4, carbon atoms, and may be as exemplified in relation to substituents (b) below and the above alkyl groups having from 1 to 6 alkyl groups, respectively, for example the methoxymethyl, 2-methoxyethyl, 2-ethoxyethyl and 2-methoxyethoxymethyl groups, of which the methoxymethyl group is preferred;
phenacyl groups and phenacyl groups which are substituted by one or more of substituents (b), as defined and exemplified below, of which the unsubstituted phenacyl group is preferred;
said substituents (b) are selected from nitro groups, cyano groups, halogen atoms, unsubstituted carbocyclic aryl groups having from 6 to 10 ring atoms, alkyl groups having from 1 to 6 carbon atoms, alkoxy groups having from 1 to 6 carbon atoms, carboxy groups, alkoxycarbonyl groups in which the alkoxy part has from 1 to 6 carbon atoms and alkylenedioxy and alkylidenedioxy groups having from 1 to 3 carbon atoms;
alkoxycarbonylalkyl groups, such as the methoxycarbonylmethyl group;
cyanoalkyl groups having from 1 to 6, preferably from 1 to 4, carbon atoms, in which the alkyl part may be as exemplified above in relation to the alkyl groups having from 1 to 6 carbon atoms, for example the 2-cyanoethyl and cyanomethyl groups;
alkylthioalkyl groups in which each alkyl part has from 1 to 6, preferably from 1 to 4, carbon atoms, and may be as exemplified above in relation to the above alkyl groups having from 1 to 6 carbon atoms, for example the methylthiomethyl and ethylthiomethyl;
arylthioalkyl groups in which the alkyl part has from 1 to 6, preferably from 1 to 4, carbon atoms, and may be as exemplified above in relation to the alkyl groups having from 1 to 6 carbon atoms, and the aryl is an aromatic carbocylic group which has from 6 to 14 (preferably from 6 or 10, and more preferably 6 or 10) ring atoms and which is unsubstituted or is substituted by least one of substituents (b), defined and exemplified above in relation to phenacyl groups; examples of the aryl groups include the phenyl, naphthyl, phenanthrenyl, anthracenyl, 2-methylphenyl, 3-methylphenyl, 4-methyl-phenyl, 2-ethylphenyl, 3-propylphenyl, 4-ethylphenyl, 2-butylphenyl, 3-pentylphenyl, 4-pentylphenyl, 3,5-dimethylphenyl, 2,5-dimethylphenyl, 2,6-dimethyl-phenyl, 2,4-dimethylphenyl, 3,5-dibutylphenyl, 2,5-dipentylphenyl, 2,6-dipropyl-4-methylphenyl, 2-methoxyphenyl, 3-methoxyphenyl, 4-methoxyphenyl, 2-ethoxyphenyl, 3-propoxyphenyl, 4-ethoxyphenyl, 2-butoxyphenyl, 3-pentyloxyphenyl and 4-pentyloxyphenyl groups, of which the phenyl, 2-methylphenyl, 3-methylphenyl, 4-methylphenyl, 2-methoxyphenyl, 3-methoxyphenyl and 4-methoxyphenyl groups are the most preferred. An example of the arylthioalkyl groups is the phenylthiomethyl group;
alkylsulphonylalkyl groups in which each alkyl part has from 1 to 6, preferably from 1 to 4, carbon atoms, and may be as exemplified above in relation to the alkyl groups having from 1 to 6 carbon atoms and may be unsubstituted or substituted by one or more halogen atoms, for example the 2-(methanesulphonyl)ethyl or 2-(trifluoromethanesulphonyl)ethyl groups;
arylsulphonylalkyl groups in which the alkyl part has from 1 to 6, preferably from 1 to 4, carbon atoms, and may be as exemplified above in relation to the alkyl groups having from 1 to 6 carbon atoms, and the aryl part may be as defined and exemplified above in relation to arylthioalkyl, for example the 2-(benzenesulphonyl)ethyl and 2-(p-toluenesulphonyl)ethyl groups;
aryl groups such as those exemplified above in relation to arylthioalkyl groups;
aralkyl groups in which the alkyl part has from 1 to 6 (more preferably from 1 to 4, still more preferably 1 or 2, and most preferably 1) carbon atoms and the aryl part is an aromatic carbocyclic group which has from 6 to 14 (preferably from 6 to 10, and more preferably 6 or 10) ring atoms and which is unsubstituted or is substituted by at least one of substituents (b), defined and exemplified above. Specific examples of alkyl groups which may form the alkyl part are as given above in relation to the above alkyl groups having from 1 to 6 carbon atoms, and specific examples of the aryl groups which may form the aryl part are as given above in relation to the arylthio groups. Examples of such aralkyl groups include the benzyl, 1- and 2-naphthylmethyl, indenylmethyl, phenanthrenylmethyl, anthracenylmethyl, diphenylmethyl, triphenylmethyl, 1-phenylethyl, phenethyl, 1-naphthylethyl, 2-naphthylethyl, 1-phenylpropyl; 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthyl-butyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl and 6-naphthylhexyl groups. In those cases where the aralkyl group contains a naphthyl group, this may be a 1- or 2-naphthyl group. Of these aralkyl groups-, we prefer those groups in which the alkyl part has from 1 to 4 carbon atoms, the benzyl group being most preferred. These groups may be unsubstituted or they may be substituted by one or more of substituents (b), defined and exemplified above. Examples of the substituted groups include those unsubstituted groups exemplified above but in which the aryl part is replaced by one of the substituted aryl groups given above. However, the unsubstituted groups are preferred;
groups of formula -SiR^{d}R^{e}R^{f} (in which 1, 2 or 3 of the groups represented by R^{d}, R^{e} and R^{f} are the same or different and each represents an alkyl group having from 1 to 6 carbon atoms, and 2, 1 or 0 of the groups represented by R^{d}, R^{e} and R^{f} are the same or different and each represents an aryl group, as defined above, the alkyl and aryl parts may be any of those groups exemplified above in relation to the alkyl groups having from 1 to 6 carbon atoms and arylthioalkyl, preferably the methyl, ethyl, t-butyl and phenyl groups. Examples of such silyl groups include the trimethylsilyl, triethylsilyl, isopropyldimethylsityl, t-butyldimethylsilyl, methyldiisopropylsilyl, methyldi-t-butylsilyl, triisopropylsilyl, diphenylmethylsilyl, diphenylbutylsilyl, diphenylisopropylsilyl and phenyldiisopropylsilyl groups, of which the trimethylsilyl, t-butyldimethylsilyl and diphenylmethylsilyl groups are preferred;
alkanoyloxyalkyl groups in which each of the alkanoyl and the alkyl parts has from 1 to 6 carbon atoms and may be as exemplified above in relation to the alkyl groups having from 1 to 6 carbon atoms and R⁴, respectively, and preferably the alkanoyl part has from 1 to 5 carbon atoms and the alkyl part has from 1 to 4 carbon atoms and more preferably the alkanoyl part has from 2 to 5 carbon atoms and the alkyl part has 1 or 2 carbon atoms; examples of such alkanoyloxyalkyl groups include the formyloxymethyl, acetoxymethyl, propionyloxymethyl, butyryloxymethyl, pivaloyloxymethyl, valeryloxymethyl, isovaleryloxymentyl, hexanoyloxymethyl, 1-(formyloxy)ethyl, 1-(acetoxy)ethyl, 1-(propionyloxy)ethyl, 1-(butyryloxy)ethyl, 1-(pivaloyloxy)ethyl, 1-(valeryloxy)ethyl, 1-(isovaleryloxy) ethyl, 1-(hexanoyloxy)ethyl, 2-(formyloxy)ethyl, 2-(acetoxy)ethyl, 2-(propionyloxy) ethyl, 2- (butyryloxy) ethyl, 2-(pivaloyloxy) ethyl, 2-(valeryloxy)ethyl, 2-(isovaleryloxy) ethyl, 2-(hexanoyloxy) ethyl, 1-(formyloxy)propyl, 1-(acetoxy)propyl, 1-(propionyloxy)propyl, 1-(butyryloxy)propyl, 1-(pivaloyloxy)propyl, 1-(valeryloxy)propyl, 1-(isovaleryloxy)propyl, 1-(hexanoyloxy)propyl, 1-(acetoxy)butyl, 1-(propionyloxy)butyl, 1-(butyryloxy)butyl, 1-(pivaloyloxy) butyl, 1-(acetoxy)pentyl, 1-(propionyloxy)pentyl, 1-(butyryloxy)pentyl, 1-(pivaloyloxy)pentyl and 1-(pivaloyloxy) hexyl groups, preferably the formyloxymethyl, acetoxymethyl, propionyloxymethyl, butyryloxymethyl, pivaloyloxymethyl, 1-(formyloxy) ethyl, 1-(acetoxy)ethyl, 1-(propionyloxy)ethyl, 1-(butyryloxy)ethyl and 1-(pivaloyloxy) ethyl groups, and more preferably the acetoxymethyl, propionyloxymethyl, butyryloxymethyl, pivaloyloxymethyl, 1-(acetoxy)ethyl, 1-(propionyloxy)ethyl, 1-(butyryloxy)ethyl and 1-(pivaloyloxy)ethyl groups and most preferably the pivaloyloxymethyl and 1-(pivaloyloxy)ethyl groups;
cycloalkanoyloxyalkyl groups in which the cycloalkyl part has 5 or 6 carbon atoms and the alkyl part has from 1 to 6 carbon atoms; preferably the alkyl part has from 1 to 4 carbon atoms and more preferably 1 or 2 carbon atoms; examples of such cycloalkanoyloxyalkyl groups include the cyclopentanoyloxymethyl, cyclohexanoyloxymethyl, 1-(cyclopentanoyloxy) ethyl, 1-(cyclohexanoyloxy)ethyl, 1-(cyclopentanoyloxy)propyl, 1-(cyclohexanoyloxy)propyl, 1-(cyclopentanoyloxy)butyl and 1-(cyclohexanoyloxy)butyl, groups, preferably the cyclopentanoyloxymethyl, cyclohexanoyloxymethyl, 1-(cyclopentanoyloxy)ethyl, and 1-(cyclohexanoyloxy)ethyl groups;
alkoxycarbonyloxyalkyl groups in which each of the alkoxy and the alkyl parts has from 1 to 6 carbon atoms as exemplified above in relation to substituents (b) and to the alkyl groups having from 1 to 6 carbon atoms, respectively, and preferably each of the alkoxy and the alkyl parts has from 1 to 4 carbon atoms and more preferably the alkoxy part has from 1 to 4 carbon atoms and the alkyl part has 1 or 2 carbon atoms; examples of such alkoxycarbonyloxyalkyl groups include the methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy) ethyl, 1-(pentyloxycarbonyloxy)ethyl, 1-(hexyloxy-carbonyloxy)ethyl, 2-(methoxycarbonyloxy)ethyl, 2-(ethoxycarbonyloxy)ethyl, 2-(propoxycarbonyloxy)ethyl, 2-(isopropoxycarbonyloxy)ethyl, 2-(butoxycarbonyloxy)ethyl, 2-(isobutoxycarbonyloxy)ethyl, 2-(pentyloxy-carbonyloxy)ethyl, 2-(hexyloxycarbonyloxy)ethyl, 1 -(methoxycarbonyloxy) propyl, 1-(ethoxycarbonyloxy)propyl, 1-(propoxycarbonyloxy)propyl, 1-(isopropoxy-carbonyloxy)propyl, 1-(butoxycarbonyloxy)propyl, 1-(isobutoxycarbonyloxy)propyl, 1-(pentyloxycarbonyl-oxy)propyl, 1-(hexyloxycarbonyloxy) propyl, 1-(methoxy-carbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl groups, preferably the methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, more preferably methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl and 1-(isobutoxycarbonyloxy)ethyl groups and most preferably the methoxycarbonyloxymethyl, ethoxycarbonyloxymethyl, isopropoxycarbonyloxymethyl, 1-(methoxycarbonyloxy)ethyl, 1-(ethoxycarbonyloxy)ethyl and 1-(isopropoxycarbonyloxy) ethyl groups;
cycloalkoxycarbonyloxyalkyl groups in which the cycloalkyl part has 5 or 6 carbon atoms and the alkyl part has from 1 to 6 carbon atoms; preferably the alkyl part has from 1 to 4 carbon atoms and more preferably 1 or 2 carbon atoms; examples of such cycloalkoxycarbonyloxyalkyl groups include the cyclopentoxycarbonyloxymethyl, cyclohexyloxycarbonyloxymethyl, 1-(cyclopentyloxycarbonyloxy)ethyl, 1-(cyclohexyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl and 1-(cyclohexyloxycarbonyloxy)butyl groups, preferably the cyclopentyloxycarbonyloxymethyl, cyclohexyloxycarbonyloxymethyl, 1-(cyclopentoxycarbonyloxy)ethyl and 1-(cyclohexyloxycarbonyloxy)ethyl groups; [5-(aryl-or alkyl-)-2-oxo-1,3-dioxolen-4-yl]methyl groups in which the alkyl part has from 1 to 6 carbon atoms and may be as exemplified above in relation to the alkyl groups having from 1 to 6 carbon atoms, and the aryl part is as defined and exemplified above in relation to the arylthioalkyl groups (and is preferably a substituted or unsubstituted phenyl group); preferably the alkyl part has from 1 to 4 carbon atoms and more preferably 1 or 2 carbon atoms; examples of such [5-(aryl-or alkyl-)-2-oxo-1,3-dioxolen-4-yl]methyl groups include the (5-phenyl-2-oxo-1,3-dioxolen-4-yl)methyl, [5-(4-methylphenyl)-2-oxo-1,3-dioxolen-4-yl]methyl, [5- (4-methoxyphenyl)-2-oxo-1,3-dioxolen-4-yl]methyl, [5-(4-chlorophenyl)-2-oxo-1,3-dioxolen-4-yl]methyl, [5-(4-fluorophenyl)-2-oxo-1,3-dioxolen-4-yl]methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl) methyl, (5-isopropyl-2-oxo-1,3-dioxolen-4-yl)methyl and (5-butyl-2-oxo-1,3- dioxolen-4-yl)methyl groups, preferably the (5-phenyl-2-oxo-1,3-dioxolen-4-yl)methyl, (5-methyl-2-oxo-1,3- dioxolen-4-yl)methyl and (5-ethyl-2-oxo-1,3-dioxolen-4-yl)methyl groups and more preferably the (5-methyl-2- oxo-1,3-dioxolen-4-yl)methyl group; and phthalidyl groups.

Preferred ester residues are, for example:
C₁ - C₄ alkyl groups;
phenyl, naphthyl and substituted phenyl groups having one or more, preferably from 1 to 3, methyl, ethyl methoxy, ethoxy, fluoro and chloro substituents, which, in the case of 2 or 3 substituents, may be the same or different;
benzyl, diphenylmethyl and a- and 6- naphthylmethyl groups, and substituted benzyl groups having one or more, preferably from 1 to 3, methyl, ethyl, methoxy, ethoxy, fluoro and chloro substituents, which, in the case of 2 or 3 substituents, may be the same or different;
groups of formula SiR^{d}R^{e}R^{f} in which 1, 2 or 3 of the groups represented by R^{d}, R^{e} and R^{f} are the same or different and each represents an alkyl group having from 1 to 4 carbon atoms, and 2, 1 or 0 are phenyl groups;
alkanoyloxyalkyl groups in which the alkanoyl group has from 1 to 5 carbon atoms and the alkyl group has from 1 to 4 carbon atoms;
cycloalkanoyloxyalkyl groups in which the cycloalkyl part has 5 or 6 carbon atoms and the alkyl part has from 1 to 4 carbon atoms;
alkoxycarbonyloxyalkyl groups in which each of the alkoxy part and the alkyl part has from 1 to 4 carbon atoms;
cycloalkoxycarbonyloxyalkyl groups in which the cycloalkyl part has 5 or 6 carbon atoms and the alkyl part has from 1 to 4 carbon atoms;
[5-(phenyl or alkyl-)-2-oxo-1 ,3-dioxolen-4-yl]methyl groups in which the alkyl part has from 1 to 4 carbon atoms, and phthalidyl groups.

More preferred ester residues are, for example,
C₁ - C₄ alkyl groups;
the benzyl group;
alkanoyloxyalkyl groups in which the alkanoyl part has from 1 to 5 carbon atoms and the alkyl part has 1 or 2 carbon atoms;
cycloalkanoyloxyalkyl groups in which the cycloalkyl part has from 5 to 6 carbon atoms and the alkyl part has 1 or 2 carbon atoms;
alkoxycarbonyloxyalkyl groups in which the alkoxy part has from 1 to 4 carbon atoms and the alkyl part has 1 or 2 carbon atoms;
cycloalkoxycarbonyloxyalkyl groups in which the cycloalkyl part has 5 or 6 carbon atoms and the alkyl part has 1 or 2 carbon atoms;
[5-(phenyl or alkyl-)-2-oxo-1,3-dioxolen-4-yl]methyl groups in which the alkyl part has 1 or 2 carbon atoms; and
phthalidyl groups.

The most preferred ester residues are, for example, the pivaloyloxymethyl, ethoxycarbonyloxymethyl, 1-(ethoxycarbonyloxy)ethyl, isopropoxycarbonyloxymethyl, (1-isopropoxycarbonyloxy) ethyl, (5-methyl-2-oxo-1,3- dioxolen-4-yl)methyl and phthalidyl groups.

Preferably R⁷ represents a carboxy group or a tetrazol-5-yl group, and, where R⁷ represents a carboxy group, salts of these compounds are also preferred. R⁷ is at the 2- or 3- position of the phenyl group, and more preferably at the 2-position.

The compounds of the present invention necessarily contain at least one basic nitrogen atom in the imidazole ring and can therefore form acid addition salts. Examples of such acid addition salts include: addition salts with inorganic acids, such as hydrochloric acid, hydrobromic acid, sulphuric acid or phosphoric acid; and addition salts with organic acids such as maleic acid, fumaric acid, tartaric acid or citric acid.

Preferred classes of compounds of the present invention are those compounds of formula (I) and salts and esters thereof, in which:
R¹ represents an alkyl group having from 2 to 5 carbon atoms;
R² and R³ are the same or different and each represents an alkyl group having from 1 to 4 carbon atoms;
R⁴ represents a hydrogen atom, a methyl group, an ethyl group or an alkanoyl group having from 1 to 5 carbon atoms;
R⁵ represents a group of formula -COOR^{5a} or a group of formula -CONR⁸R⁹, in which:
   R^{5a} represents
   a hydrogen atom,
   a methyl, ethyl or benzyl group,
   an alkanoyloxymethyl group, in which the alkanoyl part has from 1 to 5 carbon atoms,
   a 1-(alkanoyloxy)ethyl group, in which the alkanoyl part has from 1 to 5 carbon atoms,
   an alkoxycarbonyloxymethyl group, in which the alkoxy part has from 1 to 4 carbon atoms,
   a 1-(alkoxycarbonyloxy)ethyl group, in which the alkoxy part has from 1 to 4 carbon atoms,
   a [5-(phenyl-or methyl-)-2-oxo-1,3-dioxolen-4-yl]methyl group, or
   a phthalidyl group;
   R⁸ and R⁹ are the same or different and each represents a hydrogen atom, a methyl group, an ethyl group, a methoxycarbonylmethyl group, an ethoxy-carbonylmethyl group or a carboxymethyl group;
   or R⁸ and R⁹ together represent a tetramethylene, pentamethylene, 1-carboxytetramethylene or 1-carboxypentamethylene group;
R⁶ represents a hydrogen atom, or it represents a methyl group, an methoxy group, a fluorine atom or a chlorine atom at the 6-position of the benzene ring;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached.

The most preferred classes of compounds of the present invention are those compounds of formula (I) and salts and esters thereof, in which:
R¹ represents an ethyl, propyl or butyl group;
R² and R³ both represent methyl groups;
R⁴ represents a hydrogen atom or a methyl group;
R⁵ represents a group of formula -COOR^{5a}, in which R^{5a} represents a hydrogen atom, a pivaloyloxymethyl group, an ethoxycarbonyloxymethyl group, a 1-(ethoxy-carbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group, or a phthalidyl group;
R⁶ represents a hydrogen atom;
R⁷ represents a carboxy group or a tetrazol-5-yl group at the 2-position of the benzene ring; and
the benzene ring which bears the substituents represented by R⁶ and R⁷ is at the 4-position of the benzyl group towhich it is attached.

The compounds of the present invention may contain one or more asymmetric carbon atoms in their molecules, and can thus form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

Specific examples of individual compounds of the present invention are shown in the following formulae (I-1), (I-2), (I-3) and (I-5) :

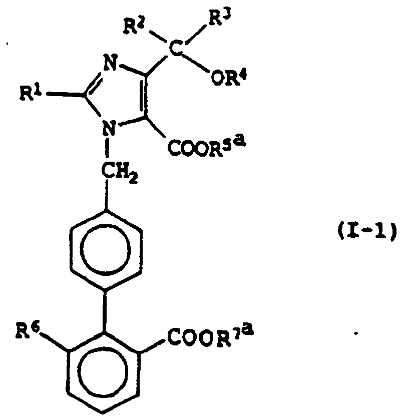

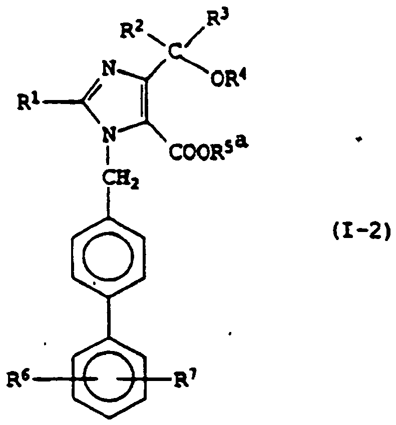

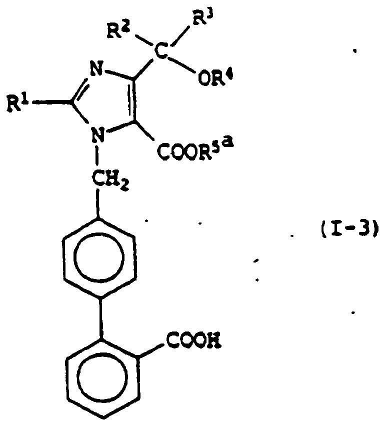

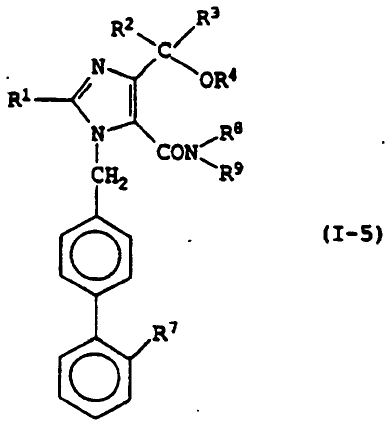

In these formulae, the meanings of the various substituent groups are as given in the following Tables 1, 2, 3 and 5, in which Table 1 relates to formula (I-1), Table 2 relates to formula (I-2), Table 3 relates to formula (I-3), and Table 5 relates to (I-5). In the Tables, the following abbreviations are used:

| | |
|---|---|
| Ac | acetyl |
| Boz | benzoyl |
| Bu | butyl |
| tBu | t-butyl |
| Buc | butoxycarbonyl |
| iBuc | isobutoxycarbonyl |
| Bz | benzyl |
| Et | ethyl |
| Etc | ethoxycarbonyl |
| Fo | formyl |
| cHx | cyclohexyl |
| Me | methyl |
| Mec | methoxycarbonyl |
| Mod | (5-methyl-2-oxo-1,3-dioxolen-4-yl)methyl |
| Phth | phthalidyl |
| Piv | pivaloyl |
| Pn | pentyl |
| cPn | cyclopentyl |
| Pr | propyl |
| iPr | isopropyl |
| iPrc | isopropoxycarbonyl |
| Tz | tetrazol-5-yl |

**Table 1**

| Cpd. No. | R¹ | R² | R³ | R⁴ | R^{5a} | R⁶ | R^{7a} |
|---|---|---|---|---|---|---|---|
| 1-31 | Bu | Me | Me | H | H | H | H |
| 1-32 | Bu | Me | Me | H | Et | H | H |
| 1-33 | Bu | Me | Me | H | Bu | H | H |
| 1-34 | Bu | Me | Me | H | Me | H | H |
| 1-35 | Bu | Me | Me | H | PivOCH2- | H | H |
| 1-36 | Bu | Me | Me | H | Mod | H | H |
| 1-37 | Bu | Me | Me | Me | H | H | H |
| 1-38 | Bu | Me | Me | Me | Et | H | H |
| 1-39 | Bu | Me | Me | Fo | H | H | H |
| 1-40 | Bu | Me | Me | Fo | Et | H | H |
| 1-41 | Bu | Me | Me | Ac | H | H | H |
| 1-42 | Bu | Me | Me | Ac | Et | H | H |
| 1-43 | Bu | Me | Me | Boz | H | H | H |
| 1-44 | Bu | Me | Me | Boz | Et | H | H |
| 1-45 | Bu | Me | Me | H | H | Cl | H |
| 1-46 | Bu | Me | Me | H | Et | Cl | H |
| 1-47 | Bu | Me | Me | H | H | OMe | H |
| 1-48 | Bu | Me | Me | H | Et | OMe | H |
| 1-49 | Pr | Me | Me | H | H | H | H |
| 1-50 | Pr | Me | Me | H | Et | H | H |
| 1-51 | Pr | Me | Me | Ac | Et | H | H |
| 1-52 | Pr | Me | Me | H | H | OMe | H |
| 1-53 | Pr | Me | Me | H | Et | OMe | H |
| 1-54 | Pn | Me | Me | H | H | H | H |
| 1-55 | Pn | Me | Me | H | Et | H | H |
| 1-82 | Bu | Et | Me | H | H | H | H |
| 1-83 | Bu | Et | Me | H | Et | H | H |
| 1-84 | Bu | Et | Et | H | H | H | H |
| 1-85 | Bu | Et | Et | H | Et | H | H |
| 1-86 | Bu | Et | Et | H | H | Cl | H |
| 1-87 | Bu | Et | Et | H | Et | Cl | H |
| 1-88 | Bu | Et | Et | H | H | OMe | H |
| 1-89 | Bu | Et | Et | H | Et | OMe | H |
| 1-108 | Pr | Me | Me | H | H | Cl | H |
| 1-109 | Pr | Me | Me | H | Et | Cl | H |
| 1-110 | Pr | Me | Me | H | H | H | Et |
| 1-111 | Pr | Me | Me | H | H | H | Bu |
| 1-112 | Pr | Me | Me | H | H | H | PivOCH₂- |
| 1-113 | Bu | Me | Me | H | H | H | Et |
| 1-114 | Bu | Me | Me | H | H | H | Bu |
| 1-115 | Bu | Me | Me | H | H | H | PivOCH₂- |
| 1-116 | Bu | Me | Me | Mec | H | H | H |
| 1-117 | Bu | Me | Me | Etc | H | H | H |
| 1-118 | Bu | Me | Me | H | Et | H | tBu |
| 1-119 | Pr | Me | Me | H | Et | H | tBu |
| 1-120 | Bu | Me | Me | H | H | F | H |
| 1-122 | Bu | Me | Me | H | H | Cl | tBu |
| 1-123 | Bu | Me | Me | H | Et | Cl | tBu |
| 1-124 | Bu | Me | Me | H | H | OMe | tBu |
| 1-125 | Bu | Me | Me | H | Et | OMe | tBu |
| 1-126 | Pr | Me | Me | H | H | Cl | tBu |
| 1-127 | Pr | Me | Me | H | Et | Cl | tBu |
| 1-128 | Pr | Me | Me | H | H | OMe. | tBu |
| 1-129 | Pr | Me | Me | H | Et | OMe | tBu |
| 1-130 | Et | Me | Me | H | Et | H | tBu |
| 1-131 | Et | Me | Me | H | Et | H | H |
| 1-132 | Et | Me | Me | H | H | H | H |

**Table 2**

| Cpd. No. | R¹ | R² | R³ | R⁴ | R^{5a} | R⁶ | R⁷ |
|---|---|---|---|---|---|---|---|
| 2-1 | Pr | Me | Me | H | H | H | 2-Tz |
| 2-2 | Bu | Me | Me | H | H | H | 2-Tz |
| 2-3 | Pn | Me | Me | H | H | H | 2-Tz |
| 2-4 | -CH=CH-Et | Me | Me | H | H | H | 2-Tz |
| 2-5 | Pr | Me | Me | Me | H | H | 2-Tz |
| 2-6 | Bu | Me | Me | Me | H | H | 2-Tz |
| 2-7 | Pr | Me | Me | H | Et | H | 2-Tz |
| 2-8 | Bu | Me | Me | H | Et | H | 2-Tz |
| 2-9 | Pr | Me | Me | H | Me | H | 2-Tz |
| 2-10 | Bu | Me | Me | H | Me | H | 2-Tz |
| 2-11 | Pr | Me | Me | Me | Me | H | 2-Tz |
| 2-12 | Bu | Me | Me | Me | Me | H | 2-Tz |
| 2-13 | Pr | Me | Me | Me | Et | H | 2-Tz |
| 2-14 | Bu | Me | Me | Me | Et | H | 2-Tz |
| 2-15 | Pr | Me | Me | H | PivOCH₂- | H | 2-Tz |
| 2-16 | Bu | Me | Me | H | PivOCH₂- | H | 2-Tz |
| 2-17 | Pr | Me | Me | H | Mod | H | 2-Tz |
| 2-18 | Bu | Me | Me | H | Mod | H | 2-Tz |
| 2-19 | Pr | Me | Me | H | EtcOCH₂- | H | 2-Tz |
| 2-20 | Bu | Me | Me | H | EtcOCH₂- | H | 2-Tz |
| 2-21 | Pr | Me | Me | H | iPrcOCH₂- | H | 2-Tz |
| 2-22 | Bu | Me | Me | H | iPrcOCH₂- | H | 2-Tz |
| 2-23 | Pr | Me | Me | H | 1-(EtcO)Et | H | 2-Tz |
| 2-24 | Bu | Me | Me | H | 1-(EtcO)Et | H | 2-Tz |
| 2-25 | Pr | Me | Me | H | 1-(iPrcO)Et | H | 2-Tz |
| 2-26 | Bu | Me | Me | H | 1-(iPrcO)Et | H | 2-Tz |
| 2-27 | Pr | Me | Me | Me | EtcOCH₂- | H | 2-Tz |
| 2-28 | Bu | Me | Me | Me | EtcOCH₂- | H | 2-Tz |
| 2-29 | Pr | Me | Me | Me | iPrcOCH₂- | H | 2-Tz |
| 2-30 | Bu | Me | Me | Me | iPrcOCH₂- | H | 2-Tz |
| 2-31 | Pr | Me | Me | Me | PivOCH₂- | H | 2-Tz |
| 2-32 | Bu | Me | Me | Me | PivOCH₂- | H | 2-Tz |
| 2-33 | Pr | Me | Me | H | H | 6-Cl | 2-Tz |
| 2-34 | Bu | Me | Me | H | H | 6-Cl | 2-Tz |
| 2-35 | Pr | Me | Me | H | H | 6-OMe | 2-Tz |
| 2-36 | Bu | Me | Me | H | H | 6-OMe | 2-Tz |
| 2-37 | Pr | Me | Et | H | H | H | 2-Tz |
| 2-38 | Bu | Me | Et | H | H | H | 2-Tz |
| 2-39 | Pr | Et | Et | H | H | H | 2-Tz |
| 2-40 | Bu | Et | Et | H | H | H | 2-Tz |
| 2-41 | Pr | Me | Me | H | Bz | H | 2-Tz |
| 2-42 | Pr | Me | Me | H | Bu - | H | 2-Tz |
| 2-43 | Bu | Me | Me | H | Bz | H | 2-Tz |
| 2-44 | Bu | Me | Me | H | Bu | H | 2-Tz |
| 2-45 | Pr | Et | Et | H | Et | H | 2-Tz |
| 2-46 | Pr | Me | Me | H | H | H | 3-Tz |
| 2-49 | Pr | Me | Me | H | Fo | H | 2-Tz |
| 2-50 | Pr | Me | Me | H | Ac | H | 2-Tz |
| 2-51 | Pr | Me | Me | H | H | 6-Cl | 3-Tz |
| 2-52 | Bu | Me | Me | H | H | 6-Cl | 3-Tz |
| 2-53 | Pr | Me | Me | H | H | 6-OMe | 3-Tz |
| 2-54 | Bu | -Me | Me | H | H | 6-OMe | 3-Tz |
| 2-55 | Pr | Me | Et | H | H | H | 3-Tz |
| 2-56 | Bu | Me | Et | H | H | H | 3-Tz |
| 2-57 | Pr | Et | Et | H | H | H | 3-Tz |
| 2-58 | Bu | Et | Et | H | H | H | 3-Tz |
| 2-59 | Pr | Me | Me | Me | Et | H | 3-Tz |
| 2-60 | Pr | Me | Me | Me | H | H | 3-Tz |
| 2-61 | Bu | Me | Me | Me | Et | H | 3-Tz |
| 2-62 | Bu | Me | Me | Me | H | H | 3-Tz |
| 2-63 | Pr | Et | Et | H | Et | H | 3-Tz |
| 2-64 | Pr | Me | Et | Me | H | H | 2-Tz |
| 2-65 | Pr | Me | Me | H | Phth | H | 2-Tz |
| 2-66 | Pr | Me | Me | Me | Mod | H | 2-Tz |
| 2-67 | Bu | Me | Me | Me | Mod | H | 2-Tz |
| 2-68 | Et | Me | Me | H | H | H | 2-Tz |
| 2-69 | Et | Me | Me | H | PivOCH₂- | H | 2-Tz |
| 2-70 | Et | Me | Me | H | EtcOCH₂- | H | 2-Tz |
| 2-71 | Et | Me | Me | H | iPrcOCH₂- | H | 2-Tz |
| 2-72 | Et | Me | Me | H | Et | H | 2-Tz |
| 2-73 | Et | Me | Me | H | Mod | H | 2-Tz |
| 2-74 | Et | Me | Me | H | Phth | H | 2-Tz |
| 2-75 | Et | Me | Me | Me | H | H | 2-Tz |
| 2-76 | Et | Me | Me | Me | PivOCH₂- | H | 2-Tz |
| 2-77 | Et | Me | Me | Me | Mod | H | 2-Tz |

**Table 3**

| Cpd. No. | R¹ | R² | R³ | R⁴ | R^{5a} |
|---|---|---|---|---|---|
| 3-1 | Pr | Me | Me | H | PivOCH₂- |
| 3-2 | Pr | Me | Me | H | AcOCH₂- |
| 3-3 | Pr | Me | Me | H | 1-(PivO)Et |
| 3-4 | Pr | Me | Me | H | 1-(AcO)Et |
| 3-5 | Pr | Me | Me | H | cPnCO.OCH₂- |
| 3-6 | Pr | Me | Me | H | cHxCO.OCH₂- |
| 3-7 | Pr | Me | Me | H | MecOCH₂- |
| 3-8 | Pr | Me | Me | H | 1-(MecO)Et |
| 3-9 | Pr | Me | Me | H | EtcOCH₂- |
| 3-10 | Pr | Me | Me | H | 1-(EtcO)Et |
| 3-13 | Pr | Me | Me | H | iPrcOCH₂- |
| 3-14 | Pr | Me | Me | H | 1-(iPrcO)Et |
| 3-17 | Pr | Me | Me | H | cPnO.CO.OCH₂- |
| 3-18 | Pr | Me | Me | H | cHxO.CO.OCH₂- |
| 3-19 | Pr | Me | Me | H | BucOCH₂- |
| 3-20 | Pr | Me | Me | H | 1-(BucO)Et |
| 3-21 | Pr | Me | Me | H | iBucOCH₂- |
| 3-22 | Pr | Me | Me | H | 1-(iBucO)Et |
| 3-23 | Pr | Me | Me | H | 1-(cPnO.CO.O)Et |
| 3-24 | Pr | Me | Me | H | 1-(cHxO.CO.O)Et |
| 3-25 | Pr | Me | Me | H | Mod |
| 3-26 | Pr | Me | Me | H | Phth |
| 3-27 | Bu | Et | Et | H | PivOCH₂- |
| 3-28 | Bu | Me | Me | H | AcOCH₂- |
| 3-29 | Bu | Me | Me | H | 1-(PivO)Et |
| 3-30 | Bu | Me | Me | H | 1-(AcO)Et |
| 3-31 | Bu | Me | Me | H | cPnCO.OCH₂- |
| 3-32 | Bu | Me | Me | H | cHxCO.OCH₂- |
| 3-33 | Bu | Me | Me | H | MeCOCH₂- |
| 3-34 | Bu | Me | Me | H | 1-(MecO)Et |
| 3-35 | Bu | Me | Me | H | EtcOCH₂- |
| 3-36 | Bu | Me | Me | H | 1-(EtcO)Et |
| 3-39 | Bu | Me | Me | H | iPrcOCH₂- |
| 3-40 | Bu | Me | Me | H | 1-(iPrcO)Et |
| 3-43 | Bu | Me | Me | H | cPnO.CO.OCH₂- |
| 3-44 | Bu | Me | Me | H | cHxO.CO.OCH₂- |
| 3-45 | Bu | Me | Me | H | BucOCH₂- |
| 3-46 | Bu | Me | Me | H | 1-(BucO)Et |
| 3-47 | Bu | Me | Me | H | iBucOCH₂- |
| 3-48 | Bu | Me | Me | H | 1-(iBucO)Et |
| 3-49 | Bu | Me | Me | H | 1-(cPnO.CO.O)Et |
| 3-50 | Bu | Me | Me | H | 1-(cHxO.CO.O)Et |
| 3-51 | Bu | Et | Et | H | Mod |
| 3-52 | Bu | Me | Me | H | Phth |
| 3-53 | Pr | Me | Me | Me | PivOCH₂- |
| 3-54 | Pr | Me | Me | Me | AcOCH₂- |
| 3-55 | Pr | Me | Me | Me | 1-(PivO)Et |
| 3-56 | Pr | Me | Me | Me | 1-(AcO)Et |
| 3-57 | Pr | Me | Me | Me | cPnCO.OCH₂- |
| 3-58 | Pr | Me | Me | Me | cHxCO.OCH₂- |
| 3-59 | Pr | Me | Me | Me | MecOCH₂- |
| 3-60 | Pr | Me | Me | Me | 1-(MecO)Et |
| 3-61 | Pr | Me | Me | Me | EtcOCH₂- |
| 3-62 | Pr | Me | Me | Me | 1-(EtcO)Et |
| 3-65 | Pr | Me | Me | Me | iPrcOCH₂- |
| 3-66 | Pr | Me | Me | Me | 1-(iPrcO)Et |
| 3-69 | Pr | Me | Me | Me | cPnO.CO.OCH₂- |
| 3-70 | Pr | Me | Me | Me | cHxO.CO.OCH₂- |
| 3-71 | Pr | Me | Me | Me | BucOCH₂- |
| 3-72 | Pr | Me | Me | Me | 1-(BucO)Et |
| 3-73 | Pr | Me | Me | Me | iBucOCH₂- |
| 3-74 | Pr | Me | Me | Me | 1-(iBucO)Et |
| 3-75 | Pr | Me | Me | Me | 1-(cPnO.CO.O)Et |
| 3-76 | Pr | Me | Me | Me | 1-(cHxO.CO.O)Et |
| 3-77 | Pr | Me | Me | Me | Mod |
| 3-78 | Pr | Me | Me | Me | Phth |
| 3-79 | Bu | Me | Me | Me | PivOCH₂- |
| 3-80 | Bu | Me | Me | Me | AcOCH₂- |
| 3-81 | Bu | Me | Me | Me | 1-(PivO)Et |
| 3-82 | Bu | Me | Me | Me | 1-(AcO)Et |
| 3-83 | Bu | Me | Me | Me | cPnCO.OCH₂- |
| 3-84 | Bu | Me | Me | Me | cHxCO.OCH₂- |
| 3-85 | Bu | Me | Me | Me | MecOCH₂- |
| 3-86 | Bu | Me | Me | Me | 1-(MecO)Et |
| 3-87 | Bu | Me | Me | Me | EtcOCH₂- |
| 3-88 | Bu | Me | Me | Me | 1-(EtcO)Et |
| 3-91 | Bu | Me | Me | Me | iPrcOCH₂- |
| 3-92 | Bu | Me | Me | Me | 1-(iPrcO)Et |
| 3-95 | Bu | Me | Me | Me | cPnO.CO.OCH₂- |
| 3-96 | Bu | Me | Me | Me | cHxO.CO.OCH₂- |
| 3-97 | Bu | Me | Me | Me | BucOCH₂- |
| 3-98 | Bu | Me | Me | Me | 1-(BucO)Et |
| 3-99 | Bu | Me | Me | Me | iBucOCH₂- |
| 3-100 | Bu | Me | Me | Me | 1-(iBucO)Et |
| 3-101 | Bu | Me | Me | Me | 1-(cPnO.CO.O)Et |
| 3-102 | Bu | Me | Me | Me | 1-(cHxO.CO.O)Et |
| 3-103 | Bu | Me | Me | Me | Mod |
| 3-104 | Bu | Me | Me | Me | Phth |
| 3-105 | Et | Me | Me | H | PivOCH₂- |
| 3-106 | Et | Me | Me | H | AcOCH₂- |
| 3-107 | Et | Me | Me | H | EtcOCH₂- |
| 3-108 | Et | Me | Me | H | 1-(EtcO)Et |
| 3-109 | Et | Me | Me | H | iPrcOCH₂- |
| 3-110 | Et | Me | Me | H | 1-(iPrcO)Et |
| 3-111 | Et | Me | Me | H | Mod |
| 3-112 | Et | Me | Me | H | Phth |
| 3-113 | Pn | Me | Me | H | PivOCH₂- |
| 3-114 | Pn | Me | Me | H | AcOCH₂- |
| 3-115 | Pn | Me | Me | H | EtcOCH₂- |
| 3-116 | Pn | Me | Me | H | 1-(EtcO)Et |
| 3-117 | Pn | Me | Me | H | iPrcOCH₂- |
| - 3-118 | Pn | Me | Me | H | 1-(iPrcO)Et |
| 3-119 | Pn | Me | Me | H | Mod |
| 3-120 | Pn | Me | Me | H | Phth |
| 3-121 | Pr | Me | Et | H | PivOCH₂- |
| 3-122 | Pr | Me | Et | H | AcOCH₂- |
| 3-123 | Pr | Me | Et | H | EtcOCH₂- |
| 3-124 | Pr | Me | Et | H | 1-(EtcO)Et |
| 3-125 | Pr | Me | Et | H | iPrcOCH₂- |
| 3-126 | Pr | Me | Et | H | 1-(iPrcO)Et |
| 3-127 | Pr | Me | Et | H | Mod |
| 3-128 | Pr | Me | Et | H | Phth |
| 3-129 | Pr | Et | Et | H | PivOCH₂- |
| 3-130 | Pr | Et | Et | H | AcOCH₂- |
| 3-131 | Pr | Et | Et | H | EtcOCH₂- |
| 3-132 | Pr | Et | Et | H | 1-(EtcO)Et |
| 3-133 | Pr | Et | Et | H | iPrcOCH₂- |
| 3-134 | Pr | Et | Et | H | 1-(iPrcO)Et |
| 3-135 | Pr | Et | Et | H | Mod |
| 3-136 | Pr | Et | Et | H | Phth |

**Table 5**

| Cpd. No. | R1 | R2 | R3 | R4 | R7 | R8 | R9 |
|---|---|---|---|---|---|---|---|
| 5-7 | Pr | Me | Me | H | COOH | H | H |
| 5-8 | Pr | Me | Et | H | COOH | H | H |
| 5-38 | Pr | Me | Me | H | Tz | H | H |
| 5-39 | Pr | Me | Et | H | Tz | H | H |
| 5-69 | Bu | Me | Me | H | COOH | H | H |
| 5-70 | Bu | Me | Et | H | COOH | H | H |
| 5-100 | Bu | Me | Me | H | Tz | H | H |
| 5-101 | Bu | Me | Et | H | Tz | H | H |
| 5-333 | Bu | iPr | iPr | H | COOH | H | H |
| 5-338 | Pr | Me | Me | H | -COOCH₂OPiv | H | H |
| 5-341 | Pr | Me | Me | H | -COOMod | H | H |
| 5-344 | Bu | Me | Me | H | -COOCH₂OPiv | H | H |
| 5-347 | Bu | Me | Me | H | -COOMod | H | H |

Of the compounds listed above, the following are preferred, that is to say Compounds No. 1-31, 1-35, 1-36, 1-37, 1-39, 1-41, 1-49, 1-54, 1-82, 1-84, 1-132, 2-1, 2-2, 2-3, 2-4, 2-5, 2-6, 2-15, 2-16, 2-17, 2-18, 2-19, 2-20, 2-21, 2-22, 2-23, 2-24, 2-25, 2-26, 2-27, 2-28, 2-29, 2-30, 2-31, 2-32, 2-37, 2-38, 2-39, 2-40, 2-49, 2-50, 2-64, 2-65, 2-66, 2-67, 2-68, 2-69, 2-70, 2-71, 2-73, 2-74, 2-75, 2-76, 2-77, 3-1, 3-9, 3-10, 3-13, 3-14, 3-25, 3-26, 3-27, 3-35, 3-36, 3-39, 3-40, 3-51, 3-52, 3-53, 3-61, 3-65, 3-77, 3-78, 3-79, 3-87, 3-91, 3-103, 3-104, 3-105, 3-107, 3-109, 3-111, 3-112, 3-121, 3-127, 3-128, 3-129, 3-135 and 3-136, of which Compounds No. 1-31, 1-35, 1-36, 1-37, 1-49, 1-54, 1-132, 2-1, 2-2, 2-3, 2-5, 2-6, 2-15, 2-16, 2-17, 2-18, 2-19, 2-20, 2-21, 2-22, 2-23, 2-24, 2-25, 2-26, 2-27, 2-28, 2-29, 2-30, 2-31, 2-32, 2-65, 2-66, 2-67, 2-68, 2-69, 2-70, 2-71, 2-73, 2-74, 2-75, 2-76, 2-77, 3-1, 3-9, 3-10, 3-13, 3-14, 3-25, 3-26, 3-35, 3-39, 3-40, 3-52, 3-53, 3-61, 3-65, 3-77, 3-78, 3-79, 3-87, 3-91, 3-103, 3-104, 3-105, 3-107, 3-109, 3-111 and 3-112 are more preferred, and Compounds No. 1-31, 1-35, 1-36, 1-49, 1-132, 2-1, 2-2, 2-3, 2-5, 2-6, 2-15, 2-16, 2-17, 2-18, 2-19, 2-20, 2-21, 2-22, 2-23, 2-24, 2-25, 2-26, 2-27, 2-28, 2-29, 2-30, 2-31, 2-32, 2-65, 2-66, 2-67, 2-68, 2-69, 2-70, 2-71, 2-73, 2-74, 2-75, 2-76, 2-77, 3-1, 3-9, 3-10, 3-13, 3-14, 3-25, 3-26, 3-53, 3-61, 3-65, 3-77 and 3-78 are still more preferred. The most preferred compounds are Compounds No.:
1-31. 2-Butyl-1-[(2'-carboxybiphenyl-4-yl)methyl]-4-(1-hydroxy-1-methylethyl)imidazole-5-carboxylic acid;
1-35. Pivaloyloxymethyl 2-butyl-1-[(2'-carboxybiphenyl-4-yl)methyl]-4-(1-hydroxy-1-methylethyl)imidazole-5-carboxylate;
1-36. (5-Methyl-2-oxo-1,3-dioxolen-4-yl)methyl 2-butyl-1-[(2'-carboxybiphenyl-4-yl)methyl]-4-(1-hydroxy-1-methylethyl)imidazole-5-carboxylate;
1-49. 1-[(2'-Carboxybiphenyl-4-yl)methyl]-4-(1-hydroxy-1-methylethyl)-2-propylimidazole-5-carboxylic acid;
1-132. 1-[(2'-Carboxybiphenyl-4-yl)methyl]-2-ethyl-4-(1-hydroxy-1-methylethyl)imidazole-5-carboxylic acid;
2-1. 4-(1-Hydroxy-1-methylethyl)-2-propyl-1-{4-[2-(tetrazol-5-yl)phenyl]phenyl}methylimidazole-5- carboxylic acid;
2-2. 2-Butyl-4-(1-hydroxy-1-methylethyl)-1-{4-[2-(tetrazol-5-yl)phenyl]phenyl}methylimidazole-5- carboxylic acid;
2-15. Pivaloyloxymethyl 4-(1-hydroxy-1-methylethyl)-2-propyl-1-{4-[2-(tetrazol-5-yl)phenyl]phenyl}methylimidazole-5-carboxylate;
2-16. Pivaloyloxymethyl 2-butyl-4-(1-hydroxy-1-methyl-ethyl)-1-{4-[2-(tetrazol-5-yl)phenyl]phenyl}methyl-imidazole-5-carboxylate;
2-17. (5-Methyl-2-oxo-1,3-dioxolen-4-yl)methyl 4-(1-hydroxy-1-methytethyl)-2-propyl-1-{4-[2-(tetrazol-5-yl)phenyl]phenyl}methylimidazole-5-carboxylate;
2-18. (5-Methyl-2-oxo-1,3-dioxolen-4-yl)methyl 2-butyl-4-(1-hydroxy-1-methylethyl)-1-{4-[2-(tetrazol-5-yl)-phenyl] phenyl}methylimidazole-5-carboxylate;
2-19. Ethoxycarbonyloxymethyl 4-(1-hydroxy-1-methyl-ethyl)-2-propyl-1-{4-[2-(tetrazol-5-yl)phenyl]phenyl}-methylimidazole-5-carboxylate;
2-21. Isopropoxycarbonyloxymethyl 4-(1-hydroxy-1-methylethyl)-2-propyl-1-{4-[2-(tetrazol-5-yl)phenyl]-phenyl} methylimidazole-5-carboxylate;
2-23. 1-(Ethoxycarbonyloxy)ethyl 4-(1-hydroxy-1-methylethyl)-2-propyl-1-{4-[2-(tetrazol-5-yl)phenyl]phenyl}-methylimidazole-5-carboxylate;
2-25. 1-(Isopropoxycarbonyloxy)ethyl 4-(1-hydroxy-1-methylethyl)-2-propyl-1-{4-[2-(tetrazol-5-yl)phenyl]-phenyl} methylimidazole-5-carboxylate;
2-69. Pivaloyloxymethyl 2-ethyl-4-(1-hydroxy-1-methylethyl)-1-{4-[2-(tetrazol-5-yl)phenyl]phenyl}methylimidazole-5-carboxylate;
2-73. (5-Methyl-2-oxo-1,3-dioxolen-4-yl)methyl 2-ethyl-4-(1-hydroxy-1-methylethyl)-1-{4-[2-(tetrazol-5-yl)-phenyl] phenyl}methylimidazole-5-carboxylate;
3-1. Pivaloyloxymethyl 1-[(2'-carboxybiphenyl-4-yl)-methyl]-4-(1-hydroxy-1-methylethyl)-2-propylimidazole-5-carboxylate;
3-25. (5-Methyl-2-oxo-1,3-dioxolen-4-yl)methyl 1-[(2'-carboxybiphenyl-4-yl)methyl]-4-(1-hydroxy-1-methylethyl)-2-propylimidazole-5-carboxylate; and
3-26. Phthalidyl 1-[(2'-carboxybiphenyl-4-yl)methyl]-4-(l-hydroxy-l-methylethyl)-2-propylimidazole-5- carboxylate;
and pharmaceutically acceptable salts thereof.

The compounds of the present invention may be prepared by a variety of methods well known in the art for the preparation of compounds of this type.

For example, in general terms, the compounds may be prepared by reacting a compound of formula (II): 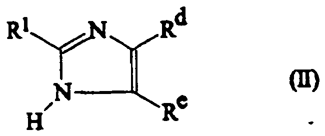 in which:
R¹ is as defined above and R^{d} represents a group of formula 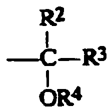 wherein R², R³ and R⁴ are as defined above,
or R^{d} represents a group of formula -COOR^{f} wherein R^{f} represents a carboxy-protecting group, R^{d} represents a group of formula -COR², wherein R² is as defined above, or R^{d} represents a cyano group; and
R^{e} represents a cyano group, a carboxy group or a group of formula -COOR^{f}, wherein R^{f} is as defined above, with a compound of formula (III) : 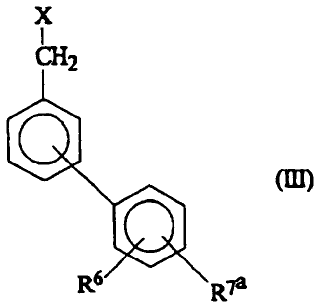 in which: R⁶ is as defined above; R^{7a} represents a protected carboxy group, a cyano group, a protected tetrazol-5-yl group, a carbamoyl group or an alkylcarbamoyl group; and X represents a halogen atom;
   to give a compound of formula (IV) : 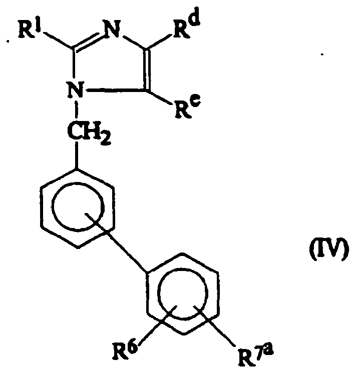 wherein R^{d}, R^{e}, R¹, R⁶ and R^{7a} are as defined above; and
   in any order, removing protecting groups, and; if necessary, converting said group R^{d} to a group of formula 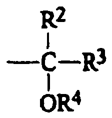 wherein R², R³ and R⁴ are as defined above,
and, if necessary, converting said group R^{e} to a group R⁵, converting said group R^{7a} to a group R⁷, or alkylating or acylating a hydroxy group in R⁴, to give a compound of formula (I); and optionally salifying or esterifying the product.

Preferably, R^{e} represents a protected carboxy group, when R^{7a} represents a protected carboxy group, a cyano group, a protected tetrazolyl group, a carbamoyl group or an alkylcarbamoyl group, and R^{e} represents a cyano group when R^{7a} represents a protected carboxy group or a protected tetrazolyl group.

In more detail, the compounds of the present invention may be prepared as described below in Reaction Schemes A to F.

### Reaction Scheme A:

In this Reaction Scheme, a compound of formula (I) is prepared by reacting an imidazole-5-carboxylic acid or ester thereof of formula (V) with a biphenylmethyl halide of formula (III), and then, if desired, removing protecting groups, converting the group of formula -COOR^{5a} to any other group represented by R⁵, converting the group represented by R^{7a} to any other group represented by R⁷ and/or alkylating or acylating a hydroxy group in R⁴, as shown below:

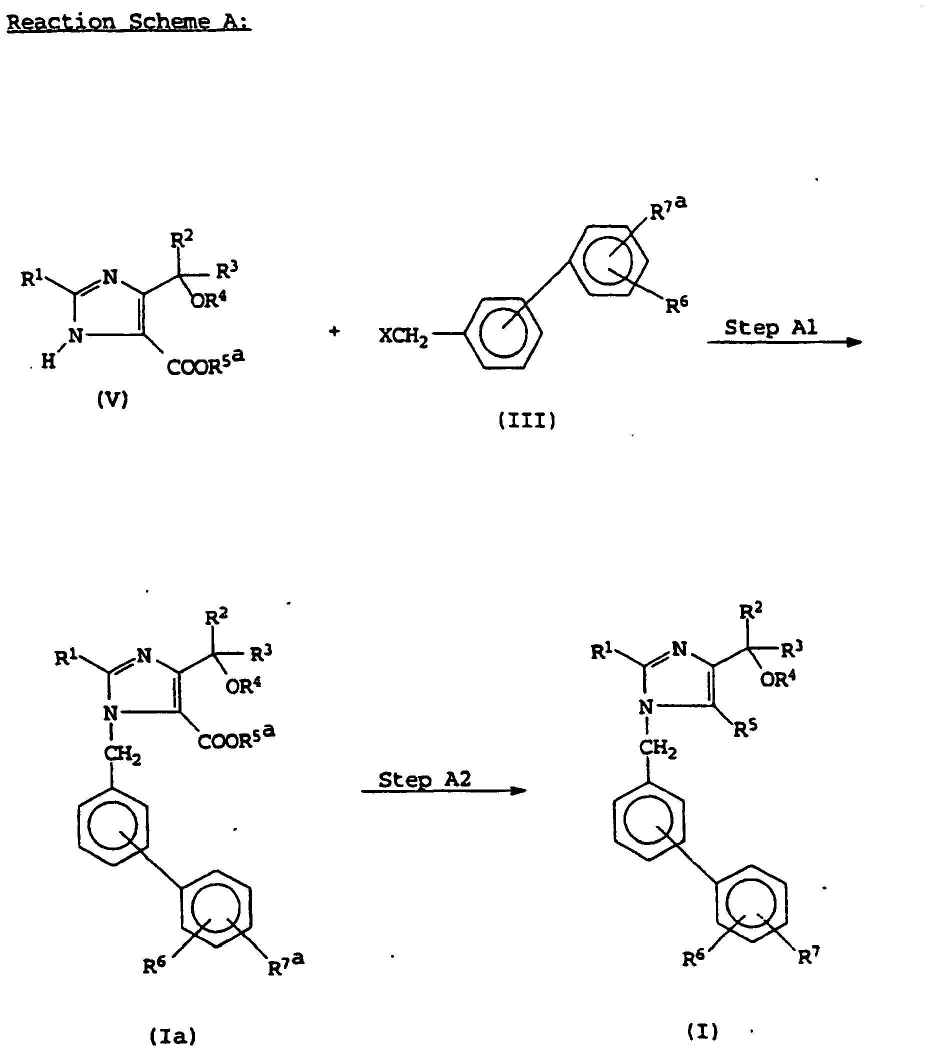

In the above reaction scheme, R¹, R², R³, R⁴, R⁵, R^{5a}, R⁶, R⁷, R^{7a} and X are as defined above, and R^{5a} preferably represents a group other than a hydrogen atom.

Where R^{7a} represents a protected carboxy group, the protecting group may be any of the ester residues illustrated above in relation to R^{5a} and R⁷. Alternatively, R^{7a} may be a carbamoyl group or a substituted carbamoyl group of formula -CONHR, where R represents a hydrogen atom or an alkyl group having from 1 to 6 carbon atoms, for example any of those illustrated above in relation to the alkyl groups having 1 to 6 carbon atoms. Examples of such carbamoyl groups which may be represented by R^{7a} include the carbamoyl, methylcarbamoyl, ethylcarbamoyl, propylcarbamoyl, butylcarbamoyl, t-butylcarbamoyl, pentylcarbamoyl, t-pentylcarbamoyl and hexylcarbamoyl groups, of which the carbamoyl, t-butylcarbamoyl and t-pentylcarbamoyl groups are preferred. Where R^{7a} represents a protected tetrazolyl group, the protecting group may be any protecting group commonly used to protect tetrazolyl groups in conventional compounds of this type. Examples of suitable protecting groups include the aralkyl groups defined and exemplified above in relation to the ester groups for R⁷, but it is preferably a benzyl, diphenylmethyl (benzhydryl) or triphenylmethyl (trityl group), most preferably a trityl group.

X represents a halogen atom, preferably a chlorine, bromine or iodine atom).

In Step A1 of this Reaction Scheme, a compound of formula (Ia) is prepared by reacting an imidazole-5-carboxylate compound of formula (V) with a biphenyl-methyl compound of formula (III). The reaction normally and preferably takes place in an inert solvent and preferably in the presence of a base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, preferably aromatic hydrocarbons, such as benzene or toluene; ethers, such as tetrahydrofuran or dioxane; alcohols, such as methanol, ethanol or t-butanol; amides, such as N,N-dimethylacetamide, N,N-dimethylformamide or N-methyl-2-pyrrolidinone; ketones, such as acetone or methyl ethyl ketone; nitriles, such as acetonitrile; and sulphoxides, such as dimethyl sulphoxide. Of these, we prefer the amides, ketones, nitriles and sulphoxides.

The nature of the base employed in the reaction is likewise not critical, and any base capable of reacting with the acid H-X can be used in this reaction. Preferred examples of bases which may be used include: alkali metal carbonates, such as sodium carbonate or potassium carbonate; alkali metal hydrides, such as sodium hydride, potassium hydride or lithium hydride; alkali metal alkoxides, such as sodium methoxide, sodium ethoxide, potassium t-butoxide or lithium methoxide; and alkali metal hydrogencarbonates, such as sodium hydrogencarbonate or potassium hydrogencarbonate. Of these, we prefer the alkali metal carbonates, alkali metal hydrides or alkali metal alkoxides.

The reaction can take place over a wide range of temperatures, and the precise reaction temperature is not critical to the invention. In general, we find it convenient to carry out the reaction at a temperature of from -10°C to 100°C, more preferably from 0°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired compound of formula (Ia) can be recovered from the reaction mixture by conventional means. For example, one suitable recovery procedure comprises: removing the solvent by distillation under reduced pressure; mixing the residue with water; extracted the residue with a water-immiscible solvent, such as ethyl acetate; drying the extract over, for example, anhydrous sodium sulphate; and freeing the product from the solvent by distillation. The resulting product can, if necessary, be purified by conventional means, for example, by recrystallization, or the various chromatography techniques, notably preparative thin layer chromatography or column chromatography.

Step A2 may comprise any one or (if appropriate) more of the following reactions:
(i) removing the carboxy-protecting groups either selectively or non-selectively from the group of formula - COOR^{5a} and/or the group R^{7a}, to convert it or them to a free carboxy group as represented by R⁵ or R⁷, respectively;
(ii) esterifying any such free carboxy group to provide an ester of the group, for example as illustrated above in relation to R⁷;
(iii) converting such a free carboxy group represented by R⁵ to a group of formula -CONR⁸R⁹;
(iv) removing the tetrazolyl-protecting group;
(v) converting a cyano group represented by R^{7a} to a tetrazolyl group;
(vi) converting a monoalkylcarbamoyl group or a carbamoyl group represented by R^{7a} first to a cyano group and then to a tetrazolyl group;
(vii) where R⁴ represents an aliphatic acyl group which can be regarded as a hydroxy-protecting group, removing the protecting group to produce a compound in which R⁴ represents a hydrogen atom; and
(viii) where R⁴ represents a hydrogen atom, alkylating or acylating this group.

### (i) Removal of carboxy-protecting groups:

The nature of the reaction employed to remove the carboxy-protecting group will, of course, depend on the nature of the group to be removed and such reactions are well known in the field of organic synthesis.

For example, where the carboxy-protecting group is an aralkyl group, such as abenzyl or p-nitrobenzyl group, the protecting group may be removed by catalytic reduction, in the presence of hydrogen, which may be under atmospheric pressure or superatmospheric pressure, for example up to 5 atmospheres pressure. The reaction normally and preferably takes place in an inert solvent (preferably an alcohol, such as methanol or ethanol, or a carboxylic acid, such as acetic acid) and in the presence of a catalyst. Any catalyst commonly used for catalytic hydrogenation or reduction may equally be employed here, preferably palladium-on-charcoal or platinum oxide.

Where the carboxy-protecting group is a t-butyl or diphenylmethyl group, it may be removed by reacting the protected compound with an acid (preferably a mineral acid, such as hydrogen chloride or sulphuric acid, or an organic acid, such as trifluoroacetic acid, methane-sulphonic acid or p-toluenesulphonic acid) in an inert solvent (preferably an alcohol, such as methanol or ethanol; an ether, such as tetrahydrofuran or dioxane; water; or a mixture of water and one or more of the above organic solvents).

Where the carboxy-protecting group is a silyl group, this may be a group of formula -SiR^{d}R^{e}R^{f}, in which R^{d}, R^{e} and R^{f} are as defined above. In this case, the protecting group may be removed by reacting the protected compound with an acid (preferably a mineral acid, such as hydrogen chloride, or an organic acid, such as acetic acid, trifluoroacetic acid, methane-sulphonic acid or p-toluenesulphonic acid) or with a fluorine salt, such as tetrabutylammonium fluoride. The reaction normally and preferably takes place in an inert solvent (preferably: an ether, such as tetrahydrofuran or dioxane; an alcohol, such as methanol or ethanol; an amide, such as N,N-dimethylformamide or N,N-dimethyl-acetamide; water; or a mixture of water and one or more of the above organic solvents).

Where the carboxy-protecting group is an ester residue, the protecting group may be removed by hydrolysis using a base (preferably an alkali metal hydroxide, such as lithium hydroxide, sodium hydroxide or potassium hydroxide, or an alkali metal carbonate, such as sodium carbonate or potassium carbonate) in an inert solvent (preferably an alcohol, such as methanol or ethanol; an ether, such as tetrahydrofuran or dioxane; water; or a mixture of water and one or more of the above organic solvents). Where R⁴ represents an acyl group, it is removed simultaneously in the course of this reaction.

The reaction can take place over a wide range of temperatures, and the precise reaction temperature is not critical to the invention. In general, we find it convenient to carry out the reaction at a temperature of from 0°C to 100°C, more preferably from about room temperature to 60°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired compound may be recovered by conventional means, the nature of which will depend on the nature of the deprotection reaction. For example, where the deprotection is carried out by catalytic reduction, the desired product can be recovered by filtering off the catalyst and then distilling off the solvent. Where the deprotection is carried out using an acid, the desired product can be recovered by collecting the precipitate in the reaction system by filtration or by concentration of the reaction mixture. Where the deprotection is carried,out by alkaline hydrolysis, the desired productcan be recovered by distilling off the solvent and then neutralizing the residue with an aqueous acid, after which the precipitate in the aqueous solvent may be collected by filtration; alternatively, it may be recovered by neutralizing the aqueous layer obtained by extracting the reaction mixture with a water-immiscible organic solvent (such as ethyl acetate or diethyl ether), extracting the neutralized solution with a water-immiscible'organic solvent (such as ethyl acetate), and then distilling off the solvent. The reaction product may, if necessary, be further purified by conventional means, for example by recrystallization or by the various chromatography techniques, notably preparative thin layer chromatography or column chromatography.

Each of the protecting groups represented by R^{5a} and R^{7a} can be selectively eliminated by appropriate choice of the protecting groups and the specific reaction conditions employed to remove them, as is well known to those skilled in the art.

### (ii) Esterification

Where a compound containing one or more free carboxy groups is produced, this group or these groups may be esterified, by methods well known in organic chemistry. For example, the reaction may be carried out by reacting the corresponding carboxylic acid with a compound of formula, R^{5b}-Y [in which R^{5b} may represent any of the groups defined above for R^{5a} other than a hydrogen atom, and Y represents a halogen atom, such as a chlorine, bromine or iodine atom, a group of formula -OSO₃R^{5b} (in which R^{5b} is as defined above) or a sulphonyloxy group, such as a methanesulphonyloxy or p-toluenesulphonyloxy group]. The reaction is carried out in the presence of a base, for example: an organic amine, such as triethylamine, pyridine or N-methyl-morpholine; an alkali metal carbonate, such as sodium carbonate or potassium carbonate; or an alkali metal hydrogencarbonate, such as sodium hydrogencarbonate or potassium hydrogencarbonate. It is also normally and preferably carried out in an inert solvent (preferably: an amide, such as N,N-dimethylformamide or N,N-dimethylacetamide; a halogenated hydrocarbon, preferably a halogenated aliphatic hydrocarbon, such as methylene chloride; a ketone, such as acetone or methyl ethyl ketone; or an ether, such as tetrahydrofuran or dioxane). Where the desired ester group is an alkyl group, the reaction may be carried out by reacting the carboxylic acid with the corresponding dialkyl sulphate.

The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

Where the carboxy-protecting group is a C₁ - C₆ alkyl group, the esterification reaction may be carried out by reacting the corresponding carboxylic acid with a C₁ - C₆ alcohol, such as methanol, ethanol, propanol or hexanol, in the presence of an acid catalyst, such as hydrogen chloride or sulphuric acid, in an inert solvent (for example: one of the C₁ - C₆ alcohols which may be used as the starting material described above; a halogenated hydrocarbon, such as methylene chloride; or an ether, such as tetrahydrofuran or dioxane) at a temperature of from 0°C to 100°C for a period of from 1 to 24 hours, or by reacting the corresponding carboxylic acid with a halogenating agent (e.g. phosphorus pentachloride, thionyl chloride or oxalyl chloride) in an inert solvent (for example: a halogenated hydrocarbon, such as methylene chloride; an ether, such as tetrahydrofuran or dioxane; or an aromatic hydrocarbon, such as benzene or toluene) at a temperature of about room temperature for a period of from 30 minutes to 5 hours to yield the corresponding acyl halide, which is then reacted with the corresponding alcohol in an inert solvent (e.g. benzene or methylene chloride) in the presence of a base (for example triethylamine; however, in the case of the t-butyl ester, potassium t-butoxide is used as the preferred base) at a temperature of about room temperature for a period of from 30 minutes to 10 hours. The desired compound can be recovered by conventional means, for example, by a similar method to that described in Step A1.

### (iii) Formation of a carbamoyl group

Conversion of a carboxy group represented by R⁵ to a group of formula -CONR⁸R⁹, in which R⁸ and R⁹ are as defined above, may be carried out using well known methods, for example by reacting the carboxylic acid compound, in which the group R⁷ is protected, with a compound of formula (VI):

R⁸R⁹NH (VI)

in which R⁸ and R⁹ are as defined above).

This reaction consists of the formation of a peptide bond and is generally well known in organic synthetic chemistry. It may be carried out in an inert solvent (preferably a halogenated hydrocarbon, more preferably a halogenated aliphatic hydrocarbon, such as methylene chloride or chloroform; an ester, such as ethyl acetate; an ether, such as tetrahydrofuran or dioxane; or an amide, such as N,N-dimethylacetamide or N,N-dimethyl-formamide) in the presence of a condensing agent.

Examples of condensing agents which may be used in this reaction include: carbodiimides, such as N,N-dicyclohexylcarbodiimide or 1-(3-dimethylamino-propyl)-3-ethylcarbodiimide hydrochloride; phosphoryl compounds, such as diphenylphosphoryl azide or diethylphosphoryl cyanide; carbonyldiimidazole; and triphenylphosphine-diethyl azodicarboxylate. Of these, we prefer the carbodiimides and diphenylphosphoryl azide. Where a phosphoryl compound is used, the reaction is preferably carried out in the presence of a tertiary amine, such as triethylamine or N-methyl-morpholine.

Alternatively, the reaction in this step can be accomplished by reacting the carboxylic acid with a lower alkyl chloroformate, such as ethyl chloroformate or isobutyl chloroformate, in the presence of a tertiary amine, such as triethylamine or N-methylmorpholine, to produce a mixed acid anhydride, or by reacting the carboxylic acid with N-hydroxysuccinimide, N-hydroxy-benzotriazole or p-nitrophenol or the like in the presence of a carbodiimide, such as N,N-dicyclohexyl-carbodiimide, to produce the corresponding active ester, and subsequently reacting the mixed acid anhydride or the active ester with the amine compound of formula (VI).

As a further alternative, the reaction in this step can be carried out by reacting the carboxylic acid with a halogenating agent, such as phosphorus pentachloride, oxalyl chloride or thionyl chloride, in an inert solvent (for example: a halogenated hydrocarbon, such as methylene chloride; an ether, such.as tetrahydrofuran or dioxane; or an aromatic hydrocarbon, such as benzene or toluene) to give the corresponding acyl halide, and then reacting the acyl halide with the amine compound of formula (VI).

All of these reactions can take place over a wide range of temperatures, and the precise reaction temperature is not critical to the invention. In general, we find it convenient to carry out the reaction at a temperature of from -20°C to 100°C, more preferably from -5°C to 50°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the reaction product can be recovered from the reaction mixture by conventional means. For example, insoluble materials in the reaction system are filtered off; a water-immiscible organic solvent, such as ethyl acetate, and water are added to the filtrate; the organic solvent layer is separated and dried over a drying agent, such as anhydrous magnesium sulphate; and then the solvent is distilled off to leave the desired product. The reaction product may, if necessary, be further purified by conventional means, for example by recrystallization or by the various chromatography techniques, notably preparative thin layer chromatography or column chromatography.

### (iv) Removal of tetrazolyl-protecting groups

This may be accomplished by reacting the protected compound with an acid. The reaction is normally and preferably effected in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; an organic acid, such as acetic acid; an ether, such as tetrahydrofuran or dioxane; an alcohol, such as methanol, ethanol or t-butanol; a ketone, such as acetone or methyl ethyl ketone; or a mixture of any two or more of these solvents. Of these, we prefer water, an organic acid, an alcohol or a mixture thereof.

There is no particular limitation upon the nature of the acid used in the reaction, provided that it can normally function as a Bronsted acid. Preferred examples of such acids include: organic acids, such as acetic acid, formic acid, oxalic acid, methanesulphonic acid, p-toluenesulphonic acid or trifluoroacetic acid; and inorganic acids, such as hydrochloric acid, hydrobromic acid, sulphuric acid or phosphoric acid. Of these, we prefer acetic acid, formic acid, trifluoro-acetic acid or hydrochloric acid.

The reaction can take place over a wide range of temperatures, and the precise reaction temperature is not critical to the invention. In general, we find it convenient to carry out the reaction at a temperature- of from -10°C to 120°C, more preferably from 0°C to 100°C. The time required for the reaction may also vary widely, depending on many factors, notably the reaction temperature and the nature of the reagents and solvent employed. However, provided that the reaction is effected under the preferred conditions outlined above, a period of from from 0.5 to 24 hours, more preferably from 1 to 16 hours, will usually suffice.

After completion of the reaction, the desired product of this reaction can be recovered from the reaction mixtu re by conventional means. For example, after distilling off the solvent, the residue is dissolved in water and a water-immiscible organic solvent. The organic layer containing the desired compound is separated and dried over anhydrous magnesium sulphate. After distilling off the solvent, the desired compound can be obtained. The reaction product may, if necessary, be further purified by conventional means, for example by recrystallization or the various chromatography techniques, notably preparative thin layer chromatography or column chromatography.

### (v) Conversion of a cyano group to a tetrazolyl group

In this step, a cyano'group is converted to a tetrazolyl group by reacting the cyano compound with an alkali metal azide.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: amides, such as N,N-dimethylformamide or N,N-dimethylacetamide; ethers, such as dioxane or 1,2-dimethoxyethane; and sulphoxides, such as dimethyl sulphoxide.

Examples of suitable alkali metal azides include lithium azide, sodium azide and potassium azide, of which sodium azide is preferred. There is no particular restriction on the amount of alkali metal azide eployed, but we generally prefer to use from 1 to 5 equivalents, more preferably from 1 to 3 equivalents, of the alkali metal azide per equivalent of the cyano compound.

We also prefer to carry out the reaction in the presence of an ammonium halide, for example ammonium fluoride, ammonium chloride or ammonium bromide, of which ammonium chloride is preferred. There is no particular restriction on the amount of ammonium halide employed, but we generally prefer to use from 0.5 to 2 equivalents, more preferably from 1 to 1.2 equivalents, of the ammonium halide per equivalent of the cyano compound.

The reaction can take place over a wide range of temperatures, and the precise reaction temperature is not critical to the invention. In general, we find it convenient to carry out the reaction at a temperature of from 70 to 150°C, more preferably from 80 to 120°C. The time required for the reaction may also vary widely, depending on many factors, notably the reaction temperature and the nature of the reagents and solvent employed. However, provided that the reaction is effected under the preferred conditions outlined above, a period of from 10 hours to 7 days, more preferably from 1 to 5 days, will usually suffice.

Alternatively, the cyano group may be converted to a tetrazolyl group by reacting the cyano compound with a trialkyltin azide or triaryltin azide, and then treating the resulting tin compound with an acid, a base or an alkali metal fluoride.

The reaction of the cyano compound with the trialkyltin azide or triaryltin azide is normally and preferably effected in the presence of a solvent. There is noparticular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as 1,2-dichloroethane or chloroform; ethers, such as dioxane or 1,2-dimethoxyethane; amides, such as N,N-dimethylformamide or N,N-dimethylacetamide; and esters, such as-ethyl acetate or butyl acetate.

Although there is no particular limitation on the nature of the trialkyltin or triaryl tin azide, and any such compound commonly used in reactions of this type may equally be employed here, we generally prefer to use: a trialkyltin azide in which each of the alkyl groups (which may be the same or different, although they are preferably the same) have from 1 to 4 carbon atoms, for example trimethyltin azide, triethyltin azide or tributyltin azide; or a triaryltin azide in which each of the aryl groups (which may be the same or different, although they are preferably the same) is as defined above in relation to the aryl groups of the arylthioalkyl groups above, preferably a phenyl or substituted phenyl group, for example triphenyltin azide or tritolyltin azide. The amount of the trialkyltin azide or triaryltin azide employed is not critical, although an amount of from 1 to 3 equivalents per equivalent of cyano compound is preferred, and from 1 to 2 equivalents is more preferred.

The reaction of the cyano compound with the trialkyltin azide or triaryltin azide can take place over a wide range of temperatures, and the precise reaction temperature is not critical to the invention. In general, we find it convenient to carry out the reaction at a temperature of from 60 to 150°C, more preferably from 80 to 120°C. The time required for the reaction may also vary widely, depending on many factors, notably the reaction temperature and the nature of the reagents and solvent employed. However, provided that the reaction is effected under the preferred conditions outlined above, a period of from 8 hours to 7 days, more preferably from 1 to 5 days, will usually suffice.

The tin-containing compound produced by this reaction is then treated with an acid, a base or an alkali metal fluoride, to convert it to the desired tetrazolyl compound. Any acid, base or alkali metal fluoride commonly used for this type of reaction may be used in this reaction, and examples of suitable compounds include: acids, especially mineral acids, such as hydrochloric acid or sulphuric acid; bases, especially inorganic bases, such as alkali metal carbonates and hydrogencarbonates (for example sodium carbonate, potassium carbonate, sodium hydrogencarbonate or potassium hydrogencarbonate) or alkali metal hydroxides (for example sodium hydroxide or potassium hydroxide); and alkali metal fluorides, such as lithium fluoride, sodium fluoride or potassium fluoride.

The reaction is normally and preferably effected in the presence of a solvent. There is no particular restriction on the nature of the solvent to be employed, provided that it has no adverse effect on the reaction or-on the reagents involved and that it can dissolve the reagents, at least to some extent. Examples of suitable solvents include those listed above for the reaction of the cyano compound with the trialkyltin azide or triaryltin azide and other solvents, such as alcohols (for example methanol or ethanol), water or aqueous alcohols. The reaction can take place over a wide range of temperatures, and the precise reaction temperature is not critical to the invention. In general, we find it convenient to carry out the reaction at a temperature of from 0°C to 100°C, preferably about room temperature. The time required for the reaction may also vary widely, depending on many factors, notably the reaction temperature and the nature of the reagents and solvent employed. However, provided that the reaction is effected under the preferred conditions outlined above, a period of from 30 minutes to 3 days, more preferably from 1 hour to 24 hours, will usually suffice.

A further alternative method of converting a cyano group to a tetrazolyl group is to react the cyano compound with a trialkyltin halide ortriaryltin halide, in the presence of an alkali metal azide, afterwhich the resulting tin compound is treated with an acid, a base or an alkali metal fluoride.

The reaction of the cyano compound with the trialkyltin halide or triaryltin halide in the presence of an alkali metal azide is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as 1,2-dichloroethane or chloroform; ,ethers, such as dioxane or 1,2-dimethoxyethane; ketones, such as acetone or methyl ethyl ketone; amides, such as N,N-dimethylformamide or N,N-dimethylacetamide; and esters, such as ethyl acetate or butyl acetate.

Although there is no particular limitation on the nature of the trialkyltin or triaryl tin halide, and any such compound commonly used in reactions of this type may equally - be employed here, we generally prefer to use: a trialkyltin halide in which each of the alkyl groups (which may be the same or different, although they are preferably the same) has from 1 to 4 carbon atoms, for example trimethyltin chloride, trimethyltin bromide, triethyltin chloride or tributyltin chloride; or-a triaryltin halide in which each of the aryl groups (which may be the same or different, although they are preferably the same) is as defined above in relation to the aryl groups of the arylthioalkyl groups above, preferably a phenyl or substituted phenyl group, for example triphenyltin chloride or tritolyltin chloride. The amount of the trialkyltin halide or triaryltin halide employed is not critical, although an amount of from 1 to 3 equivalents per equivalent of cyano compound is preferred, and from 1 to 2 equivalents is more preferred.

There is no particular restriction on the alkali metal azide which is also employed in this reaction. Examples include lithium azide, sodium azide and potassium azide, of which sodium azide is preferred. The amount of the alkali metal azide employed is not critical, although an-amount of from 1 to 3 equivalents per equivalent of cyano compound is preferred, and from 1 to 2 equivalents is more preferred.

The reaction of the cyano compound with the trialkyltin halide or triaryltin halide in the presence of an alkali metal azide can take place over a wide range of temperatures, and the precise reaction temperature is not critical to the invention. In general, we find it convenient to carry out the reaction at a temperature of from 60 to 150°C, more preferably from 80 to 120°C. The time required for the reaction may also vary widely, depending on many factors, notably the reaction temperature and the nature of the reagents and solvent employed. However, provided that the reaction is effected under the preferred conditions outlined above, a period of from 8 hours to 7 days, more preferably from 1 to 5 days, will usually suffice.

The tin-containing compound produced by this reaction is then treated with an acid, a base or an alkali metal fluoride, to convert it to the desired tetrazolyl compound. The reaction is essentially the same as the reaction of the tin-containing compound (produced by reacting the cyano compound with a trialkyltin azide or triaryltin azide) with an acid, a base or an alkali metal fluoride, and may be carried out using the same solvents and reaction conditions.

### (vi) Conversion of an alkylcarbamoyl group or a carbamoyl group to a cyano group

To convert an alkylcarbamoyl group to a cyano group, the alkylcarbamoyl compound is reacted with a halogen compound capable of acting as a halogenating agent, preferably chlorinating agent, for example oxalyl chloride, phosphorus oxychloride or sulphonyl chloride. There is no particular restriction on the amount of halogen compound employed, although we generally find it convenient to use from 1 to 3 equivalents, more preferably from .1 to 2 equivalents, per equivalent of the alkylcarbamoyl compound.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as methylene chloride or chloroform; ethers, such as dioxane, tetrahydrofuran or diethyl ether; and esters, such as ethyl acetate or butyl acetate.

The reaction can take place over a wide range of temperatures, and the precise reaction temperature is not critical to the invention. In general, we find it convenient to carry out the reaction at a temperature of from -10 to 100°C, more preferably from 0 to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 16 hours, more preferably from 30 minutes to 6 hours, will usually suffice.

To convert a carbamoyl group to a cyano group, the carbamoyl compound is reacted with a dehydrating agent, for example acetic anhydride, trifluoroacetic anhydride, methanesulphonic anhydride, trifluoromethanesulphonic anhydride, oxalyl chloride or sulphonyl chloride, in the presence of an organic amine, for example triethylamine, pyridine or N-methylmorpholine.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as methylene chloride or chloroform; ethers, such as dioxane, tetrahydrofuran or diethyl ether; and esters, such as ethyl acetate or butyl acetate.

The reaction can take place over a wide range of temperatures, and the precise reaction temperature is not critical to the invention. In general, we find it convenient to carry out the reaction at a temperature of from -10 to 100°C, more preferably from 0 to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 16 hours, more preferably from 30 minutes to 6 hours, will usually suffice.

The desired product of these reactions can be recovered from the reaction mixture by conventional means, for example by neutralizing the mixture with a weak base, such as sodium hydrogencarbonate and then working up the product in a similar manner to that described in Step A1 of Reaction Scheme A.

The cyano compound thus obtained may then be converted to the corresponding tetrazolyl compound, using any of the reactions described above.

### (vii) Removing hydroxy-protecting groups

Where R⁴ represents an acyl group, which can be regarded as a hydroxy-protecting group, the protecting group is removed, to produce a compound in which R⁴ represents a hydrogen atom. The nature of the reaction employed to remove the protecting group, will, of course, depend on the nature of the protecting group, as is well known in the art, and any of the many well known reactions used for deprotecting compounds of this type may equally be used here.

Where the hydroxy-protecting group is an aliphatic acyl group, a benzoyl group or an alkoxycarbonyl group, it can be removed by treating the protected compound with a base.

There is no particular limitation upon the nature of the base used, provided that it does not affect other parts of the compound. Preferred examples of such bases include: metal alkoxides, especially alkali metal alkoxides, such as sodium methoxide; alkali metal carbonates, such as sodium carbonate or potassium carbonate; alkali metal hydroxides, such as sodium hydroxide or potassium hydroxide; and ammonia, which is preferably in the form of aqueous ammonia or a concentrated solution of ammonia in methanol.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; organic solvents, such as alcohols (e.g. methanol, ethanol or propanol) or ethers (e.g. tetrahydrofuran or dioxane); or a mixture of water and one or more of these organic solvents.

The reaction can take place over a wide range of temperatures, and the precise reaction temperature is not critical to the invention. In general, we find it convenient to carry out the reaction at a temperature of from 0°C to 150°C, more preferably from 0°C to 60°C. The time required for the reaction may also vary widely, depending on many factors, notably the reaction temperature and the nature of the reagents and solvent employed. However, provided that the reaction is effected under the preferred conditions outlined above, a period of from 1 to 20 hours, more preferably from 1 to 16 hours, will usually suffice.

After completion of any of the above reactions, the desired compound of the invention can be recovered from the reaction mixture by conventional means depending on the nature of the reaction and the reaction medium. An example of one such technique comprises: neutralizing the reaction mixture appropriately; removing any insoluble material which may.exist in the mixture, for example by filtration; adding a water-immiscible organic solvent; washing with water; and finally distilling off the solvent. The resulting product can, if necessary, be purified by conventional means, for example, by recrystallization, or by the various chromatography techniques, notably preparative thin layer chromatography or column chromatography.

Under the conditions used for removing the hydroxy-protecting group, simultaneous deprotection of a protected carboxy group may take place occasionally.

### (viii) Alkylation and acylation of hydroxy groups

Alkylation of a hydroxy group may be carried out by reacting the hydroxy compound with an alkyl halide in which the alkyl group has 1 or 2 carbon atoms, preferably methyl iodide, ethyl iodide or ethyl bromide, or a dialkyl sulphate (in which each alkyl group has 1 or 2 carbon atoms and may be the same or different, although they are preferably the same), such as dimethyl sulphate or diethyl sulphate.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: amides, such as N,N-dimethylformamide, N,N-dimethylacetamide or N-methylpyrrolidinone; ketones, such as acetone or methyl ethyl ketone; or sulphoxides, such as dimethyl sulphoxide.

The reaction is effected in the presence of a base, the nature of which is not critical, provided that it does not damage the reagents or products. Preferred examples of bases which may be used include alkali metal hydrides, such as sodium hydride, potassium hydride or lithium hydride. The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

Acylation of a hydroxy group may also be carried out by well known methods commonly used in organic synthetic chemistry. For example, it can be carried out by reacting the hydroxy compound with: an alkanoyl halide, containing from 2 to 5 carbon atoms, such as acetyl chloride, propionyl chloride, butyryl bromide or valeryl chloride; a carboxylic acid anhydride or mixed carboxylic acid anhydride, in which the group derived from the or each carboxylic acid contains from 1 to 5, preferably from 2 to 5, carbon atoms, such as a mixed anhydride of formic acid and acetic acid, acetic anhydride, propionic anhydride or valeric anhydride; an alkoxycarbonyl halide, in which the alkoxy group contains from 1 to 4 carbon atoms, such as methoxycarbonyl chloride, methoxycarbonyl bromide, ethoxycarbonyl chloride, propoxycarbonyl chloride or butoxycarbonyl chloride; an arylcarbonyl halide, such as benzoyl chloride or benzoyl bromide; or an alkenoyl chloride containing from 3 to 5 carbon atoms, such as acryloyl chloride, methacryloyl chloride, crotonoyl chloride, 3-methyl-2-butenoyl chloride or 2-methyl-2-butenoyl chloride.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons; such as methylene chloride or chloroform; esters, such as ethyl acetate; and ethers, such as tetrahydrofuran or dioxane. The reaction is effected in the presence of a base, preferably an organic tertiary amine, such as triethylamine, pyridine, diethylisopropylamine or 4-dimethylaminopyridine. The reaction can take place over a wide range of temperatures, and the precise reaction temperature is not critical to the invention. In general, we find it convenient to carry out the reaction at a temperature of from -10°C to 120°C, more preferably from 0°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of either of the above reactions, the desired product can be recovered from the reaction mixture by conventional means. For example, one suitable recovery method is as already described for recovering the product of Step A1.

### Reaction Scheme B:

Compounds of formula (Ia) in which R⁴ represents a hydrogen atom, that is to say compounds of formula (Ib), may also be prepared as shown in the following Reaction Scheme B:

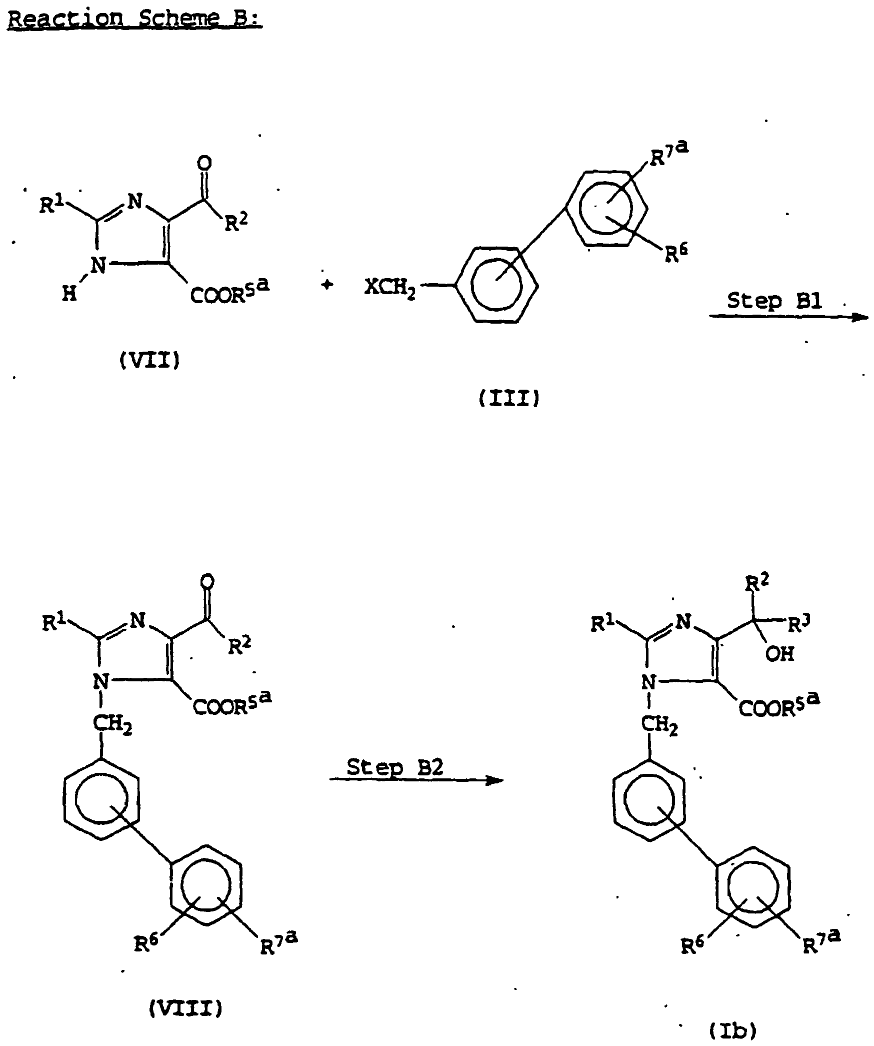

In the above formulae, R¹, R², R³, R^{5a}, R⁶, R^{7a} and X are as defined above, and R^{5a} preferably represents a group other than a hydrogen atom.

In Step B1, an imidazole-5-carboxylate compound of formula (VII) is reacted with a biphenylmethyl compound of formula (III), to give a compound of formula (VIII). This reaction is essentially the same as that of Step A1 in Reaction Scheme A, and may be carried out using the same reagents and reaction conditions.

In Step B2, a compound of formula (Ib) is prepared by reacting a compound of formula (VIII) with a Grignard reagent of formula, R^{3a}-Mg-X (in which R^{3a} represents any of the groups defined above for R³, and X is as defined above).

The reaction of the compound of formula (VIII) with a Grignard reagent is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic, such as hexane or toluene; halogenated hydrocarbons, especially halogenated aliphatic hydrocarbons, such as methylene chloride or 1,2-dichloroethane; and ethers, such as tetrahydrofuran or diethyl ether, of which the ethers and halogenated hydrocarbons are preferred.

The reaction can take place over a wide range of temperatures, and the precise reaction temperature is not critical to the invention. In general, we find it convenient to carry out the reaction at a temperature of from -50°C to 100°C, more preferably from -10°C to 50°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of any of the above reactions, the desired compounds of each reaction can be recovered from the reaction mixture by conventional means. For example, one suitable recovery procedure comprises: mixing the reaction mixture with water or with an aqueous solution of ammonium chloride; and stirring it at room temperature, after which it is extracted with a water-immiscible solvent, such as ethyl acetate. The extract may then be washed with water and dried over a drying agent, such as anhydrous magnesium sulphate, after which the solvent is distilled off; if necessary, the product can be further purified by conventional means, for example, by recrystallization, or by the various chromatography techniques, notably preparative thin layer chromatography or column chromatography.

### Reaction Scheme C:

Compounds of formula (VIII), which are intermediates in reaction Scheme B, can be prepared as shown in Reaction Scheme C:

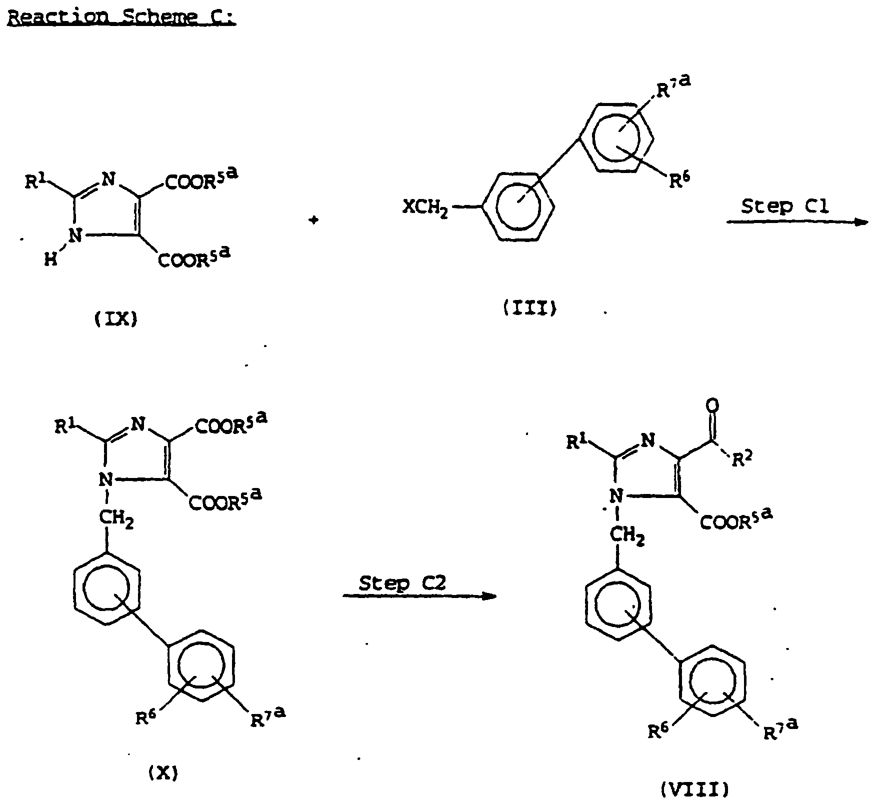

In the above formulae, R¹, R², R^{5a}, R⁶, R^{7a} and X are as defined above, and R^{5a} preferably represents a group other than a hydrogen atom.

In Step C1 of this reaction scheme, an-imidazole-5-carboxylate compound of formula (IX) is reacted with a biphenylmethyl compound of formula (III), to give a dicarboxylate compound of formula (X). This reaction is essentially the same as that described above in Step A1 of Reaction Scheme A, and may be carried out using the same reagents and reaction conditions.

In Step C2 of this reaction scheme, the dicarboxylate compound of formula (X) obtained as shown in Step C1 is reacted with about one equivalent of a Grignard reagent of formula R^{2a}MgX (in which X is as defined above and R^{2a} represents any of the groups defined above for R²)to give the compound of formula (VIII). These reactions are essentially the same as those described above in Step B2 of Reaction Scheme B, and may be carried out using the same reagents and reaction conditions.

After completion of any of the above reactions, the desired product of the reaction can be recovered from the reaction mixture by conventional means. For example, the reaction mixture is mixed with water and with a water-immiscible solvent, such as ethyl acetate. The organic layer is separated, washed with water and dried over a drying agent, such as anhydrous magnesium sulphate; the solvent is then removed by distillation, normally under reduced pressure. If necessary, the product can be further purified by conventional means, for example, by recrystallization, or by the various chromatography techniques, notably preparative thin layer chromatography or column chromatography.

The resulting compound of formula (VIII) may then, if desired, be allowed to react with a Grignard reagent of formula R^{3a}MgX (in which R^{3a} and X are as defined above) according to the method described above in Step B2 of Reaction Scheme B, to give the corresponding compound having a group of formula -CR²(R^{3a})-OH (in which R² and R^{3a} are as described above) at the 4-position of the imidazolyl ring - not shown in the reaction scheme.

### Reaction Scheme D:

In this reaction scheme, a cyano compound of formula (XII) is first prepared, and then this is converted to a compound of formula (I):

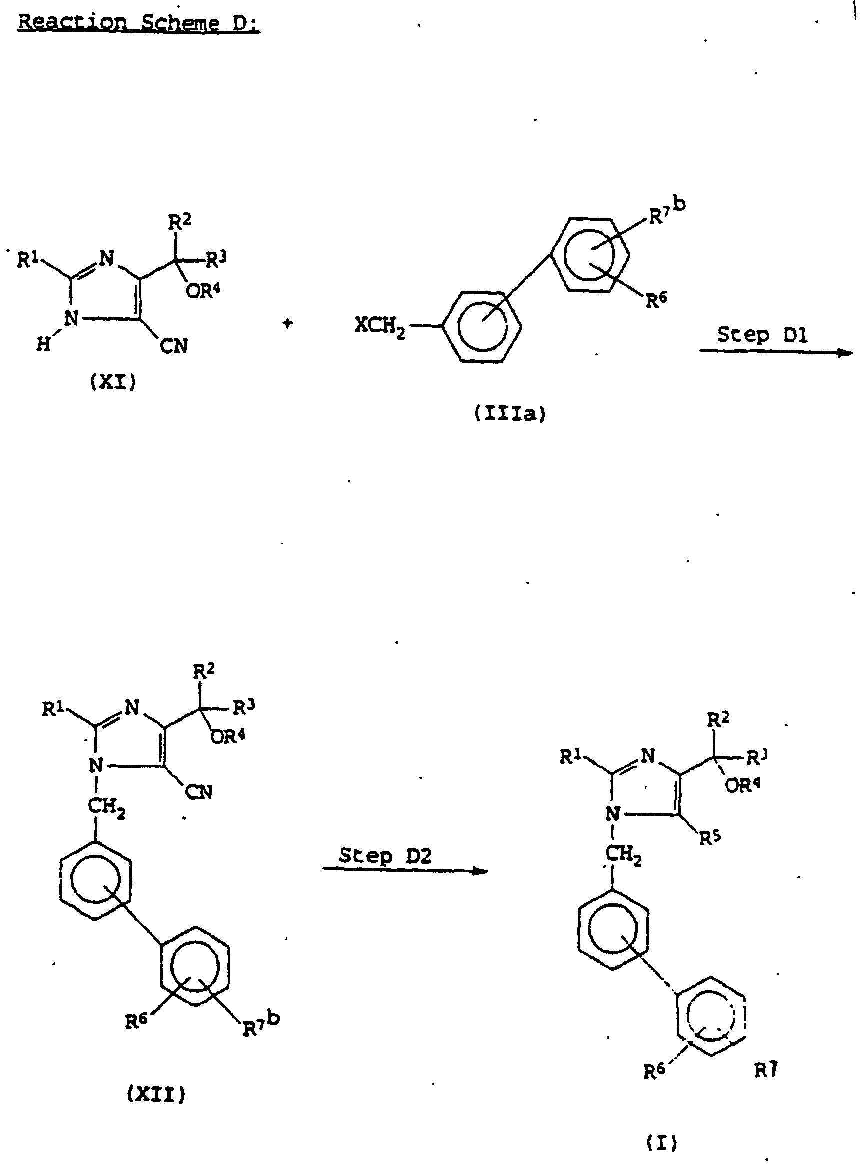

In the above formulae, R¹, R², R³, R⁴, R⁵, R⁶, R⁷ and X are as defined above, and R^{7b} represents a protected carboxy group or a protected tetrazolyl group, both of which may be as previously exemplified in relation to R^{7a}.

In Step D1 of this reaction scheme, an imidazole-5-carbonitrile compound of formula (XI) is reacted with a biphenylmethyl compound of formula (IIIa), to give a compound of formula (XII). This reaction is essentially the same as that described above in Step A1 of Reaction Scheme A, and may be carried out using the same reagents and reaction conditions.

In Step D2, the resulting compound of formula (XII) may be subjected to any one or (in appropriate cases) more of the following reactions:
(ix) converting the cyano group at the 5-position of the imidazole ring to a carboxy group;
(x) converting the cyano group at the 5-position of the imidazole ring to a carbamoyl group;
(xi) removing any carboxy-protecting groups;
(xii) esterifying the carboxy group at the 5-position of the imidazole ring or on the biphenyl group;
(xiii) converting the carboxy group at the 5-position of the imidazole ring to a group of formula -CONR⁸R⁹;
(xiv) removing the tetrazolyl-protecting group;
(xv) where R⁴ represents an acyl group, which can be regarded as a hydroxy-protecting group, removing the protecting group to produce a compound in which R⁴ represents a hydrogen atom; and
(xvi) where R⁴ represents a hydrogen atom, alkylating or acylating this group.

The above reactions may be carried out as follows:

### (ix) Conversion of a cyano group to a carboxy group

The conversion is effected by hydrolysis of the cyano group in the compound of formula (XII) via a carbamoyl group. This reaction is well known in chemical synthesis generally, and may be carried out using any reagent known for this purpose. For example, an alkali metal hydroxide, such as sodium hydroxide, potassium hydroxide or lithium hydroxide.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; alcohols, such as methanol or ethanol; ethers, such as tetrahydrofuran or dioxane; or a mixture of any two or more of these solvents; an aqueous solvent is preferred.

The reaction can take place over a wide range of temperatures, and the precise reaction temperature is not critical to the invention. In general, we find it convenient to carry out the reaction at a temperature of from 0°C to 120°C, more preferably from 20°C to 100°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired product can be recovered from the reaction mixture by conventional means. For example, one suitable recovery procedure comprises: neutralizing the reaction mixture by adding a mineral acid, such as hydrochloric acid; if the desired product of formula (I) precipitates, it can then be recovered by filtration; alternatively, after neutralizing the reaction mixture, the solvent is distilled off and the resulting residue is purified by column chromatography to give the desired product; alternatively, the residue is mixed with water and with a water-immiscible solvent, such as ethyl acetate, and the resulting mixture is extracted with an organic solvent, after which the extract is dried over a drying agent, such as anhydrous magnesium sulphate, and freed from the solvent to give the desired product. If necessary, the product can be further purified by conventional means, for example, by recrystallization, or by the various chromatography techniques, notably preparative thin layer chromatography or column chromatography.

In this reaction, where the starting material is a compound, in which R⁴ represents an acyl group and/or R^{7b} represents an ester group of a primary or secondary alcohol (such as methanol, ethanol or isopropanol), the acyl group of R⁴ and the ester residue of R^{7b} are simultaneously removed.

### (x) Conversion of a cyano group to a carbamoyl, group

In this reaction, a cyano group in the compound of formula (XII) is converted to a carbamoyl group.

The product of this reaction is an intermediate of the previous reaction (ix). Therefore the reaction is carried out in a similar way but under milder conditions than those employed in reaction (ix).

The reaction is carried out by treating the compound of formula (XII) with an alkali, for example: an alkali metal hydroxide, such as lithium hydroxide, sodium hydroxide or potassium hydroxide; or an alkali metal carbonate, such as sodium carbonate or potassium carbonate. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; a mixture of water and an alcohol, such as methanol or ethanol; or a mixture of water and an ether, such as tetrahydrofuran or dioxane.

The reaction can take place over a wide range of temperatures, and the precise reaction temperature is not critical to the invention. In general, we find it convenient to carry out the reaction at a temperature of from 0°C to 100°C, more preferably from 10°C to 80°C. The time required for the reaction may also vary widely, depending on many factors, notably the reaction temperature and the nature of the reagents and solvent employed. However, provided that the reaction is effected under the preferred conditions outlined above, a period of from 0.5 to 24 hours, more preferably from 1 to 8 hours, will usually suffice. The reaction can be accelerated by adding a catalytic amount of hydrogen perioxide.

After completion of the reaction, the reaction product can be recovered from the reaction mixture by conventional means. For example, one suitable recovery procedure comprises: neutralizing the reaction mixture with a mineral acid, such as hydrochloric acid; distilling off the solvent under reduced pressure; adding water to the residue; extracting the mixture with a water-immiscible solvent, such as ethyl acetate; drying the organic extract solution over a drying agent, such as anhydrous magnesium sulphate; and distilling off the solvent. If necessary, the product can be further purified by conventional means, for example, by recrystallization, or by the various chromatography techniques, notably preparative thin layer chromatography or column chromatography.

### (xi) Removing carboxy-protecting groups

This is the same reaction as is involved in reaction (i) of Step A2 of Reaction Scheme A, and may be carried out using the same reagents and reaction conditions.

### (xii) Esterification

This is the same reaction as is involved in reaction (ii) of Step A2, and may be carried out using the same reagents and reaction conditions.

### (xiii) Conversion of a carboxy group to a group of formula -CONR⁸R⁹

This is the same reaction as is involved in reaction (iii) of Step A2, and may be carried out using the same reagents and reaction conditions.

### (xiv) Removal of tetrazolyl-protecting groups

This is the same reaction as is involved in reaction (iv) of Step A2, and may be carried out using the same reagents and reaction conditions.

### (xv) Removing hydroxy-protecting groups

This is the same reaction as is involved in reaction (vii) of Step A2, and may be carried out using the same reagents and reaction conditions.

### (xvi) Alkylation and acylation of hydroxy groups

This is the same reaction as is involved in reaction (viii) of Step A2, and may be carried out using the same reagents and reaction conditions.

### Reaction Scheme E:

In this reaction scheme, a compound of formula (XII) in which R⁴ is hydrogen, that is to say a compound of formula (XV), is prepared from the corresponding compound of formula (XIII) having a ketonic [-C(O)R²] group at the 4-position of the imidazole ring.

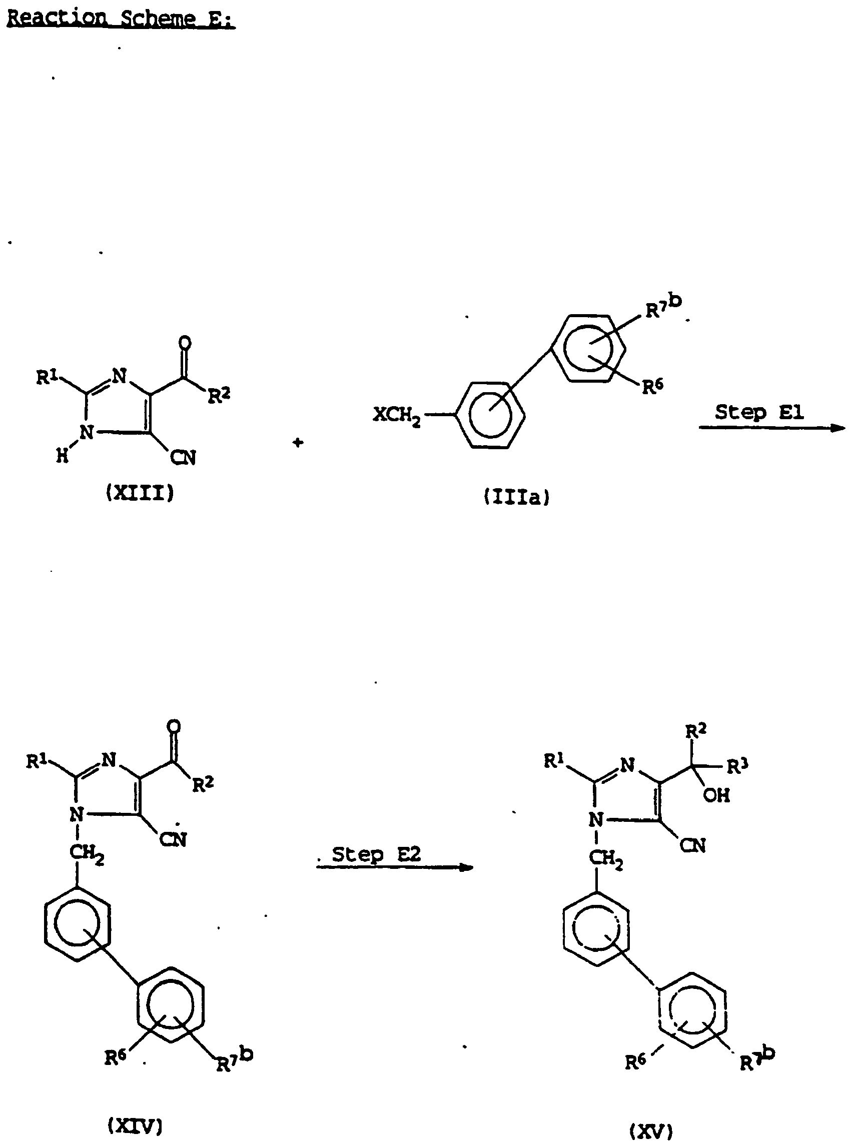

In the above formulae, R¹, R², R³, R⁶, R^{7b} and X are as defined above.

In Step E1 of this reaction scheme, an imidazole-5-carboxylate compound of formula (XIII) is reacted with a biphenylmethyl compound of formula (IIIa), to give a compound of formula (XIV). This reaction is essentially the same as that described above in Step A1 of Reaction Scheme A, and may be carried out using the same reagents and reaction conditions.

The resulting compound of formula (XIV) is then reacted in Step E2 with a Grignard reagent of formula, R^{3a}-Mg-X (in which R^{3a} and X are as defined above). This reaction is essentially the same as that described above in Step B2 of Reaction Scheme B, and may be carried out using the same reagents and reaction conditions. The resulting product may then be recovered and, if desired, further purified, as described in Step B2.

### Reaction Scheme F:

Certain 5-cyanoimidazole derivatives, for use as intermediates in the foregoing reaction schemes may be prepared as illustrated in the following Reaction Scheme F:

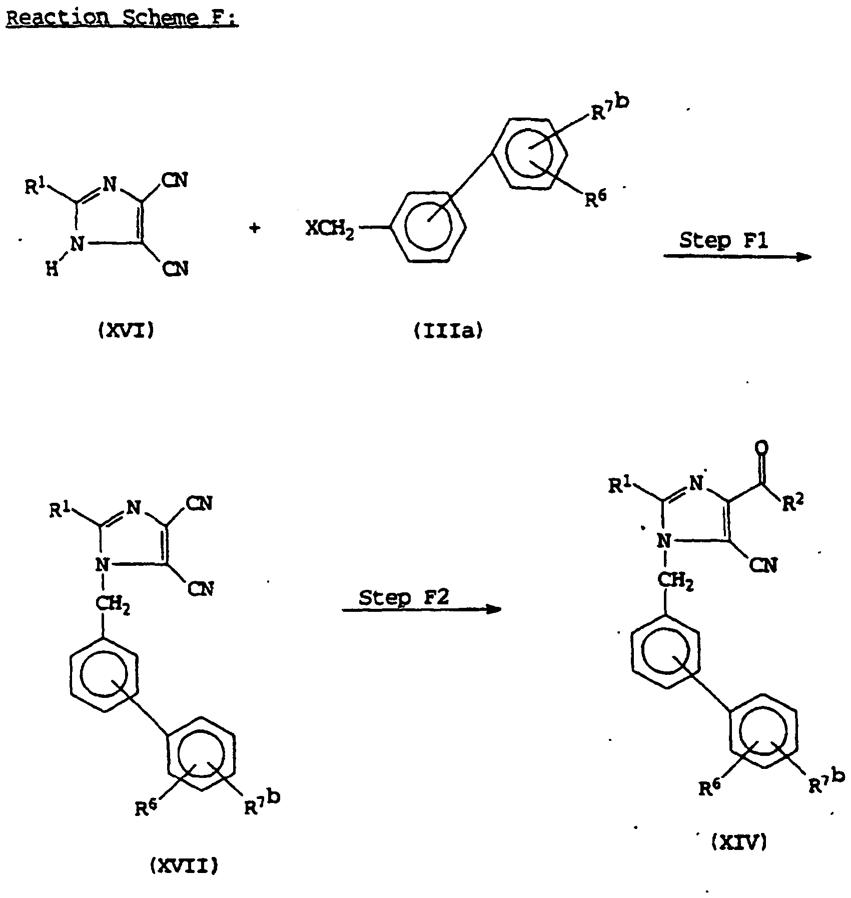

In the above formulae, R¹, R², R⁶, R^{7b} and X are as defined above.

In Step F1 of this reaction scheme, an imidazole-5-carboxylate compound of formula (XVI) is reacted with a biphenylmethyl compound of formula (IIIa), to give a compound of formula (XVII). This reaction is essentially the same as that described above in Step A1 of Reaction Scheme A, and may be carried out using the same reagents and reaction conditions.

Step F2 is essentially the same as Step C2 of Reaction Scheme C, and may be carried out using the same reagents and reaction conditions. The resulting product may then be recovered and, if desired, further purified, as described in Reaction Scheme C.

The preparation of certain of the starting materials used in the above reaction schemes is shown in Reaction Schemes G and H:

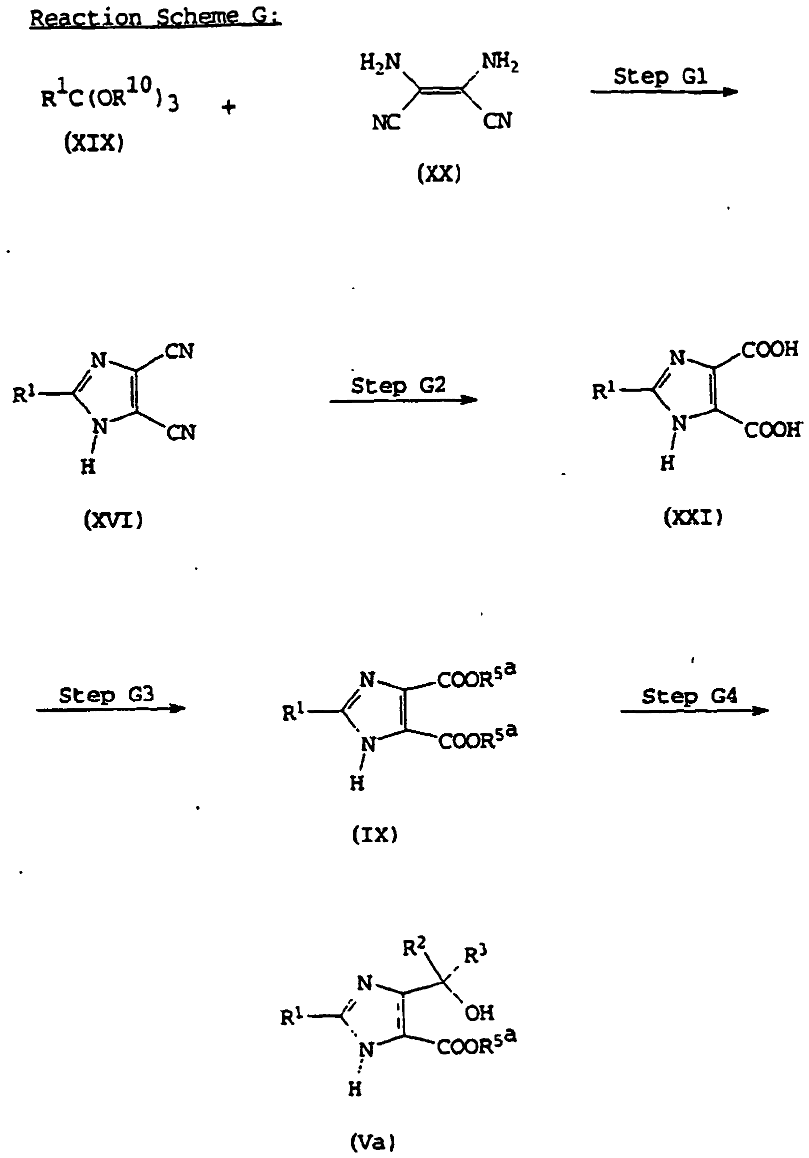

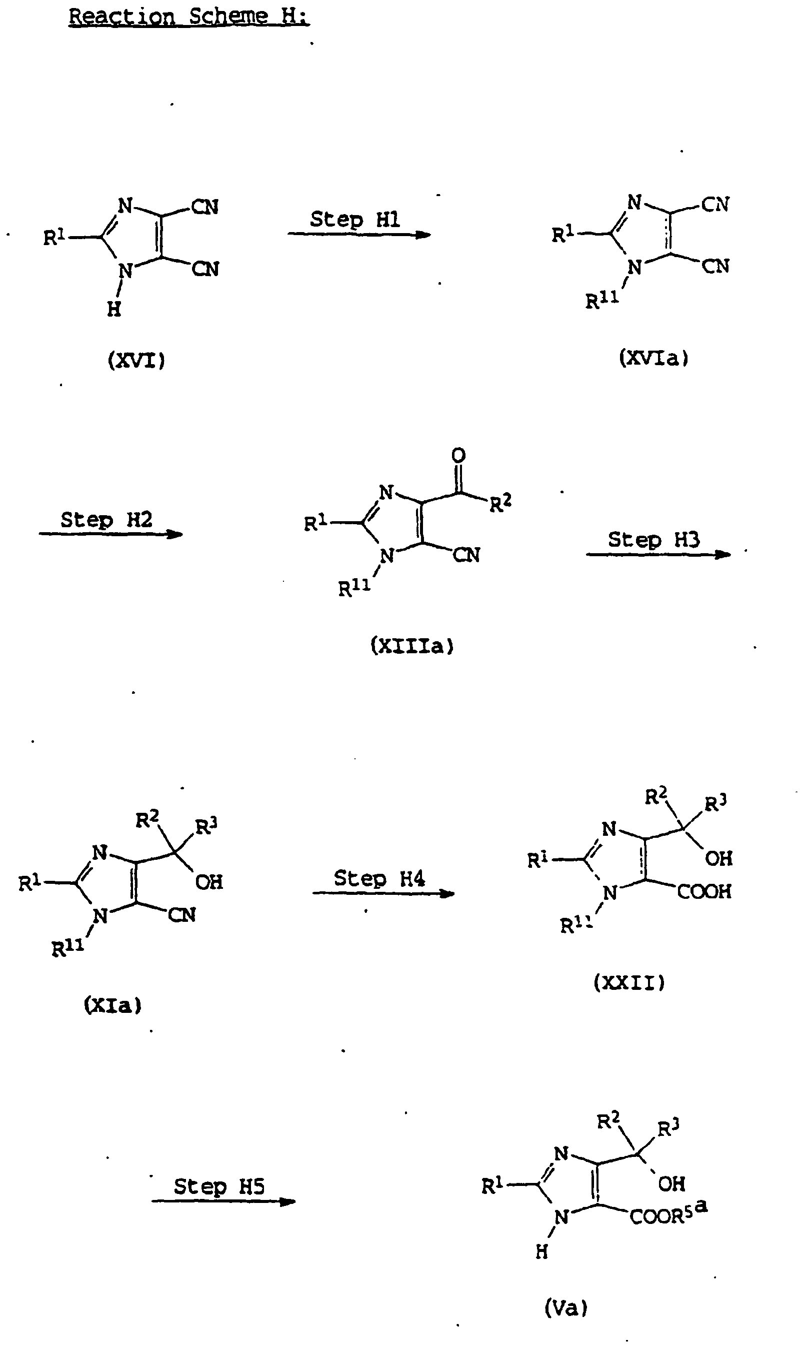

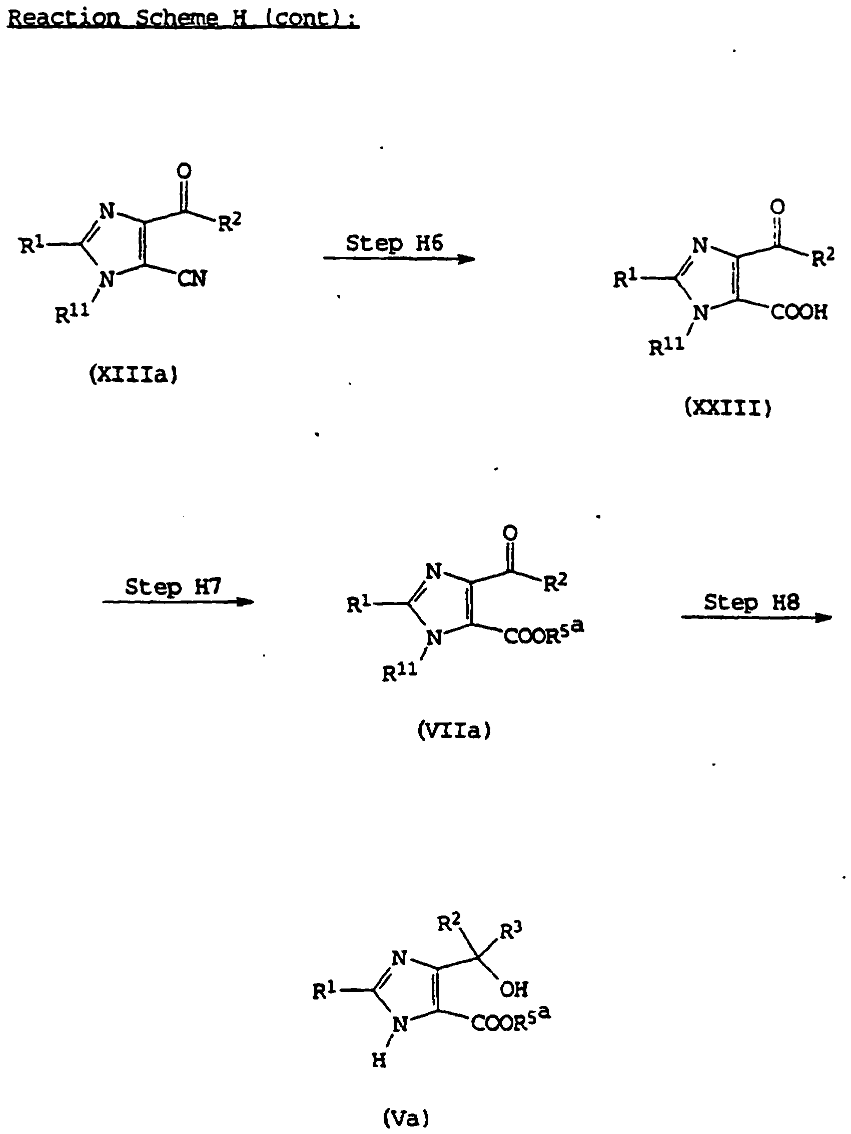

In the above formulae, R¹, R², R³ and R^{5a} are as defined above. R¹⁰ represents an alkyl group containing from 1 to 6 carbon atoms, such as those illustrated in relation to the above alkyl groups having 1 to 6 carbon atoms and is preferably an alkyl group having from 1 to 4 carbon atoms, and more preferably a methyl or ethyl group. R¹¹ represents a hydrogen atom or a imidazolyl-protecting group, for example an aralkyl group, such as a trityl group, a diphenylmethyl group or a benzyl group, or an alkoxy-methyl group in which the alkoxy part has from 1 to 4 carbon atoms, such as a methoxymethyl, ethoxymethyl, propoxymethyl or butoxymethyl group, preferably a trityl group, a benzyl group, a methoxymethyl group or an ethoxymethyl group, more preferably a trityl group.

### Reaction Scheme G:

In this Reaction Scheme G, a compound of formula (V) in which R⁴ represents a hydrogen atom, that is a compound of formula (Va), (IX) or (XVI) (which are starting materials in Reaction Schemes A, C or F, respectively) is prepared. The compound of formula (Va) may then, if desired, be protected, e.g. by alkylation or acylation. These reactions, may be carried out as described in reaction (viii) of Step A2 of Reaction Scheme A, to give the corresponding compound in which R⁴ represents any of the groups represented by R⁴ other than a hydrogen atom.

In Step G1, a compound of formula (XVI) is prepared by reacting an ortho ester compound of formula (XIX) with diaminomaleonitrile, which has the formula (XX). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene, toluene or xylene; halogenated hydrocarbons, especially halogenated aliphatic hydrocarbons, such as 1,2-dichloroethane orcarbon tetrachloride; ethers, such as tetrahydrofuran or dioxane; and nitriles, such as acetonitrile.

The reaction can take place over a wide range of temperatures, and the precise reaction temperature is not critical to the invention. In general, we find it convenient to carry out the reaction at a temperature of from 50°C to 180°C, more preferably from 80°C to 150°C. The time required for the reaction may also vary widely, depending on many factors, notably the reaction temperature and the nature of the reagents and solvent employed. However, provided that the reaction is effected under the preferred conditions outlined above, a period of from 1 to 24 hours, more preferably from 2 to 10 hours, will usually suffice.

The reaction product of formula (XVI) can be recovered by collecting the crystals deposited in the reaction system or by distilling off the solvent. The product can, if necessary, be further purified by conventional means, for example, by recrystallization, or by the various chromatography techniques, notably preparative thin layer chromatography or column chromatography.

Step G2 consists of preparing an imidazole-4,5-dicarboxylic acid compound of formula (XXI) by hydrolyzing the compound of formula (XVI) prepared in Step G1. This reaction may be carried out by heating the compound of formula (XVI) under reflux with an aqueous mineral acid, such as aqueous hydrochloric acid, sulphuric acid or nitric acid, for a period of from 1 to 24 hours (preferably from. 3 to 16 hours). The product of formula (XXI) can be recovered by collecting the crystals deposited in the reaction mixture upon cooling, by filtration or by distilling off the solvent.

Step G3, an optional step, consists of preparing a diester compound of formula (IX) by protecting the carboxy group of the imidazole-4,5-dicarboxylic acid compound of formula (XXI) prepared in Step G2. This reaction may be carried out by reacting the compound (XXI) with a compound of formula R^{5b}-Y, in which R^{5b} and Y are as defined above.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, especially aromatic hydrocarbons, such as benzene or toluene; halogenated hydrocarbons, especially halogenated aliphatic hydrocarbons, such as methylene chloride or chloroform; ethers, such as tetrahydrofuran or dioxane; alcohols, such as methanol, ethanol or t-butanol; amides, such as N,N-dimethylacetamide, N,N-dimethylformamide or N-methyl-2-pyrrolidinone; ketones, such as acetone or methyl ethyl ketone; nitriles, such as acetonitrile; and sulphoxides, such as dimethyl sulphoxide. Of these, we prefer the nitriles, halogenated hydrocarbons or amides.

We also prefer that the reaction should be carried out in the presence of a base, the nature of which is not critical, provided that it does not affect any other parts of the reagents. Preferred examples of bases which may be used include: organic amines, such as triethylamine, N,N-diisopropylethylamine or N-methylmorpholine.

The reaction can take place over a wide range of temperatures, and the precise reaction temperature is not critical to the invention, although the preferred temperature may varies depending upon the nature of the starting material, the solvent and the base. In general, we find it convenient to carry out the reaction at a temperature of from -10°C to 100°C, more preferably -from 0°C to 80°C. The time required for the reaction may also vary widely, depending on many factors, notably the reaction temperature and the nature of the reagents and solvent employed. However, provided that the reaction is effected under the preferred conditions outlined above, a period of from 0.5 to 24 hours, more preferably from 1 to 16 hours, will usually suffice.

After completion of the reaction, the desired compound can be recovered from the reaction mixture by conventional means. For example, after distilling off the solvent, the residue is mixed with water; the mixture is extracted with a water-immiscible organic solvent, such as ethyl acetate; the extract is dried over a drying agent, such as anhydrous magnesium sulphate; and the solvent is distilled off. The product can, if necessary, be further purified by conventional means, for example, by recrystallization, or by the various chromatography techniques, notably preparative thin layer chromatography or column chromatography.

Alternatively, the dicarboxylic acid compound of formula (XXI) may be esterified, to give the diester of formula (IX). The reaction employed for this will, as is well known in the art, depend on the nature of the ester residue R^{5b}.

For example, where the group represented by R^{5b} is a C₁ - C₄ alkyl group or an aralkyl group, such as a benzyl group, the compound of formula (IX) can be prepared by reacting the corresponding dicarboxylic acid with a C₁ - C₄ alcohol, such as methanol, ethanol or propanol, or an aralkyl alcohol, such as a benzyl alcohol, in the presence of an acid catalyst, such as hydrogen chloride or sulphuric acid in an inert solvent (for example: one of the C₁ - C₄ alcohols which may be used as the starting material described above; a halogenated hydrocarbon, such as methylene chloride; or an ether, such as tetrahydrofuran or dioxane) at a temperature of from 0°C to 100°C, preferably from 20°C to 80°C, for a period of from 1 hour to 3 days, preferably from 16 to 24 hours; or by treating the corresponding dicarboxylic acid with a halogenating agent (e.g. phosphorus pentachloride, thionyl chloride or oxalyl chloride) in an inert solvent (for example: a halogenated hydrocarbon, such as methylene chloride; an- ether, such as tetrahydrofuran or dioxane; or an aromatic hydrocarbon, such as benzene or toluene) at about room temperature for a period of from 30 minutes to 5 hours, preferably from 1 to 3 hours, to give the corresponding acyl halide and then reacting this acyl halide with the corresponding alcohol (when the t-butyl ester is prepared, it is desirable to use potassium t-butoxide in place of the alcohol) in an inert solvent (e.g. benzene or methylene chloride) in the presence of a base (e.g. triethylamine) at about room temperature for a period of from 30 minutes to 10 hours.

The desired compound can be recovered from the reaction mixture by conventional means. For example, after distilling off the solvent, the residue is dissolved in water and a water-immiscible organic solvent, such as ethyl acetate, and the resulting solution is neutralized with sodium hydrogencarbonate; the organic layer is then separated and dried over a drying agent, such as anhydrous magnesium sulphate; the solvent is then distilled off to leave the desired product. The product can, if necessary, be further purified by conventional means, for example, by recrystallization, or by the various chromatography techniques, notably preparative thin layer chromatography or column chromatography.

In Step G4, a compound of formula (Va) is prepared by reacting a diester compound of formula (IX) with a Grignard reagent of formula R^{2a}MgX and/or R^{3a}MgX (in which R^{2a}, R^{3a} and X are as defined above).

The reaction is essentially the same as that described above in Step B2 of Reaction Scheme B, and may be carried out using the same reagents and reaction conditions.

### Reaction Scheme H:

These reactions prepare compounds of formulae (XIIIa), (Xla) and (VIIa), in each of -which R¹¹ is a hydrogen atom, that is to say compounds of formulae (XIII), (XI) and (VII), and a compound of formula (Va), which are starting materials used in Reaction Schemes E, D, A and B, respectively.

In Step H1, which is an optional step, a compound of formula (XVIa) is prepared by reacting a dinitrile compound of formula (XVI) with a compound of formula R^{11a}-X (in which X is as defined above and R^{11a} represents any of the groups defined above for R¹¹ other than a hydrogen atom) in the presence of a base.

Examples of suitable bases include: alkali metal hydrides, such as lithium hydride or sodium hydride; alkali metal carbonates, such as sodium carbonate or potassium carbonate; and alkali metal alkoxides, such as sodium methoxide, sodium ethoxide or potassium t-butoxide.

The reaction is normally and preferably effected in the presence of a solvent. There is no particular restriction on the nature of the solvent to be employed, provided that 'it has no adverse effect on the reaction or on the reagents involved and that it can dissolve the reagents, at least to some extent. Examples of suitable solvents include: halogenated hydrocarbons, such as methylene chloride or chloroform; ethers, such as tetrahydrofuran or dioxane; amides, such as dimethylformamide or dimethylacetamide; and ketones, such as acetone or methyl ethyl ketone. The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 1 to 24 hours, more preferably from 3 to 8 hours, will usually suffice.

After completion of the reaction, the desired compound can be recovered from the reaction mixture by conventional means. For example, one suitable recovery procedure comprises: adding water to the reaction mixture; extracting the mixture with a water-miscible organic solvent, such as ethyl acetate; washing the extract with water and drying it over a drying agent, such as anhydrous magnesium sulphate; and finally distilling off the solvent. The product can, if necessary, be further purified by conventional means, for example, by recrystallization, or by the various chromatography techniques, notably preparative thin layer chromatography or column chromatography.

In Step H2, a compound of formula (XIIIa) is prepared by reacting a dinitrile compound of formula (XVIa) with a Grignard reagent of formula R^{2a}MgX, in which R^{2a} and X are as defined above. This reaction is essentially the same as that described above in Step B2 of Reaction Scheme B, and may be carried out using the same reagents and reaction conditions.

An imidazolyl-protecting group of a compound of formula (XIIIa) may optionally be removed by treating the compound of formula (Xllla) in a conventional manner, depending on the nature of the protecting group, to give the compound of formula (XIII).

For example, when the protecting group is a trityl group or an alkoxymethyl group, it may be removed by reacting the protected compound with an acid.

Examples of suitable acids include: inorganic acids, such as hydrochloric acid or sulphuric acid; and organic acids, such as acetic acid, formic acid, trifluoroacetic acid, methanesulphonic acid or p-toluenesulphonic acid.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: ethers, such as tetrahydrofuran or dioxane; alcohols, such as methanol or ethanol; acids, such as acetic acid; water; or a mixture of any two or more of the above solvents.

The reaction can take place over a wide range of temperatures, and the precise reaction temperature is not critical to the invention. In general, we find it convenient to carry out the reaction at a temperature of from 0°C to 120°C, more preferably from 10°C to 100°C. The time required for the reaction may also vary widely, depending on many factors, notably the reaction temperature and the nature of the reagents and solvent employed. However, provided that the reaction is effected under the -preferred conditions outlined above, a period of from 30 minutes to 24 hours, more preferably from 1 to 16 hours, will usually suffice.

After completion of the reaction, the desired compound can be recovered from the reaction mixture by conventional means. For example, one suitable recovery procedure comprises: evaporating the solvent and purifying the product by recrystallization or chromatography; or neutralizing the reaction mixture with a weak base (such as sodium hydrogencarbonate), extracting with a water-immiscible organic solvent, such as ethyl acetate, and evaporating off the solvent. The product can, if necessary, be further purified by conventional means, for example, by recrystallization, or by the various chromatography techniques, notably preparative thin layer chromatography or column chromatography.

When the imidazolyl-protecting group is an aralkyl group, such as a benzyl or diphenylmethyl group, it can be removed by catalytic hydrogenation. The reaction is essentially the same as that described above in reaction (i) of Step A2 of Reaction Scheme A, in which the carboxy-protecting group is an an aralkyl group, and may be carried out using the same reagents and reaction conditions.

In Step H3, the resulting carbonyl compound of formula (XIIIa) is then reacted with a Grignard reagent of formula R^{3a}MgX, in which R^{3a} and X are as defined above, to give the compound of formula (Xla). This reaction is essentially the same as that described above in Step B2 of Reaction Scheme B, and may be carried out using the same reagents and reaction conditions.

If desired, the imidazolyl-protecting group of the compound of formula (Xla) can be removed by essentially the same reaction as that optional reaction described above as Step H2 of Reaction Scheme H, which may be carried out using the same reagents and reaction conditions.

In Step H4, a carboxylic acid compound of formula (XXII) is prepared by hydrolyzing the remaining cyano group at the 5-position of the imidazole ring. The reaction may be carried out using an alkali metal hydroxide, such as sodium hydroxide, potassium hydroxide or lithium hydroxide, in an inert solvent (preferably water.; an alcohol, such as methanol or ethanol; an ether, such as tetrahydrofuran or dioxane; or a mixture of any two or more of the above solvents). The reaction can take-place over a wide range of temperatures; and the precise reaction temperature is not critical to the invention. In general, we find it convenient to carry out the reaction at a temperature of from 0°C to 120°C, more preferably from 20°C to 100°C. The time required for the reaction may also vary widely, depending on many factors, notably the reaction temperature and the nature of the reagents and solvent employed. However, provided that the reaction is effected under the preferred conditions outlined above, a period of from 0.5 to 24 hours, more preferably from 1 to 16 hours, will usually suffice. After completion of the reaction, the reaction product can be recovered by conventional means. For example, the reaction mixture is neutralized by adding a mineral acid, such as hydrochloric acid; if the desired compound of formula (XXII) appears as a precipitate in the reaction medium, it can be collected by filtration. Alternatively, the desired compound can be recovered as follows: after neutralizing the reaction mixture, the solvent is distilled off and the residue is subjected to column chromatography; alternatively, the residue may be mixed with water and a water-immiscible organic solvent and extracted with the organic solvent, after which the extract is dried over a drying agent, such as anhydrous magnesium sulphate, and the solvent is distilled off to leave the desired product. The product can, if necessary, be further purified by conventional means, for example, by recrystallization, or by the various chromatography techniques, notably preparative thin layer chromatography or column chromatography.

In Step H5, an optional step, a compound of formula (Va) is prepared by esterification of the carboxylic acid compound of formula (XXII), optionally followed by deprotection of the imidazolyl group. This esterification reaction is essentially the same as that described above in reaction (ii) of Step A2 of Reaction Scheme A, and the optional deprotection is essentially the same as Step H2 of Reaction Scheme H, and each may be carried out using the same reagents and reaction conditions.

In Step H6, a compound of formula (XXIII) is prepared by hydrolysing a compound of formula (Xllla). This reaction is essentially the same as that described above in Step H4 of Reaction Scheme H, and may be carried out using the same reagents and reaction conditions.

In Step H7, a compound of formula (VIIa) is prepared by esterification of the compound of formula (XXIII). This reaction is essentially the same as that described above in Step H5 of Reaction Scheme H, and may be carried out using the same reagents and reaction conditions.

If desired, the imidazolyl-protecting group of the compound of formula (VIIa) can be removed by essentially the same reaction as that optional reaction described above as Step H2 of Reaction Scheme H, which may be carried out using the same reagents and reaction conditions.

In Step H8, a compound of formula (Va) is prepared by reacting a compound of formula (VIIa) with a Grignard reagent, and then optionally deprotecting the imidazolyl group. This reaction is essentially the same as that described above in Step B2 of Reaction Scheme B, and the optional deprotection is essentially the same as Step H2 of Reaction Scheme H, and each may be carried out using the same reagents and reaction conditions.

The compounds of the present invention can form salts. There is no particular restriction on the nature of these salts, provided that, where they are intended for therapeutic use, they are pharmaceutically acceptable. Where they are intended for non-therapeutic uses, e.g. as intermediates in the preparation of other, and possibly more active, compounds, even this restriction does not apply. The compounds of the present invention can form salts with bases. Examples of such salts include: salts with an alkali metal, such as sodium, potassium or lithium; salts with an alkaline earth metal, such as barium or calcium; salts with another metal, such as magnesium or aluminium; organic base salts, such as a salt with dicyclohexylamine, guanidine or triethylamine; and salts with a basic amino acid, such as lysine or arginine. Also, the compound of the present invention contains a basic group in its molecule and can therefore form acid addition salts. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid or citric acid; and salts with amino acids, such as glutamic acid or aspartic acid. The compounds of the present invention can be converted to a pharmaceutically acceptable salt by treatment with an acid or a base by conventional means, as is well known in the art.

The compounds of the present invention exhibit an excellent inhibitory effect against the elevation of blood pressure induced by angiotensin II and are therefore extremely useful for prevention or treatment of circulatory diseases as a hypotensive drug or a therapeutic drug for heart diseases.

Their biological activity was determined by the following experiment.

### Evaluation of All receptor blocking activity by Inhibition of pressor response to angiotensin II

The biological activity of each compound was assessed by determining the dose required to inhibit the pressor response to intravenous angiotensin II by fifty percent (ID₅₀) in rats. Male Wister-Imamichi rats, each weighing 300 to 400 g, were anesthesized by intraperitoneal injection of 100 mg/Kg of sodium thiobutabarbital [Inactin (trade name)] and two cannulae were inserted: one into the femoral artery for measuring blood pressure and the other into the femoral vein for injecting drugs. Fifty ng/kg of angiotension II were intravenously administered at intervals of about 10 minutes, and the elevation of blood pressure (normally about 50 mmHg) was observed. After constant pressor responses to angiotensin II were obtained, a test compound was intravenously administered. Two minutes later, angiotension II was again injected, and the inhibitory effect of the test compound was estimated. The percent inhibitions of the pressor response to angiotensin II by progressive increase of the test compound was used to calculate the value of ID₅₀. Angiotensin II was used in this test dissolved in 0.5 % bovine serum albumin (BSA) and the test compounds were dissolved in 100% dimethyl sulphoxide (DMSO). Table 7 shows the ID₅₀ values thus determined.

In addition to the compounds of the invention (which are identified hereafter by the number of the one of the following Examples which illustrates their preparation), we also carried out the same experiment using a prior art compound (identified in the Table as "compound A"), which is 2-[4-(2-butyl-5-chloro-4-chloromethylimidazol-1-ylmethyl) phenyl]benzoic acid, which is disclosed in Example 118 of European Patent Publication No. 253 310.

**Table 7**

| Test compound (Compound of Example No.) | ID50 (mg/kg, i.v.) |
|---|---|
| 10 | 0.066 |
| 17 | 0.056 |
| 19 | 0.008 |
| 22 | 0.017 |
| 23 | 0.043 |
| 24 | 0.014 |
| 50 | 0.22 |
| 69 | 0.019 |
| A | 3.3 |

The compounds of the present invention can be administered, for example, orally in the form of tablets, capsules, granules, powders, syrups or the like, or parenterally by injection, suppository or the like. These pharmaceutical preparations can be produced in the conventional manner using the adjuvants generally known in the art, such as excipients, binders, disintegrating agents, lubricants, stabilizers, corrigents and the like. Although the dosage may vary depending upon the symptoms and age of the patient, the nature and severity of the disease or disorder and the route and manner of administration, in the case of oral administration to an adult human patient, the compounds of the present invention may normally be administered at a total daily dose of from 1 to 1000 mg, preferably from 5 to 300 mg, either in a single dose, or in divided doses, for example two orthree times a day; in the case of intravenous injection, a dose of from 0.1 to 100 mg, preferably from 0.5 to 30 mg, may be administered between one and three times a day.

The invention is further illustrated by the following Examples, which demonstrate the preparation of various of the compounds of the invention. The preparation of certain starting materials used in these Examples is shown in the subsequent Preparations.

### EXAMPLE 10

### 2-Butyl-1-[(2'-carboxybiphenyl-4-yl)methyl]-4-[(1-hydroxy-1-methyl)ethyl]imidazole-5-carboxylic acid (Compound No. 1-31)

### 10(a) 1-[(2'-t-Butoxycarbonylbiphenyl-4-yl)methyl]-2- butyl-5-cyano-4-[(1-hydroxy-1-methyl)ethyl]- imidazole

48 mg of sodium hydride (as a 55% w/w dispersion in mineral oil) were added, at room temperature and under an atmosphere of nitrogen, whilst stirring, to a solution of 207 mg of 2-butyl-5-cyano-4-[(1-hydroxy-1-methyl)ethyl] imidazole (prepared as described in Preparation 7) in 10 ml of N,_N-dimethylacetamide, and the resulting mixture was stirred for 30 minutes; at the end of this time, 347 mg of t-butyl 4'-bromomethyl-biphenyl-2-carboxylate were added. The reaction mixture was then stirred at room temperature for 2 hours, after which it was poured into a mixture of ice and sodium chloride and extracted with ethyl acetate. The extract was dried over anhydrous magnesium sulphate and concentrated by evaporation under reduced pressure, to give an oily crude product. This was purified by column chromatography through silica gel, using a 1 : 1 by volume mixture of hexane and ethyl acetate as the eluent, to give 462 mg of the title compound.

Nuclear Magnetic Resonance Spectrum (CDCl₃) δ ppm:
0.90 (3H, triplet, J = 7 Hz);
1.1 - 2.1 (4H, multiplet);
1.21 (9H, singlet);
1.61 (6H, singlet);
2.70 (2H, triplet, J = 7 Hz);
3.40 (1 H, singlet);
5.22 (2H, singlet);
7.0-8.0 (8H, multiplet).

### 10(b) 2-Butyl-1-[(2'-carboxybiphenyl-4-yl)methyl]-5-cyano-4-[(1-hydroxy-1-methyl)ethyl]imidazole

A solution of 462 mg of 1-[(2'-t-butoxycarbonyl-biphenyl-4-yl)methyl]-2-butyl-5-cyano-4-[(1-hydroxy-1-methyl) ethyl]imidazole [prepared as described in step (a) above] in 10 ml of a 4 N solution of hydrogen chloride in dioxane was allowed to stand overnight at room temperature. At the end of this time, the reaction mixture was concentrated by evaporation under reduced pressure, and the concentrate was dissolved in methylene chloride. The precipitate which deposited was collected by filtration and dried, to give 457 mg of the hydrochloride of the title compound as a colourless powder, melting at 209 - 210°C.

Nuclear Magnetic Resonance Spectrum (hexadeuterated dimethyl sulphoxide) δ ppm:
0.85 (3H, triplet, J = 7 Hz);
1.0 - 1.8 (4H, multiplet);
1.58 (6H, singlet);
3.00 (2H, triplet, J = 7 Hz);
5.51 (2H, singlet);
7.1 - 8.0 (8H, multiplet).

### 10(c) 2-Butyl-1-[(2'-carboxybiphenyl-4-yl)methyl]-4-[(1-hydroxy-1-methyl)ethyl]imidazole-5-carboxylic acid

A solution of 314 mg of 2-butyl-1-[(2'-carboxy-biphenyl-4-yl)methyl]-5-cyano-4-[(1-hydroxy-1-methyl)-ethyl] imidazole hydrochloride [prepared as described in step (b) above] in an aqueous solution of 460 mg of sodium hydroxide in 5 ml of water was stirred in an oil bath kept at 100°C for 5 hours. At the end of this time, the reaction mixture was cooled, and its pH was adjusted to a value of 3 to 4 by the addition of 1 N aqueous hydrochloric acid. The colourless precipitate which deposited was collected by filtration, washed with water and dried over anhydrous magnesium sulphate, to give 244 mg of the title compound, melting at 139 - 141°C.

Nuclear Magnetic Resonance Spectrum (hexadeuterated dimethyl sulphoxide) δ ppm:
0.86 (3H, triplet, J = 7 Hz);
1.0 - 1.9 (4H, multiplet);
1.60 (6H, singlet);
2.66 (2H, triplet, J = 7 Hz);
5.70 (2H, singlet);
6.9 - 7.9 (8H, multiplet).

### EXAMPLE 13

### Ethyl 1-[(2'-t-butoxycarbonylbiphenyl-4-yl)methyl]-2-butyl-4-(1-hydroxy-1-methylethyl)imidazole-5-carboxylate (Compound No. 1-118)

Following a procedure similar to that described in Preparation 46(a), but using 0.92 g of ethyl 2-butyl-4-(1-hydroxy-1-methylethyl)imidazole-5-carboxylate (prepared as described in Preparation 8) and 1.28 g of t-butyl 4'-bromomethylbiphenyl-2-carboxylate, 1.23 g of the title compound were obtained as crystals, melting at 92 - 93°C.

Nuclear Magnetic Resonance Spectrum (CDCl₃) δ ppm:
0.90 (3H, triplet, J = 7 Hz);
1.23 (3H, triplet, J = 7 Hz);
1.26 (9H, singlet);
1.2 - 2.05 (4H, multiplet);
1.65 (6H, singlet);
2.69 (2H, triplet, J = 7 Hz);
4.24 (2H, quartet, J = 7 Hz);
5.52 (2H, singlet);
5.73 (1H, singlet);
6.88 - 7.9 (8H, multiplet).

### EXAMPLE 14

### Ethyl 2-butyl-1-[(2'-carboxybiphenyl-4-yl)methyl]-4-(1-hydroxy-1-methylethyl)imidazole-5-carboxylate (Compound No. 1-32)

Following a procedure similar to that described in Preparation 44, but using 0.50 g of ethyl 1-[(2'-t-butoxycarbonylbiphenyl-4-yl)methyl]-2-butyl-4-(1-hydroxy-1-methylethyl)imidazole-5-carboxylate (prepared as described in Example 13) and a 4 N solution of hydrogen chloride in dioxane, 0.45 g of the hydrochloride of the title compound was obtained as an amorphous powder, melting at above 80°C (with softening).

Nuclear Magnetic Resonance Spectrum (hexadeuterated dimethyl sulphoxide) δ ppm:
0.82 (3H, triplet, J = 7 Hz);
1.14 (3H, triplet, J = 7 Hz);
1.2 - 1.35 (2H, multiplet);
1.41 - 1.55 (2H, multiplet);
1.60 (6H, singlet);
3.00 (2H, triplet, J = 7 Hz);
4.21 (2H, quartet, J = 7 Hz);
5.63 (2H, singlet);
7.14 - 7.75 (8H, multiplet).

### EXAMPLE 15

### Ethyl 1-[(2'-t-butoxycarbonylbiphenyl-4-yl)methyl]-4-(1-hydroxy-1-methylethyl)-2-propylimidazole-5-carboxylate (Compound No. 1-119)

Following a procedure similar to that described in Preparation 46(a), but using 0.845 g of ethyl 4-(1-hydroxy-1-methylethyl)-2-propylimidazole-5-carboxylate (prepared as described in Preparation 9) and 1.22 g of t-butyl 4'-bromomethylbiphenyl-2-carboxylate, 1.31 g of the title compound were obtained as a gum. This compound was allowed to stand at room temperature, which caused it to crystallize. It was then recrystallized from a mixture of diisopropyl ether and hexane, to give pure title compound, melting at 90 - 91 °C.

Nuclear Magnetic Resonance Spectrum (CDCl₃) δ ppm:
0.97 (3H, triplet, J = 7 Hz);
1.23 (3H, triplet, J = 7 Hz);
1.25 (9H, singlet);
1.60 (6H, singlet);
1.82 (2H, sextet, J = 7 Hz);
2.67 (2H, triplet, J = 7 Hz);
4.24 (2H, quartet, J = 7 Hz);
5.51 (2H, singlet);
5.72 (1H, singlet);
6.87 - 7.85 (8H, multiplet).

### EXAMPLE 16

### Ethyl 1-[(2'-carboxybiphenyl-4-yl)methyl]-4-(1-hydroxy-1-methylethyl)-2-propylimidazole-5-carboxylate (Compound No. 1-50)

Following a procedure similar to that described in Preparation 44, but using 0.80 g of ethyl 1-[(2'-t-butoxycarbonylbiphenyl-4-yl)methyl]-4-(1-hydroxy-1-methyl-ethyl)-2-propylimidazole-5-carboxylate (prepared as described in Example 15) and a 4 N solution of hydrogen chloride in dioxane, 0.67 g of the hydrochloride of the title compound was obtained as an amorphous powder.

Nuclear Magnetic Resonance Spectrum (hexadeuterated dimethyl sulphoxide) δ ppm:
0.88 (3H, triplet, J = 7 Hz);
1.14 (3H, triplet, J = 7 Hz);
1.50 - 1.65 (2H, multiplet);
1.60 (6H, singlet);
3.00 (2H, triplet, J = 7 Hz);
4.20 (2H, quartet, J = 7 Hz);
5.63 (2H, singlet);
7.13 - 7.75 (8H, multiplet).

### EXAMPLE 17

### 1-[(2'-Carboxybiphenyl-4-yl)methyl]-4-(1-hydroxy-1-methylethyl)-2-propylimidazole-5-carboxylic acid (Compound No. 1-49)

A solution of 0.20 g of ethyl 1-[(2'-carboxy-biphenyl-4-yl) methyl] -4-(1-hydroxy-1-methylethyl)-2-propylimidazole-5-carboxylate hydrochloride (prepared as described in Example 16) in an aqueous solution of 84 mg of lithium hydroxide monohydrate in 5 ml of water was stirred at room temperature for 6 hours. At the end of this time, 2 ml of 1 N aqueous hydrochloric acid were added dropwise to the reaction mixture, and the resulting precipitate was collected by filtration, to give 0.17 g of the title compound, melting at 176 - 179°C (with decomposition).

Nuclear Magnetic Resonance Spectrum (hexadeuterated dimethyl sulphoxide) δ ppm:
0.88 (3H, triplet, J = 7 Hz);
1.5 - 1.65 (2H, multiplet);
1.56 (6H, singlet);
2.66 (2H, triplet, J = 7 Hz);
5.69 (2H, singlet);
7.03 - 7.72 (8H, multiplet).

### EXAMPLE 18

### Ethyl 4-(1-hydroxy-1-methylethyl)-2-propyl-1-{4-[2-(tetrazol-5-yl)phenyl]phenyl}methylimidazole-5-carboxylate (Compound No. 2-7)

### 18(a) Ethyl 4-(1-hydroxy-1-methylethyl)-2-propyl-1-{4-[2-(trityltetrazol-5-yl)phenyl]phenyl}methyl-imidazole-5-carboxylate

48 mg of sodium hydride (as a 55% w/w dispersion in mineral oil) were added to a solution of 0.26 g of ethyl 4-(1-hydroxy-1-methylethyl)-2-propylimidazole-5- carboxylate (prepared as described in Preparation 9) in 5 ml of N, N-dimethylformamide, and the resulting mixture was stirred at room temperature for 30 minutes. A solution of 0.72 g of 4-[2-(trityltetrazol-5-yl)phenyl]- benzyl bromide in 5 ml of N,N-dimethylformamide was then added, and the reaction mixture was stirred at room temperature for 2 hours and then at 60°C for 4 hours. At the end of this time, it was dissolved in ethyl acetate and the solution was washed three times with water. The solution was then dried over anhydrous sodium sulphate, after which it was freed from the solvent by distillation. The residue was purified by column chromatography through silica gel, using a 1 : 1 by volume mixture of hexane and ethyl acetate as the eluent, to give 0.62 g of the title compound as an amorphous solid. This was crystallized from diisopropyl ether, to give the title compound as crystals, melting at 167 - 168°C (with decomposition).

Nuclear Magnetic Resonance Spectrum (CDCl₃) δ ppm:
0.88 (3H, triplet, J = 7 Hz);
1.08 (3H, triplet, J = 7 Hz);
1.5 - 1.8 (2H, multiplet);
1.64 (6H, singlet);
2.52 (2H, triplet, J = 8 Hz);
4.12 (2H, quartet, J = 7 Hz);
5.38 (2H, singlet);
5.78 (1H, singlet);
6.7 - 7.6 (22H, multiplet);
7.8 - 8.1 (1H, multiplet).

### 18(b) Ethyl 4-(1-hydroxy-1-methylethyl)-2-propyl-1-{4-[2-(tetrazol-5-yl)phenyl]phenyl}methylimidazole-5-carboxylate

A solution of 0.50 g of ethyl 4-(1-hydroxy-1-methylethyl)-2-propyl-1-{4-[2-(trityltetrazol-5-yl)phenyl]phenyl} methylimidazole-5-carboxylate [prepared as described in Example 18(a)] dissolved in 5 ml of a 4 N solution of hydrogen chloride in dioxane was allowed to stand overnight at room temperature, after which the reaction mixture was concentrated by evaporation under reduced pressure. The resulting residue was triturated with diisopropyl ether and then washed with diisopropyl ether, to give 0.34 g of the hydrochloride of the title compound, melting at 100 - 103°C.

Nuclear Magnetic Resonance Spectrum (CD₃OD) δ ppm:
0.97 (3H, triplet, J = 7 Hz);
1.24 (3H, triplet, J = 7 Hz);
1.50 - 1.65 (2H, multiplet);
1.70 (6H, singlet);
3.00 (2H, triplet, J = 8 Hz);
4.30 (2H, quartet, J = 7 Hz);
5.70 (2H, singlet);
6.9 - 7.8 (8H, multiplet).

### EXAMPLE 19

### 4-(1-Hydroxy-1-methylethyl)-2-propyl-1-{4-[2-(tetrazol-5-yl)phenyl]phenyl}methylimidazole-5-carboxylic acid (Compound No. 2-1)

3.65 ml of a 1 N aqueous solution of sodium hydroxide were added to a solution of 0.31 g of ethyl 4-(1-hydroxy-1-methylethyl)-2-propyl-1-{4-[2-(tetrazol-5-yl)phenyl]phenyl}methylimidazole-5-carboxylate hydrochloride [prepared as described in Example 18(b)] in 6 ml of methanol, and the resulting mixture was allowed to stand overnight at room temperature. At the end of this time, the reaction mixture was concentrated by evaporation under reduced pressure to remove the methanol. The concentrate was diluted with water and its pH was adjusted to a value of 3 by the addition of dilute hydrochloric acid, after which it was extracted with ethyl acetate. The organic extract was dried over anhydrous sodium sulphate and then concentrated by evaporation under reduced pressure. The resulting residue was triturated with diisopropyl ether, to give 0.15 g of the title compound, melting at 166 - 169°C.

Nuclear Magnetic Resonance Spectrum (hexadeuterated dimethyl sulphoxide) δ ppm:
0.85 (3H, triplet, J = 7.5 Hz);
1.54 (6H, singlet);
1.4 - 1.6 (2H, multiplet);
2.58 (2H, triplet, J = 8 Hz);
5.64 (2H, singlet);
6.94 (2H, doublet, J-= 8.5 Hz);
7.06 (-2H, doublet, J = 8.5 Hz);
7.5 - 7'.7 (4H, multiplet).

### EXAMPLE 20

### Pivaloyloxymethyl 4-(1-hydroxy-1-methylethyl)-2-propyl-1-{4-[2-(tetrazol-5-yl)phenyl]phenyl}methylimidazole-5-carboxylate (Compound No. 2-15)

### 20(a) Pivaloyloxymethyl 4-(1-hydroxy-1-methylethyl)-2-propyl-1-{4-[2-(trityltetrazol-5-yl)phenyl]-phenyl} methylimidazole-5-carboxylate

5.30 ml of a 1 N aqueous solution of sodium hydroxide, followed by 5 ml of tetrahydrofuran, were added to a solution of 0.76 g of ethyl 4-(1-hydroxy-1- methylethyl)-2-propyl-1-{4-[2-(trityltetrazol-5-yl)-phenyl]phenyl}methylimidazole-5-carboxylate [prepared as described in Example 18(a)] in 30 ml of methanol, and the resulting mixture was stirred at room temperature for 8 hours. The reaction mixture was then concentrated by evaporation under reduced pressure to remove the methanol and tetrahydrofuran. Water was added to the concentrate, and the pH of the mixture was adjusted to a value of 4 by the addition of dilute hydrochloric acid, whilst ice-cooling. The mixture was then extracted with ethyl acetate. The extract was dried over anhydrous sodium sulphate and concentrated by evaporation to dryness. The residue was dissolved in 10 ml of dimethylacetamide, and 0.23 g of potassium carbonate and 0.13 ml of pivaloyloxymethyl chloride were added to the resulting solution. The mixture was then stirred at 50°C for 4 hours, after which 0.06 ml of pivaloyloxy-methyl chloride was added, and the mixture was stirred for a further 2 hours. The reaction mixture was then diluted with ethyl acetate, and washed three times with water. The organic layer was separated, dried over anhydrous sodium sulphate and concentrated by evaporation under reduced pressure. The concentrate was purified by column chromatography through silica gel, using a 1 : 1 by volume mixture of hexane and ethyl acetate as the eluent, to give 0.23 g of the title compound as an amorphous solid.

Nuclear Magnetic Resonance Spectrum (CDCl₃) δ ppm:
0.86 (3H, triplet, J = 7 Hz);
1.12 (9H, singlet);
1.62 (6H, singlet);
1.4 - 1.9 (2H, multiplet);
2.51 (2H, triplet, J = 7 Hz);
5.37 (1H, broad singlet);
5.40 (2H, singlet);
5.72 (2H, singlet);
6.6 - 8.1 (23H, multiplet).

### 20(b) Pivaloyloxymethyl 4-(1-hydroxy-1-methylethyl)-2-propyl-1-{4-[2-(tetrazol-5-yl)phenyl]phenyl}-methylimidazole-5-carboxylate

5 ml of a 4 N solution of hydrogen chloride in dioxane were added to 0.20 g of pivaloyloxymethyl 4-(1-hydroxy-1-methylethyl)-2-propyl-1-{4-[2-(trityl-tetrazol-5-yl)phenyl]phenyl}methylimidazole-5- carboxylate [prepared as described in Example 20(a)], and the resulting mixture was allowed to stand at room temperature overnight. At the end of this time, the reaction mixture was concentrated to dryness by evaporation under reduced pressure. The resulting residue was triturated with diisopropyl ether to induce crystallization and give 0.13 g of the hydrochloride of the title compound as crystals, melting at 104 - 107°C.

Nuclear Magnetic Resonance Spectrum (hexadeuterated dimethyl sulphoxide) δ ppm:
0.84 (3H, triplet, J = 7.5 Hz);
1.09 (9H, singlet);
1.35 - 1.50 (2H, multiplet);
1.56 (6H, singlet);
2.88 (2H, triplet, J = 8 Hz);
5.58 (2H, singlet);
5.85 (2H, singlet);
7.05 (2H, doublet, J = 8.5 Hz);
7.10 (2H, doublet, J = 8.5 Hz);
7.5 - 7.7 (4H, multiplet).

### EXAMPLE 21

### 2-Butyl-4-(1-ethyl-1-hydroxypropyl)-1-{4-[2-(tetrazol-5-yl)phenyl]phenyl}methylimidazole-5-carboxylic acid (Compound No. 2-40)

### 21(a) Ethyl 2-butyl-4-(1-ethyl-1-hydroxypropyl)-1-{4-[2-(trityltetrazol-5-yl)phenyl]phenyl}- methylimidazole-5-carboxylate

Following a procedure similar to that described in Example 18(a), but using 0.75 g of ethyl 2-butyl-4-(1-ethyl-1-hydroxypropyl)imidazole-5-carboxylate (prepared as described in Preparation 13), 0.12 g of sodium hydride (as a 55% w/w dispersion in mineral oil) and 1.51.g of 4-[2-(trityltetrazol-5-yl)phenyl]benzyl bromide, there were obtained 1.05 g of the title compound as an amorphous solid. Nuclear Magnetic Resonance Spectrum (CDCl₃) δ ppm:
0.83 (6H, triplet, J = 7.5 Hz);
0.85 (3H, triplet, J = 6 Hz);
1.11 (3H, triplet, J = 7 Hz);
1.23 - 1.32 (2H, multiplet);
1.56 - 1.65 (2H, multiplet);
1.80 - 1.89 (2H, multiplet);
2.03 - 2.14 (2H, multiplet);
2.55 (2H, triplet, J = 8 Hz);
4.12 (2H, quartet, J = 7.5 Hz);
5.37 (2H, singlet);
5.64 (1H, broad singlet);
6.70 (2H, doublet, J = 8.5 Hz);
6.9 - 7.0 (6H, multiplet);
7.10 (2H, doublet, J = 8.5 Hz);
7.2 - 7.4 (10H, multiplet);
7.4 - 7.5 (2H, multiplet);
7.85 - 7.90 (1H, multiplet).

### 21(b) 2-Butyl-4-(1-ethyl-1-hydroxypropyl)-1-{4-[2-(tetrazol-5-yl)phenyl]phenyl}methylimidazole-5-carboxylic acid

1.71 ml of 1 N aqueous hydrochloric acid were added to a solution of 0.65 g of ethyl 2-butyl-4-(1-ethyl-1-hydroxypropyl)-1-{4-[2-(trityltetrazol-5-yl)phenyl]-phenyl}methylimidazole-5-carboxylate [prepared as described in step. (a) above] in 10 ml of methanol, and the resulting mixture was allowed to stand overnight at room temperature. At the end of this time, the solvent was removed by distillation under reduced pressure, and the concentrate was again dissolved in 10 ml of methanol. The resulting solution was mixed with 4.28 ml of a 1 N aqueous solution of sodium hydroxide and then allowed to stand overnight at room temperature. The reaction mixture was then concentrated by evaporation under reduced pressure to remove the methanol. The pH of the concentrate was adjusted to a value of 3 by the addition of dilute aqueous hydrochloric acid, and the crystals which precipitated were collected by filtration. The crystals thus obtained were suspended in diisopropyl ether and then again collected by filtration and dried to give 0.35 g of the title compound, melting at 181 - 183°C.

Nuclear Magnetic Resonance Spectrum (hexadeuterated dimethyl sulphoxide) δ ppm:
0.74 (6H, triplet, J = 7.5 Hz);
0.79 (3H, triplet, J = 7.5 Hz);
1.1 - 1.3 (2H, multiplet);
1.40 - 1.55 (2H, multiplet);
1.67 - 1.80 (2H, multiplet);
.1.90 - 2.05 (2H, multiplet);
2.59 (2H, triplet, J = 7.5 Hz);
5.67 (2H, singlet);
6.88 (2H, doublet, J = 8.5 Hz);
7.05 (2H, doublet, J = 8.5.Hz);
7.5 - 7.7 (4H, multiplet).

### EXAMPLE 22

### 2-Butyl-4-(1-hydroxy-1-methylethyl)-1-{4-[2-(tetrazol-5-yl)phenyl]phenyl}methylimidazole-5-carboxylic acid (Compound No. 2-2)

### 22(a) Ethyl 2-butyl-4-(1-hydroxy-1-methylethyl)-1-{4-[2-(trityltetrazol-5-yl)phenyl]phenyl}methyl-imidazole-5-carboxylate

Following a procedure similar to that described in Example 18(a), but using 0.26 g of ethyl 2-butyl-4-(1-hydroxy-1-methylethyl)imidazole-5-carboxylate (prepared as described in Preparation 8), 45.5 mg of sodium hydride (as a 55% w/w dispersion in mineral oil) and 0.63 g of 4-[2-(trityltetrazol-5-yl)phenyl]benzyl bromide, 0.28 g of the title compound were obtained as an oil.

Nuclear Magnetic Resonance Spectrum (CDCl3) δ ppm:
0.85 (3H, triplet, J = 7 Hz);
1.09 (3H, triplet, J = 7 Hz);
1.64 (6H, singlet);
1.3 - 1.8 (4H, multiplet);
2.56 (2H, triplet, J = 8 Hz);
4.14 (2H, quartet, J = 7 Hz);
5.38 (2H, singlet);
5.78 (1H, singlet);
6.6 - 7.6 (22H, multiplet);
7.7 - 8.1 (1H, multiplet).

### 22(b) 2-Butyl-4-(1-hydroxy-1-methylethyl)-1-{4-[2-(tetrazol-5-yl)phenyl]phenyl}methylimidazole-5-carboxylic acid

Following a procedure similar to that described in Example 21 (b), 78 mg of the title compound, melting at 138 - 141°C, were obtained by treating 0.28 g of ethyl 2-butyl-4-(1-hydroxy-1-methylethyl)-1-{4-[2-(trityl-tetrazol-5-yl)phenyl]phenyl}methylimidazole-5- carboxylate [prepared as described in step (a) above] with 0.42 ml of 1 N aqueous hydrochloric acid and then treating the product with 1.70 ml of a 1 N aqueous solution of sodium hydroxide.

Nuclear Magnetic Resonance Spectrum (hexadeuterated dimethyl sulphoxide) δ ppm:
0.81 (3H, triplet, J = 7.5 Hz);
1.15 - 1.35 (2H, multiplet);
1.4 - 1.6 (2H, multiplet);
1.53 (6H, singlet);
2.58 (2H, triplet, J = 8.5 Hz);
5.64 (2H, singlet);
6.94 (2H, doublet, J = 8.5 Hz);
7.06 (2H, doublet, J = 8.5 Hz);
7.15 - 7.70 (4H, multiplet).

### EXAMPLE 23

### 2-Butyl-4-(1-hydroxy-1-methylpropyl)-1-{4-[2-(tetrazol-5-yl)phenyl]phenyl}methylimidazole-5-carboxylic acid (Compound No. 2-38)

### 23(a) 2-Butyl-5-cyano-4-(1-hydroxy-1-methylpropyl)-1-{4-[2-(trityltetrazol-5-yl)phenyl]phenyl}- methylimidazole

Following a procedure similar to that described in Example 18(a), but using 465 mg of 2-butyl-5-cyano-4-(1-hydroxy-1-methylpropyl)imidazole (prepared as described in Preparation 19), 92 mg of sodium hydride (as a 55% w/w dispersion in mineral oil) and 1.11 g of 4-[2-(trityltetrazol-5-yl)phenyl]benzyl bromide, 1.00 g of the title compound was obtained as a gum.

Nuclear Magnetic Resonance Spectrum (CDCl₃) δ ppm:
0.86 (3H, triplet, J = 7.5 Hz);
0.87 (3H, triplet, J = 7 Hz);
1.21 - 1.34 (2H, multiplet);
1.54 - 1.66 (2H, multiplet);
1.60 (3H, singlet);
1.82 - 1.97 (2H, multiplet);
2.51 (2H, triplet, J = 7.5 Hz);
3.22 (1H, singlet);
5.04 (2H, singlet);
6.87 - 7.52 (22H, multiplet);
7.93 - 7.96 (1H, multiplet).

### 23(b) 2-Butyl-5-cyano-4-(1-hydroxy-1-methylpropyl)-1-{4-[2-(tetrazol-5-yl)phenyl]phenyl}methyl-imidazole

A mixture of 1.00 g of 2-butyl-5-cyano-4-(1-hydroxy-1-methylpropyl)-1-{4-[2-(trityltetrazol-5-yl)phenyl]-phenyl}methylimidazole [prepared as described in step (a) above] and 25 ml of 20% v/v aqueous acetic acid was stirred at 60°C for 2 hours, and then the solvent was removed by distillation under reduced pressure. The residual water and acetic acid were removed as a toluene azeotrope by distillation under reduced pressure, and the resulting residue was purified by column chromatography through silica gel, using mixtures of methanol and methylene chloride ranging from 1 : 9 to 1 : 4 by volume as the eluent, to give 0.65 g of the title compound as a glass.

Nuclear Magnetic Resonance Spectrum (CDCl₃) δ ppm:
0.83 (3H, triplet, J = 7 Hz);
0.88 (3H, triplet, J = 7 Hz);
1.23 - 1.37 (2H, multiplet);
1.57 (3H, singlet);
1.55 - 1.70 (2H, multiplet);
1.82 - 1.89 (2H, multiplet);
2.64 (2H, triplet, J = 7 Hz);
5.12 (2H, singlet);
6.9 - 7.1 (4H, multiplet);
7.29 - 7.60 (3H, multiplet);
7.87 (1H, doublet, J = 7.5 Hz).

### 23(c) 2-Butyl-4-(1-hydroxy-1-methylpropyl)-1-{4-[2-(tetrazol-5-yl)phenyl]phenyl}methylimidazole-5-carboxylic acid

A mixture of 360 mg of 2-butyl-5-cyano-4-(1-hydroxy-1-methylpropyl)-1-{4-[2-(tetrazol-5-yl)phenyl]-phenyl} methylimidazole [prepared as described in step (b) above], 266 mg of lithium hydroxide monohydrate and 3.6 ml of water was stirred in an oil bath kept at 115°C for 16 hours. At the end of this time, the reaction mixture was cooled and 6.4 ml of 1 N aqueous hydrochloric acid were added to the mixture, whilst ice-cooling. The crystals which precipitated were collected by filtration, to give 302 mg of the title compound, melting at 152 - 154°C.

Nuclear Magnetic Resonance Spectrum (hexadeuterated dimethyl sulphoxide) δ ppm:
0.79 (3H, triplet, J = 7 Hz);
0.82 (3H, triplet, J = 7 Hz);
1.20 - 1.34 (2H, multiplet);
1.44 - 1.55 (2H, multiplet);
1.55 (3H, singlet);
1.71 - 1.95 (2H, multiplet);
2.62 (2H, triplet, J = 7.5 Hz);
5.68 (2H, AB-quartet, Δδ=0.10 ppm, J = 17 Hz);
6.86 - 7.10 (4H, multiplet);
7.53 - 7.72 (4H, multiplet).

### EXAMPLE 24

### 4-(1-Hydroxy-1-methylpropyl)-2-propyl-1-{4-[2-(tetrazol-5-yl)phenyl]phenyl}methylimidazole-5-carboxylic acid (Compound No. 2-37)

### 24(a) 5-Cyano-4-(1-hydroxy-1-methylpropyl)-2-propyl-1-{4-[2-(trityltetrazol-5-yl)phenyl]phenyl}-methylimidazole

Following a procedure similar to that described in Example 18(a), but using 380 mg of 5-cyano-4-(1-hydroxy-1-methylpropyl)-2-propylimidazole (prepared as described in Preparation 20), 88 mg of sodium hydride (as a 55% w/ w dispersion in mineral oil) and 1.07 g of 4-[2-(trityltetrazol-5-yl)phenyl]benzyl bromide, 0.97 g of the title compound were obtained as an amorphous solid.

Nuclear Magnetic Resonance Spectrum (CDCl₃) δ ppm:
0.86 (3H, triplet, J = 8 Hz);
0.87 (3H, triplet, J = 7.5 Hz);
1.60 (3H, singlet);
1.60 - 1.75 (2H, multiplet);
1.80 - 2.00 (2H, multiplet);
2.48 (2H, triplet, J = 8 Hz);
5.04 (2H, singlet);
6.88 (2H, doublet, J = 8.5 Hz);
6.9 - 7.0 (4H, multiplet);
7.14 (2H, doublet, J = 8.5 Hz);
7.2 - 7.4 (14H, multiplet);
7.45 - 7.55 (1H, multiplet).

### 24(b) 5-Cyano-4-(1-hydroxy-1-methylpropyl)-2-propyl-1- {4-[2-(tetrazol-5-yl)phenyl]phenyl}methyl-imidazole

Following a procedure similar to that described in Example 23(b), 0.32 g of the title compound were obtained as crystals, melting at 141 - 145°C, by treating 0.51 g of 5-cyano-4-(1-hydroxy-1-methylpropyl)- 2-propyl-1-{4-[2-(trityltetrazol-5-yl)phenyl]phenyl}-methylimidazole [prepared as described in step (a) above] with 75% v/v aqueous acetic acid.

Nuclear Magnetic Resonance Spectrum (CD₃OD) δ ppm:
0.84 (3H, triplet, J = 8 Hz);
.0.90 (3H, triplet, J = 8.5 Hz);
1.52 (3H, singlet);
1.5 - 1.7 (2H, multiplet);
1.75 - 1.90 (2H, multiplet);
2.65 (2H, triplet, J = 8 Hz);
5.27 (2H, singlet);
7.03 (2H, doublet, J = 8.5 Hz);
7.14 (2H, doublet, J = 8.5 Hz);
7.45 - 7.63 (4H, multiplet).

### 24(c) 4-(1-Hydroxy-1-methylpropyl)-2-propyl-1-{4-[2-(tetrazol-5-yl)phenyl]phenyl}methylimidazole-5-carboxylic acid

Following a procedure similar to that described in Example 23(c), 0.14 g of the title compound were obtained as a powder, melting at 174 - 177°C, by treating 0.19 g of 5-cyano-4-(1-hydroxy-1-methylpropyl)- 2-propyl-1-{4-[2-(tetrazol-5-yl)phenyl]phenyl}methyl-imidazole [prepared as described in step (b) above] with 0.15 g of lithium hydroxide monohydrate.

Nuclear Magnetic Resonance Spectrum (CD₃OD) δ ppm:
0.88 (3H, triplet, J = 7.5 Hz);
0.94 (3H, triplet, J = 7.5 Hz);
1.50 - 1.65 (2H, multiplet);
1.63 (3H, singlet);
1.85 - 2.05 (2H, multiplet);
2.76 (2H, triplet, J = 7.5 Hz);
5.80 (2H, AB-quartet, Δδ=0.14 ppm, J = 16.5 Hz);
7.01 (2H, doublet, J = 8.5 Hz);
7.11 (2H, doublet, J = 8.5 Hz);
7.48 - 7.75 (4H, multiplet).

### EXAMPLE 25

### Pivaloyloxymethyl 1-[(2'-carboxybiphenyl-4-yl)methyl]-4-(1-hydroxy-1-methylethyl)-2-propylimidazole-5-carboxylate (Compound No. 3-1)

### 25(a) Ethyl 1-[(2'-t-butoxycarbonylbiphenyl-4-yl)- methyl]-4-(1-hydroxy-1-methylethyl)-2-propyl-imidazole-5-carboxylate

3.00 g of potassium t-butoxide were added, whilst ice-cooling, to a solution of 6 g of ethyl 4-(1-hydroxy-1-methylethyl)-2-propylimidazole-5-carboxylate (prepared as described in Preparation 9) in 40 ml of N,N-dimethylacetamide, and the resulting mixture was stirred for 10 minutes, after which a solution of 9.00 g of t-butyl 4'-bromomethylbiphenyl-2-carboxylate in 40 ml of N,N-dimethylacetamide was added. After the reaction mixture had been stirred at room temperature for 1 hour and then at 50°C for 2 hours, it was mixed with water and extracted with ethyl acetate. The extract was dried over anhydrous magnesium sulphate, and the solvent was removed by distillation under reduced pressure, after which the residue was purified by column chromatography through silica gel, using a 1 : 1 by volume mixture of hexane and ethyl acetate as the eluent, to give 11.6 g-of the title compound as a solid, softening at above 85°C.

Nuclear Magnetic Resonance Spectrum (CDCl₃) δ ppm:
0.97 (3H, triplet, J = 7 Hz);
1.23 (3H, triplet, J = 7 Hz);
1.25 (9H, singlet);
1.60 (6H, singlet);
1.82 (2H, sextet, J = 7 Hz);
2.67 (2H, triplet, J = 7 Hz);
4.24 (2H, quartet, J = 7 Hz) ;
5.51 (2H, singlet);
5.72 (1H, singlet);
6.87 - 7.85 (8H, multiplet).

### 25 (b 1-[(2'-t-Butoxycarbonylbiphenyl-4-yl)methyl]-4-(1-hydroxy-1-methylethyl)-2-propylimidazole-5-carboxylic acid

A solution of 4.8 g of lithium hydroxide monohydrate in 100 ml of water was added to a solution of 11.6 g of ethyl 1-[(2'-t-butoxycarbonylbiphenyl-4-yl)methyl]-4- (1-hydroxy-1-methylethyl)-2-propylimidazole-5-carboxylate [prepared as described in step (a) above] in 60 ml of dioxane, and the resulting mixture was stirred at room temperature for 16 hours. The dioxane was removed by distillation under reduced pressure, and then the concentrate was mixed with ice-water and with ethyl acetate, after which 114 ml of 1 N aqueous hydrochloric acid were added. The ethyl acetate layer was separated, dried over anhydrous magnesium sulphate and freed from the solvent by distillation under reduced pressure. The crystalline residue was triturated in diisopropyl ether and collected by filtration to give 9.09 g of the title compound, melting at 155 - 157°C.

Nuclear Magnetic Resonance Spectrum (CDCl₃) δ ppm:
0.85 (3H, triplet, J = 7.5 Hz);
1.23 (9H, singlet);
1.53 - 1.65 (2H, multiplet);
1.65 (6H, singlet);
2.91 (3H, triplet, J = 7.5 Hz);
5.90 (2H, singlet);
7.09 (2H, doublet, J = 8 Hz);
7.21 - 7.48 (5H, multiplet);
7.75 (1H, doublet, J = 9 Hz).

### 25(c) Pivaloyloxymethyl 1-[(2'-t-butoxycarbonylbiphenyl-4-yl)methyl]-4-(1-hydroxy-1-methylethyl)-2-propylimidazole-5-carboxylate

2.13 ml of chloromethyl pivalate and 3.99'g of potassium carbonate were added to a solution of 6 g of 1-[(2'-t-butoxycarbonylbiphenyl-4-yl)methyl]-4-(1-hydroxy-1-methylethyl)-2-propylimidazole-5-carboxylic acid [prepared as described in step (b) above] in 70 ml of N,N-dimethylacetamide, and the resulting mixture was stirred at room temperature for 1 hour and then at 50°C for 2 hours. At the end of this time, the reaction mixture was mixed with ethyl acetate and water. The ethyl acetate layer was separated and dried over anhydrous magnesium sulphate, after which the solvent was removed by distillation under reduced pressure. The resulting residue was purified by column chromatography through silica gel, using a 1 : 1 by volume mixture of ethyl acetate and hexane as the eluent, to give 6.80 g of the title compound as crystals, melting at 106 - 107°C.

Nuclear Magnetic Resonance Spectrum (CDCl₃) δ ppm:
1.07 (3H, triplet, J = 7 Hz);
1.25 (9H, singlet);
1.32 (9H, singlet);
1.71 (6H, singlet);
1.79 - 1.90 (2H, multiplet);
2.75 (2H, triplet, J = 8 Hz);
5.50 (1H, singlet);
5.59 (2H, singlet);
5.92 (2H, singlet);
7.05 (2H, doublet, J = 8 Hz);
7.34 - 7.56 (5H, multiplet);
7.85 (1H, doublet, J = 7 Hz).

### 25 (d) Pivaloyloxymethyl 1-[(2'-carboxybiphenyl-4-yl)-methyl]-4-(1-hydroxy-1-methylethyl)-2-propylimidazole-5-carboxylate

A mixture of 6.6 g of pivaloyloxymethyl 1-[(2'-t-butoxycarbonylbiphenyl-4-yl)methyl]-4-(1-hydroxy-1-methylethyl)-2-propylimidazole-5-carboxylate [prepared as described in step (c) above] and 57 ml of a 4 N solution of hydrogen chloride in dioxane was stirred at room temperature for 4 hours. At the end of this time, the reaction mixture was concentrated by evaporation under reduced pressure, and the residue was triturated with ethyl acetate to crystallize it, giving 6.52 g of the title compound as the hydrochloride, melting at 170 - 173°C.

Nuclear Magnetic Resonance Spectrum (hexadeuterated dimethyl sulphoxide) δ ppm:
0.87 (3H, triplet, J = 7 Hz);
1.10 (9H, singlet);
1.45 - 1.60 (2H, multiplet);
1.58 (6H, singlet);
2.96 (2H, triplet, J = 7.5 Hz);
5.65 (2H, singlet);
5.87 (2H, singlet);
7.17 (2H, doublet, J = 8 Hz);
7.33 (2H, doublet, J = 8 Hz) ;
7.43 - 7.60 (3H, multiplet) ;
7.74 (1H, doublet, J = 8 Hz).

### EXAMPLE 26

### Isopropoxycarbonyloxymethyl 1-[(2'-carboxybiphenyl-4-yl)methyl]-4-(1-hydroxy-1-methylethyl)-2-propylimidazole-5-carboxylate (Compound No. 3-13)

### 26(a) Isopropoxycarbonyloxymethyl 1-[(2'-t-butoxycarbonyl-biphenyl-4-yl)methyl]-4-(1-hydroxy-1-methylethyl)-2-propylimidazole-5-carboxylate

Following a procedure similar to that described in Example 25(c), 0.58 g of the title compound was obtained as crystals, melting at 85 - 87°C, by stirring a mixture comprising 0.50 g of 1-[(2'-t-butoxycarbonylbiphenyl-4-yl)methyl]-4-(1-hydroxy-1-methylethyl)-2-propylimidazole-5-carboxylic acid [prepared as described in Example 25(b)], 0.19 g of isopropoxycarbonyloxymethyl chloride and 0.33 g of potassium carbonate in 6 ml of N,N-dimethylacetamide at room temperature for 3 hours.

Nuclear Magnetic Resonance Spectrum (CDCl₃) δ ppm:
0.99 (3H, triplet, J = 7 Hz);
1.23 (9H, singlet);
1.29 (6H, doublet, J = 6 Hz);
1.63 (6H, singlet);
1.70 - 1.85 (2H, multiplet);
2.68 (2H, triplet, J = 8 Hz);
4.89 (1H, quintet, J = 6 Hz);
5.38 (1H, singlet);
5.51 (2H, singlet);
5.82 (2H, singlet);
6.97 (2H, doublet, J = 8 Hz);
7.26 - 7.48 (5H, multiplet);
7.77 (1H, doublet, J = 8 Hz).

### 26(b) Isopropoxycarbonyloxymethyl 1-[(2'-carboxybiphenyl-4-yl)methyl]-4-(1-hydroxy-1-methylethyl)-2-propylimidazole-5-carboxylate

Following a procedure similar to that described in Example 25(d), 0.36 g of the hydrochloride of the title compound was obtained as an amorphous powder, melting at 153 - 155°C, by treating 0.46 g of isopropoxycarbonyloxymethyl 1-[(2'-t-butoxycarbonylbiphenyl-4-yl)methyl]-4-(1-hydroxy-1-methylethyl)-2-propylimidazole-5-carboxylate [prepared as described in step (a) above] with a 4 N solution of hydrogen chloride in dioxane.

Nuclear Magnetic Resonance Spectrum (CDCl₃) δ ppm:
0.98 (3H, triplet, J = 7 Hz);
1.29 (6H, doublet, J = 6 Hz);
1.50 - 1.65 (2H, multiplet);
1.76 (6H, singlet);
3.13 (2H, triplet, J = 7 Hz);
4.90 (1H, quintet, J = 6 Hz);
5.55 (2H, singlet);
5.82 (2H, singlet);
7.02 (2H, doublet, J = 6.5 Hz);
7.21 - 7.57 (5H, multiplet);
7.96 (1H, doublet, J = 8 Hz).

### EXAMPLE 27

### Ethoxycarbonyloxymethyl 1-[(2'-carboxybiphenyl-4-yl)methyl]-4-(1-hydroxy-1-methylethyl)-2-propylimidazole-5-carboxylate (Compound No. 3-9)

### 27(a) Ethoxycarbonyloxymethyl 1-[(2'-t-butoxycarbonylbiphenyl-4-yl)methyl]-4-(1-hydroxy-1-methylethyl)-2-propylimidazole-5-carboxylate

Following a procedure similar to that described in Example 25(c), 0.69 g of the title compound was obtained as an oil from 0.55 g of 1-[(2'-t-butoxycarbonyl-biphenyl-4-yl)methyl]-4-(1-hydroxy-1-methylethyl)-2-propylimidazole-5-carboxylic acid [prepared as described in Example 25(b)], 0.30 g of ethoxycarbonyloxymethyl chloride and 0.50 g of potassium carbonate.

Nuclear Magnetic Resonance Spectrum (CDCl₃) δ ppm:
0.99 (3H, triplet, J = 7 Hz);
1.23 (9H, singlet);
1.29 (3H, triplet, J = 7 Hz);
1:64 (6H, singlet);
1.74 - 1.85 (2H, multiplet);
2.69 (2H, triplet, J = 7.5 Hz);
4.21 (2H, quartet, J = 7 Hz);
5.39 (1H, singlet);
5.52 (2H, singlet);
5.83 (2H, singlet);
6.97 (2H, doublet, J = 8 Hz);
7.26 - 7.51 (5H, multiplet);
7.77 (1H, doublet, J = 6.5 Hz).

### 27(b) Ethoxycarbonyloxymethyl 1-[(2'-carboxybiphenyl-4-yl)methyl]-4-(1-hydroxy-1-methylethyl)-2-propyl-imidazole-5-carboxylate

Following a procedure similar to that described in Example 25(d), 0.48 g of the hydrochloride of the title compound was obtained as an amorphous powder, softening at above 70°C, by treating 0.69 g of ethoxycarbonyloxymethyl 1-[(2'-t-butoxycarbonylbiphenyl-4-yl)methyl]-4-(1-hydroxy-1-methylethyl)-2-propylimidazole-5- carboxylate [prepared as described in step (a) above] with a 4 N solution of hydrogen chloride in dioxane.

Nuclear Magnetic Resonance Spectrum (hexadeuterated dimethyl sulphoxide) δ ppm:
0.88 (3H, triplet, J = 7 Hz);
1.19 (3H, triplet, J = 7 Hz);
1.5 - 1.65 (2H, multiplet);
1.59 (6H, singlet);
2.96 (2H, triplet, J = 7.5 Hz);
4.15 (2H, quartet, J = 7 Hz);
5.64 (2H, singlet);
5.84 (2H, singlet);
7.18 (2H, doublet, J = 8 Hz);
7.32 - 7.61 (5H, multiplet);
7.74 (1H, doublet, J = 7 Hz).

### EXAMPLE 28

### 1-(Isopropoxycarbonyloxy)ethyl 1-[(2'-carboxybiphenyl4-yl)methyl]-4-(1-hydroxy-1-methylethyl)-2-propyl-imidazole-5-carboxylate (Compound No. 3-14)

### 28(a) 1-(Isopropoxycarbonyloxy)ethyl 1-[(2'-t-butoxy-carbonylbiphenyl-4-yl)methyl]-4-(1-hydroxy-1-methylethyl)-2-propylimidazole-5-carboxylate

Following a procedure similar to that described in Example 25(c), 0.60 g of the title compound was obtained as a gum by stirring 0.50 g of 1-[(2'-t-butoxycarbonyl-biphenyl-4-yl)methyl]-4-(1-hydroxy-1-methylethyl)-2-propylimidazole-5-carboxylic acid [prepared as described in Example 25(b)] and 0.21 g of 1-(isopropoxycarbonyl-oxy)ethyl chloride with a solution of 0.40 g of potassium carbonate in 6 ml of N,N-dimethylacetamide at 60°C for 16 hours.

Nuclear Magnetic Resonance Spectrum (CDCl₃) δ ppm:
0.97 (3H, triplet, J = 7.5 Hz);
1.26 (9H, singlet);
1.27 (6H, doublet of doublets, J = 4.5 & 6 Hz);
1.42 (3H, doublet, J = 5.5 Hz);
1.64 (6H, doublet, J = 3 Hz);
1.75 - 1.80 (2H, multiplet);
2.65 (2H, doublet, J = 7.5 Hz);
4.86 (1H, quintet, J = 6 Hz);
5.50 (2H, singlet);
6.90 (1H, quartet, J = 5.5 Hz);
6.97 (2H, doublet, J= 8.5 Hz);
7.26 - 7.50 (5H, multiplet);
7.78 (1H, doublet, J = 8 Hz).

### 28(b) 1-(Isopropoxycarbonyloxy)ethyl 1-[(2'-carboxybiphenyl-4-yl)methyl]-4-(1-hydroxy-1-methylethyl)-2-propylimidazole-5-carboxylate

Following a procedure similar to that described in Example 25(d), 0.41 g of the hydrochloride of the title compound, melting at 94 - 96°C, was obtained as an amorphous powder by treating 0.60 g of 1-(isopropoxy-carbonyloxy)ethyl 1-[(2'-t-butoxycarbonylbiphenyl-4-yl)-methyl]-4-(1-hydroxy-1-methylethyl)-2-propylimidazole-5-carboxylate [prepared as described in step (a) above] with a 4 N solution of hydrogen chloride in dioxane.

Nuclear Magnetic Resonance Spectrum (CDCl₃) δ ppm:
0.94 (3H, triplet, J = 7 Hz);
1.27 (6H, doublet of doublets, J = 6.5 & 11 Hz);
1.47 (3H, doublet, J = 5.5 Hz);
1.50 - 1.65 (2H, multiplet);
1.76 (6H, doublet, J = 8.5 Hz);
3.08 (2H, broad triplet, J = 8 Hz);
4.86 (1H, septet, J = 6 Hz);
5.56 (2H, singlet);
6.87 (1H, quartet, J = 5.5 Hz);
7.04 (2H, doublet, J = 7.5 Hz);
7.27 - 7.65 (5H, multiplet);
7.97 (1H, doublet, J = 8 Hz).

### EXAM PLE 29

### (5-Methyl-2-oxo-1,3-dioxolen-4-yl)methyl 1-[(2'-carboxybiphenyl-4-yl)methyl]-4-(1-hydroxy-1-methyl-ethyl)-2-propylimidazole-5-carboxylate (Compound No.. 3-25)

### 29(a) (5-Methyl-2-oxo-1,3-dioxolen-4-yl)methyl1-[(2'-t-butoxycarbonylbiphenyl-4-yl)methyl]-4-(1-hydroxy-1-methylethyl)-2-propylimidazole-5-carboxylate

Following a procedure similar to that described in Example 25(c), 0.65 g of the title compound was obtained as a gum from 0.50 g of 1-[(2'-t-butoxycarbonylbiphenyl-4-yl)methyl]-4-(1-hydroxy-1-methylethyl)-2-propyl-imidazole-5-carboxylic acid [prepared as described in Example 25(b)], 0.27 g of (5-methyl-2-oxo-1,3-dioxolen-4-yl)methyl bromide and 0.3 g of potassium carbonate in 6 ml of N,N-dimethylacetamide.

Nuclear Magnetic Resonance Spectrum (CDCl₃) δ ppm:
0.99 (3H, triplet, J = 6.5 Hz);
1.28 (9H, singlet);
1.64 (6H, singlet);
1.55 - 1.90 (2H, multiplet);
2.07 (3H, singlet);
2.70 (2H, triplet, J = 7 Hz);
4.90 (2H, singlet);
5.47 (2H, singlet);
5.51 (1H, singlet);
6.91 (2H, doublet, J = 8.5 Hz);
7.2 - 7.9 (6H, multiplet).

### 29(b) (5-Methyl-2-oxo-1,3-dioxolen-4-yl)methyl1-[(2'-carboxybiphenyl-4-yl)methyl]-4-(1-hydroxy-1-methylethyl)-2-propylimidazole-5-carboxylate

Following a procedure similar to that described in Example 25(d), 0.54 g of the hydrochloride of the title compound was obtained as an amorphous powder, melting at 90 - 93°C, by treating 0.65 g of (5-methyl-2-oxo-1,3-dioxolen-4-yl)methyl 1-[(2'-t-butoxycarbonylbiphenyl-4-yl)methyl]-4-(1-hydroxy-1-methylethyl)-2-propyl-imidazole-5-carboxylate [prepared as described in step (a) above] with a 4 N solution of hydrogen chloride in dioxane.

Nuclear Magnetic Resonance Spectrum (hexadeuterated dimethyl sulphoxide) δ ppm:
0.88 (3H, triplet, J = 7.5 Hz);
1.5 - 1.7 (2H, multiplet);
1.59 (6H, singlet);
2.11 (3H, singlet);
3.00 (2H, triplet, J = 7.5 Hz);
5.13 (2H, singlet);
5.63 (2H, singlet);
7.13 (2H, doublet, J = 8 Hz);
7.26 - 7.75 (6H, multiplet).

### EXAMPLE 30

### Pivaloyloxymethyl 1-[(2'-carboxybiphenyl-4-yl)methyl]-4-(1-hydroxy-1-methylethyl)-2-propylimidazole-5-carboxylate (Compound No. 3-1)

### 30(a) Pivaloyloxymethyl 1-[(2'-t-butoxycarbonylbiphenyl-4-yl)methyl]-4-(1-hydroxy-1-methylethyl)-2-propylimidazole-5-carboxylate

Following a procedure similar to that described in Example 25(a), 0.81 g of the title compound was obtained from 500 mg of pivaloyloxymethyl 4-(1-hydroxy-1-methylethyl) -2-propylimidazole-5-carboxylate [prepared as described in Preparation 22(ii)] and 560 mg of t-butyl 4'-bromomethylbiphenyl-2-carboxylate. The melting point and Nuclear Magnetic Resonance Spectrum of the product were identical with those of the compound obtained as described in Example 25(c).

### 30(b) Pivaloyloxymethyl 1-[(2'-carboxybiphenyl-4-yl)-methyl]-4-(1-hydroxy-1-methylethyl)-2-propylimidazole-5-carboxylate

Following a procedure similar to that described in Example 25(d), 0.45 g of the hydrochloride of the title compound was obtained as crystals from 0.5 g of pivaloyloxymethyl 1-[(2'-t-butoxycarbonylbiphenyl-4-yl)-methyl]-4-(1-hydroxy-1-methylethyl)-2-propylimidazole-5-carboxylate [prepared as described in step (a) above]. The melting point and Nuclear Magnetic Resonance Spectrum of the product were identical with those of the compound prepared as described in Example 25(d).

### EXAMPLE 31

### Pivaloyloxymethyl 2-butyl-1-[(2'-carboxybiphenyl-4-yl)methyl]-4-(1-hydroxy-1-methylethyl)imidazole-5-carboxylate (Compound No. 3-27)

### 31 (a) Methyl 1-[(2'-t-butoxycarbonylbiphenyl-4-yl)-methyl]-2-butyl-4-(1-hydroxy-1-methylethyl)-imidazole-5-carboxylate

Following a procedure similar to that described in Example 25(a), 3.54 g of the title compound were obtained as a syrup from 2.00 g of methyl 2-butyl-4-(1-hydroxy-1-methylethyl)imidazole-5-carboxylate (prepared as described in Preparation 21) and 3.03 g of t-butyl 4'-bromomethylbiphenyl-2-carboxylate.

Nuclear Magnetic Resonance Spectrum (CDCl₃) δ ppm:
0.92 (3H, triplet, J = 7.5 Hz);
1.25 (9H, singlet);
1.33 - 1.46 (2H, multiplet);
1.64 (6H, singlet);
1.68-1.78 (2H, multiplet);
2.70 (2H, triplet, J = 8 Hz);
3.78 (3H, singlet);
5.50 (2H, singlet);
5.70 (1H, singlet);
6.97 (2H, doublet, J = 8.5 Hz);
7.26 - 7.33 (3H, multiplet);
7.37 - 7.54 (2H, multiplet);
7.76 - 7.81 (1H, multiplet).

### 31 (b) 1-[(2'-t-Butoxycarbonylbiphenyl-4-yl)methyl]-2-butyl-4-(1-hydroxy-1-methylethyl) imidazole-5-carboxylic acid

Following a procedure similar to that described in Example 25(b), 2.46 g of the title compound were obtained as crystals, melting at 158 - 159°C, by hydrolyzing 3.31 g of methyl 1-[(2'-t-butoxycarbonyl-biphenyl-4-yl)methyl]-2-butyl-4-(1-hydroxy-1-methyl-ethyl)imidazole-5-carboxylate [prepared as described in step (a) above] with 1.37 g of lithium hydroxide monohydrate.

Nuclear Magnetic Resonance Spectrum (CDCl₃) δ ppm:
0.84 (3H, triplet, J = 7.5 Hz);
1.23 (9H, singlet);
1.25 - 1.38 (2H, multiplet);
1.52 - 1.65 (2H, multiplet);
1.68 (6H, singlet);
2.83 (2H, triplet, J = 6.5 Hz);
5.81 (2H, singlet);
7.07 (2H, doublet, J = 8.0 Hz);
7.22 - 7.28 (3H, multiplet);
7.34 - 7.50 (2H, multiplet);
7.74 - 7.78 (1H, multiplet).

### 31(c) Pivaloyloxymethyl 1-[(2'-t-butoxycarbonylbiphenyl-4-yl)methyl]-2-butyl-4-(1-hydroxy-1-methylethyl)-imidazole-5-carboxylate

Following a procedure similar to that described in Example 25(c), 0.48 g of the title compound was obtained as a syrup by esterifying 0.40 g of 1-[(2'-t-butoxycarbonylbiphenyl-4-yl)methyl]-2-butyl-4-(1-hydroxy-1-methylethyl)imidazole-5-carboxylic acid [prepared as described in step (b) above] with chloromethyl pivalate and potassium carbonate.

Nuclear Magnetic Resonance Spectrum (CDCl₃) δ ppm:
0.92 (3H, triplet, J = 7.5 Hz);
1.17 (9H, singlet);
1.24 (9H, singlet);
1.32 - 1.47 (2H, multiplet);
1.63 (6H, singlet);
1.66 - 1.79 (2H, multiplet);
2.69 (2H, triplet, J = 8 Hz);
5.41 (1H, singlet);
5.51 (2H, singlet);
5.83 (2H, singlet);
6.97 (2H, doublet, J = 8 Hz);
7.25 - 7.28 (3H, multiplet);
7.38 - 7.51 (2H, multiplet);
7.75 - 7.79 (1H, multiplet).

### 31(d) Pivaloyloxymethyl 2'-butyl-1-[(2'-carboxybiphenyl-4-yl)methyl]-4-(1-hydroxy-1-methylethyl)imidazole-5-carboxylate

Following a procedure similar to that described in Example 25(d), 0.45 g of the hydrochloride of the title compound was obtained as an amorphous solid, melting at. 139 - 144°C (softening at 127°C), by treating 0.48 g of pivaloyloxymethyl 1-[(2'-t-butoxycarbonylbiphenyl-4-yl)-methyl]-2-butyl-4-(1-hydroxy-I-methylethyl)imidazole-5-carboxylate [prepared as described in step (c) above] with a 4 N solution of hydrogen chloride in dioxane.

Nuclear Magnetic Resonance Spectrum (hexadeuterated dimethyl sulphoxide) δ ppm:
0.80 (3H, triplet, J = 7.5 Hz);
1.10 (9H, singlet);
1.21 - 1.35 (2H, multiplet);
1.39 - 1.50 (2H, multiplet);
1.58 (6H, singlet);
2.96 (2H, triplet, J = 7.5 Hz);
5.64 (2H, singlet);
5.88 (2H, singlet);
7.17 (2H, doublet, J = 8.5 Hz);
7.32 - 7.34 (3H, multiplet);
7.43 - 7.49 (1H, multiplet);
7.55 - 7.61 (1H, multiplet);
7.73 - 7.75 (1H, multiplet).

### EXAMPLE 32

### Isopropoxycarbonyloxymethyl 2-butyl-1-[(2'-carboxybiphenyl-4-yl)methyl]-4-(1-hydroxy-1-methylethyl)-imidazole-5-carboxylate (Compound No. 3-39)

### 32(a) Isopropoxycarbonyloxymethyl 1-[(2'-t-butoxycarbonylbiphenyl-4-yl)methyl]-2-butyl-4-(1-hydroxy-1-methylethyl) imidazole-5-carboxylate

Following a procedure similar to that described in Example 25(c), 0.46 g of the title compound was obtained as crystals, melting at 91 - 93°C, from 0.40 g of 1-[(2'-t-butoxycarbonylbiphenyl-4-yl)methyl]-2-butyl-4-(1-hydroxy-1-methylethyl)imidazole-5-carboxylic acid [prepared as described in Example 31 (b)], 0.15 g of isopropoxycarbonyloxymethyl chloride and 0.31 g of potassium carbonate.

Nuclear Magnetic Resonance Spectrum (CDCl₃) δ ppm:
0.92 (3H, triplet, J = 7.5 Hz);
1.23 (9H, singlet);
1.29 (6H, doublet, J = 6 Hz);
1.35 - 1.45 (2H, multiplet);
1.63 (6H, singlet);
1.65 - 1.80 (2H, multiplet);
2.71 (2H, triplet, J = 7.5 Hz);
4.90 (1H, septet, J = 6 Hz);
5.39 (1H, singlet);
5.51 (2H, singlet);
5.82 (2H, singlet);
6.98 (2H, doublet, J = 8 Hz);
7.25 - 7.30 (3H, multiplet);
7.35 - 7.52 (2H, multiplet);
7.75 - 7.80 (1H, multiplet).

### 32(b) Isopropoxycarbonyloxymethyl 2-butyl-1-[(2'-carboxybiphenyl-4-yl)methyl]-4-(1-hydroxy-1-methylethyl) imidazole-5-carboxylate

Following a procedure similar to that described in Example 25(d), 0.39 g of the hydrochloride of the title compound was obtained as crystals, melting at 154 - 156°C, by treating 0.40 g of isopropoxycarbonyl-oxymethyl 1-[ (2'-t-butoxycarbonylbiphenyl-4-yl)methyl]-2- butyl-4-(1-hydroxy-1-methylethyl)imidazole-5-carboxylate [prepared as described in step (a) above] with a 4 N solution of hydrogen chloride in dioxane.

Nuclear Magnetic Resonance Spectrum (hexadeuterated dimethyl sulphoxide) δ ppm:
0.81 (3H, triplet, J = 7.5 Hz);
1.21 (6H, doublet, J = 6.5 Hz);
1.23 - 1.36 (2H, multiplet);
1.38 - 1.52 (2H, multiplet);
1.59 (6H, singlet);
2.98 (2H, triplet, J = 6.5 Hz);
4.79 (1H, septet, J = 6.5 Hz);
5.65 (2H, singlet);
5.85 (2H, singlet);
7.18 (2H, doublet, J = 8 Hz);
7.30 - 7.38 (3H, multiplet);
7.42 - 7.62 (2H, multiplet);
7.74 (1H, doublet, J = 7.5 Hz).

### EXAMPLE 33

### (5-Methyl-2-oxo-1,3-dioxolen-4-yl)methyl 2-butyl-1-[(2'-carboxybiphenyl-4-yl)methyl]-4-(1-hydroxy-1-methylethyl) imidazole-5-carboxylate (Compound No. 3-51)

### 33(a) (5-Methyl-2-oxo-1,3-dioxolen-4-yl)methyl 1-[(2'-t-butoxycarbonylbiphenyl-4-yl)methyl]-2-butyl-4-(1-hydroxy-1-methylethyl)imidazole-5-carboxylate

Following a procedure similar to that described in Example 25(c), 0.43 g of the title compound was obtained as crystals, melting at 156 - 157°C, from 0.40 g of 1-[(2'-t-butoxycarbonylbiphenyl-4-yl)methyl]-2-butyl-4- (1-hydroxy-1-methylethyl)imidazole-5-carboxylic acid [prepared as described in Example 31 (b)], 0.22 g of (5-methyl-2-oxo-1,3-dioxolen-4-yl)methyl bromide and 0.26 g of potassium carbonate in 5 ml of N,N-dimethyl-acetamide.

Nuclear Magnetic Resonance Spectrum (CDCl₃) δ ppm:
0.92 (3H, triplet, J = 7.5 Hz);
1.27 (9H, singlet);
1.30 - 1.45 (2H, multiplet);
1.62 (6H, singlet);
1.65 - 1.80 (2H, multiplet);
2.07 (3H, singlet);
2.70 (2H, triplet, J = 7.5 Hz);
4.89 (2H, singlet);
5.46 (2H, singlet);
5.55 (1H, singlet);
6.91 (2H, doublet, J = 8.5 Hz);
7.26 - 7.50 (5H, multiplet);
7.76 (1H, doublet, J = 6.5 Hz).

### 33(b) (5-Methyl-2-oxo-1,3-dioxolen-4-yl)methyl 2-butyl-1-[(2'-carboxybiphenyl-4-yl)methyl]-4-(1-hydroxy-1-methylethyl)imidazole-5-carboxylate

Following a procedure similar to that described in Example 25(d), 0.26 g of the hydrochloride of the title compound was obtained as a powder, melting at above 70°C (softening), by treating 0.32 g of (5-methyl-2-oxo-1,3-dioxolen-4-yl)methyl 1-[(2'-t-butoxycarbonylbiphenyl-4-yl)methyl]-4-(1-hydroxy-1-methylethyl)imidazole-5- carboxylate [prepared as described in step (a) above] with a 4 N solution of hydrogen chloride in dioxane.

Nuclear Magnetic Resonance Spectrum (hexadeuterated dimethyl sulphoxide) δ ppm:
0.82 (3H, triplet, J = 7 Hz);
1.20 - 1.40 (2H, multiplet);
1.40 - 1.60 (2H, multiplet);
1.59 (6H, singlet);
2.12 (3H, singlet);
2.98 (2H, triplet, J = 7.5 Hz);
5.14 (2H, singlet);
5.63 (2H, singlet);
7.13 (2H, doublet, J = 7.5 Hz);
7.30 - 7.60 (5H, multiplet);
7.74 (1H, doublet, J = 7.5 Hz).

### EXAMPLE 34

### Phthalidyl 1-[(2'-carboxybiphenyl-4-yl)methyl]-4-(1-hydroxy-1-methylethyl)-2-propylimidazole-5-carboxylate (Compound No 3-26)

### 34(a) Phthalidyl 1-[(2'-t-butoxycarbonylbiphenyl-4-yl)methyl]-4-(1-hydroxy-1-methylethyl)-2-propylimidazole-5-carboxylate

Following a procedure similar to that described in Example 25(c), 0.62 g of the title compound was obtained as crystals, melting at 144°C, from 0.50 g of 1-[(2'-t-butoxycarbonylbiphenyl)methyl]-4-(1-hydroxy-1-methylethyl)-2-propylimidazole-5-carboxylic acid [prepared as described in Example 25(b)], 0.25 g of 3-bromophthalide and 0.3 g of potassium carbonate in 6 ml of N,N-dimethylacetamide.

Nuclear Magnetic Resonance Spectrum (CDCl₃) δ ppm:
0.97 (3H, triplet, J = 7.5 Hz);
1.25 (9H, singlet);
1.62 (6H, singlet);
1.75 (2H, sextet, J = 7.5 Hz);
2.66 (2H, triplet, J = 6.5 Hz);
5.38 (2H, AB-quartet, Δδ = 0.10 ppm, J = 17 Hz);
5.42 (1H, singlet);
6.69 (2H, doublet, J = 7.5 Hz);
7.15 (2H, doublet, J = 7.5 Hz);
7.28 - 7.89 (9H, multiplet).

### 34(b) Phthalidyl 1-[(2'-carboxybiphenyl-4-yl)methyl]-4-(1-hydroxy-1-methylethyl)-2-propylimidazole-5-carboxylate

Following a procedure similar to that described in Example 25(d), 0.37 g of the hydrochloride of the title compound was obtained as an amorphous powder, melting at 142 - 144°C, by treating 0.45 g of phthalidyl 1-[(2'-t-butoxycarbonylbiphenyl-4-yl)methyl]-4-(1-hydroxy-1-methylethyl)-2-propylimidazole-5-carboxylate [prepared as described in step (a) above] with a 4 N solution of hydrogen chloride in dioxane.

Nuclear Magnetic Resonance Spectrum (hexadeuterated dimethyl sulphoxide) δ ppm:
0.92 (3H, triplet, J = 7.5 Hz);
1.50 - 1.70 (2H, multiplet);
1.59 (6H, singlet);
3.00 (2H, triplet, J = 7.5 Hz);
5.65 (2H, singlet);
7.01 (2H, doublet, J = 8 Hz);
7.27 (2H, doublet, J = 8 Hz);
7.36 - 7.98 (9H, multiplet).

### EXAMPLE 50

### 2-Butyl-1-[(2'-carboxybiphenyl-4-yl)methyl]-4-(1-hydroxy-1-methylethyl)imidazole-5-carboxamide (Compound No. 5-69)

### 50(a) 1-[(2'-t-Butoxycarbonylbiphenyl-4-yl)methyl]-2-butyl-4-(1-hydroxy-1-methylethyl)imidazole-5-carboxamide

10 ml of a 1 N aqueous solution of sodium hydroxide were added to a solution of 232 mg of 1-[(2'-t-butoxy-carbonylbiphenyl-4-yl)methyl]-2-butyl-4-(1-hydroxy-lmethylethyl)imidazole-5-carbonitrile [prepared as described in Example 10(a)] in 10 ml of ethanol, and the resulting mixture was heated under reflux for 3 hours. At the end of this time, the reaction mixture was worked up in a similar manner to that described in Example 45(c), to afford 185 mg of the title compound as an amorphous solid.

Nuclear Magnetic Resonance Spectrum (CDCl₃) δ ppm::
0.89 (3H, triplet, J = 7 Hz);
1.0 - 2.0 (4H, multiplet);
1.23 (9H, singlet);
1.68 (6H, singlet);
2.62 (2H, triplet, J = 7 Hz);
5.63 (2H, singlet);
6.9 - 7.9 (8H, multiplet).

### 50(b) 2-Butyl-1-[(2'-carboxybiphenyl-4-yl)methyl]-4-(1-hydroxy-1-methylethyl)imidazole-5-carboxamide

Following a procedure similar to that described in Preparation 45(d), but using 185 mg of 1-[(2'-t-butoxycarbonylbiphenyl-4-yl)methyl]-2-butyl-4-(1-hydroxy-1-methylethyl)imidazole-5-carboxamide [prepared as described in step (a) above] and 10 ml of a 4 N solution of hydrogen chloride in dioxane, 88 mg of the hydrochloride of the title compound were obtained as an amorphous solid, melting at 130 - 138°C (with softening).

Nuclear Magnetic Resonance Spectrum (hexadeuterated dimethyl sulphoxide) δ ppm:
0.78 (3H, triplet, J = 7 Hz);
1.17 - 1.30 (2H, multiplet);
1.30 - 1.42 (2H, multiplet);
1.61 (6H, singlet);
2.96 (2H, triplet, J = 7.5 Hz);
5.55 (2H, singlet);
7.20 - 7.75 (8H, multiplet).

### EXAMPLE 51

### 2-Butyl-1-[(2'-carboxybiphenyl-4-yl)methyl]-4-[1-hydroxy-2-methyl-1-(1-methylethyl)propyl]imidazole-5-carboxamide (Compound No. 5-333)

### 51 (a) 1-[(2'-t-Butoxycarbonylbiphenyl-4-yl)methyl]-2-butyl-4-(1-hydroxy-2-methyl-1-(1-methylethyl)-propyl]imidazole-5-carbonitrile

Following a procedure similar to that described in Preparation 45(a), but using 282 mg of 2-butyl-4-[1-hydroxy-2-methyl-1-(1-methylethyl)propyl]imidazole-5-carbonitrile (prepared as described in Preparation 30), 409 mg of t-butyl 4'-(bromomethyl)biphenyl-2-carboxylate and 47 mg of sodium hydride (as a 55% w/w dispersion in mineral oil) in 5 ml of N,N-dimethylacetamide, 513 mg of the title compound were obtained as a viscous oil.

Nuclear Magnetic Resonance Spectrum (CDCl₃) δ ppm:
0.7 - 1.1 (15H, multiplet);
1.0 - 2.0 (4H, multiplet);
1.21 (9H, singlet);
2.15 - 2.60 (2H, multiplet);
2.68 (2H, triplet, J = 7 Hz);
3.20 (1H, singlet);
5.26 (2H, singlet);
6.9 - 8.0 (8H, multiplet).

### 51(b) 1-[(2'-t-Butoxycarbonylbiphenyl-4-yl)methyl]-2-butyl-4-[1-hydroxy-2-methyl-1-(1-methylethyl)-propyl]imidazole-5-carboxamide

10 ml of a 1 N aqueous solution of sodium hydroxide were added to a solution of 500 mg of 1-[(2'-t-butoxycarbonylbiphenyl-4-yl)methyl]-2-butyl-4-[1-hydroxy-2-methyl-1-(1-methylethyl)propyl]imidazole-5-carbonitrile [prepared as described in step (a) above] in 10 ml of ethanol, and the resulting mixture was heated under reflux for 20 hours. At the end of this time, the reaction mixture was worked up in a similar manner to that described in Preparation 45(c), to give 220 mg of the title compound as an amorphous solid.

Nuclear Magnetic Resonance Spectrum (CDCl₃) δ ppm:
0.7 - 1.1 (15H, multiplet);
1.0 - 2.1 (4H, multiplet);
1.20 (9H, singlet);
2.2 - 2.9 (4H, multiplet);
5.59 (2H, singlet);
6.8 - 7.9 (8H, multiplet).

### 51(c) 2-Butyl-1-[(2'-carboxybiphenyl-4-yl)methyl]-4-[1-hydroxy-2-methyl-1-(1-methylethyl)propyl]imidazole-5-carboxamide

Following a procedure similar to that described in Preparation 45(d), but using 220 mg of 1-[(2'-t-butoxycarbonylbiphenyl-4-yl)methyl]-2-butyl-4-[1-hydroxy-2-methyl-1-(1-methylethyl)propyl]imidazole-5-carboxamide [prepared as described in step (b) above] and 4.5 ml of a 4 N solution of hydrogen chloride in dioxane, 201 mg of the hydrochloride of the title compound were obtained as an amorphous solid, melting at 178 - 181°C.

Nuclear Magnetic Resonance Spectrum (hexadeuterated dimethyl sulphoxide) δ ppm:
0.76 (3H, triplet, J = 7.5 Hz);
0.8 - 0.9 (12H, multiplet);
1.1 - 1.4 (4H, multiplet);
2.2 - 2.4 (2H, multiplet);
2.8 - 3.1 (2H, multiplet);
5.51 (2H, singlet);
7.2 - 7.8 (8H, multiplet).

### EXAMPLE 61

### (5-Methyl-2-oxo-1,3-dioxolen-4-yl)methyl 4-(1-hydroxy-1-methylethyl)-2-propyl-1-{4-[2-(tetrazol-5-yl)phenyl]phenyl} methylimidazole-5-carboxylate (Compound No. 2-17)

### 61 (a) (5-Methyl-2-oxo-1,3-dioxolen-4-yl)methyl 4-(1-hydroxy-1-methylethyl)-2-propyl-1-{4-[2-(trityl-tetrazol-5-yl) phenyl]phenyl}methylimidazole-5-carboxylate

A suspension of 0.97 g of potassium carbonate in 100 ml of N,N-dimethylacetamide was warmed at 60°C, and then a solution of 1.14 g of (5-methyl-2-oxo-1,3- dioxolen-4-yl)methyl 4-(1-hydroxy-1-methylethyl)-2-propylimidazole-5-carboxylate (prepared as described in Preparation 31) and 2.35 g of 4-[2-(trityltetrazol-5-yl)phenyl]benzyl bromide in 50 ml of N,N-dimethyl-acetamide was added dropwise to the' warm suspension, whilst stirring. The reaction mixture was stirred at 60°C for 3.5 hours, and it was then diluted with ethyl acetate. The ethyl acetate layer was separated, washed with water and dried over anhydrous magnesium sulphate, and then the solvent was removed by distillation under reduced pressure. The resulting residue was purified by column chromatography through silica gel, using a 1 : 1 by volume mixture of hexane and ethyl acetate as the eluent, to give 1.4 g of the title compound as an amorphous solid. This product was crystallized from diisopropyl ether, to give pure title compound, melting at 98 - 99°C (with decomposition).

Nuclear Magnetic Resonance Spectrum (CDCl₃), δ ppm:
0.89 (3H, triplet, J = 7.5 Hz);
1.62 (6H, singlet);
1.6 - 1.75 (2H, multiplet);
1.97 (3H, singlet);
2.54 (2H, triplet, J = 8 Hz);
4.70 (2H, singlet);
5.30 (2H, singlet);
5.61 (1H, singlet);
6.68 (2H, doublet, J = 7.5 Hz);
6:90 - 7.52 (20H, multiplet);
7.87 (1H, doublet, J = 7.5 Hz).

### 61 (b) (5-Methyl-2-oxo-1,3-dioxolen-4-yl)methyl 4-(1-hydroxy-1-methylethyl)-2-propyl-1-{4-[2-(tetrazol-5-yl)phenyl] phenyl}methylimidazole-5-carboxylate

A mixture of 1.4 g of (5-methyl-2-oxo-1,3-dioxolen-4-yl)methyl 4-(1-hydroxy-1-methylethyl)-2-propyl-1-{4-[2-(trityltetrazol-5-yl)phenyl]phenyl}methylimidazole-5- carboxylate [prepared as described in step (a) above] and 48 ml of 75% v/v aqueous acetic acid was stirred at 60°C for 1 hour, after which it was concentrated by evaporation under reduced pressure. The residue was dissolved in toluene, and the resulting solution was concentrated by distillation under reduced pressure; this was repeated a further time in order to remove the remaining water and acetic acid. The residue thus obtained was purified by column chromatography through silica gel using 1 : 9 and 1 : 4 by volume mixtures of methanol and methylene chloride as the eluent, to give 0.73 g of the title compound, melting at 170 - 172°C.

Nuclear Magnetic Resonance Spectrum (CDCl₃), δ ppm:
0.93 (3H, triplet, J = 7.5 Hz);
1.63 (6H, singlet);
1.6 - 1.8 (2H, multiplet);
2.19 (3H, singlet);
2.70 (2H, triplet, J = 7.5 Hz);
5.00 (2H, singlet);
5.45 (2H, singlet);
6.83 (2H, doublet, J = 8 Hz);
7.10 (2H, doublet, J = 8 Hz);
7.42 - 7.63 (3H, multiplet);
7.83 (1H, doublet of doublets, J = 1 & 7.5 Hz).

### EXAMPLE 62

### Pivaloyloxymethyl 4-(1-hydroxy-1-methylethyl)-2-propyl-1-{4-[2-(tetrazol-5-yl)phenyl]phenyl}methylimidazole-5-carboxylate (Compound No. 2-15)

### 62(a) Pivaloyloxymethyl 4-(1-hydroxy-1-methylethyl)-2-propyl-1-{4-[2-(trityltetrazol-5-yl)phenyl]-phenyl} methylimidazole-5-carboxylate

Following a procedure similar to that described in Example 61 (a), but using 0.85 g of pivaloyloxymethyl 4-(1-hydroxy-1-methylethyl)-2-propylimidazole-5-carboxylate [prepared as described in Preparation 22(ii)], 1.52 g of 4-[2-(trityltetrazol-5-yl)phenyl]- benzyl bromide and 0.72 g of potassium carbonate, 1.02 g of the title compound were obtained as an amorphous solid.

The Nuclear Magnetic Resonance spectrum of this compound was identical with that of the compound obtained as described in Example 20(a).

### 62 (b) Pivaloyloxymethyl4-(1-hydroxy-1-methylethyl)-2-propyl-1-{4-[2-(tetrazol-5-yl)phenyl]phenyl}-methylimidazole-5-carboxylate

The pivaloyloxymethyl 4-(1-hydroxy-1-methylethyl)-2-propyl-1-{4-[2-trityltetrazol-5-yl)phenyl]phenyl}-methylimidazole-5-carboxylate prepared as described in step (a) above was detritylated following a procedure similar to that described in Example 20(b), to give the hydrochloride of the title compound in an 80% yield.

The melting point and Nuclear Magnetic Resonance spectrum of this compound were identical with those of the compound obtained as described in Example 20(b).

### EXAMPLE 63

### Phthalidyl 4-(1-hydroxy-1-methylethyl)-2-propyl-1-{4-[2-(tetrazol-5-yl)phenyl]phenyl}methylimidazole-5-carboxylate

### (Compound No. 2-65)

### 63(a) Phthalidyl 4-(1-hydroxy-1-methylethyl)-2-propyl-1-{4-[2-(trityltetrazol-5-yl)phenyl]phenyl}-methylimidazole-5-carboxylate

Following a procedure similar to that described in Example 61 (a), but using 0.456 g of phthalidyl 4-(1-hydroxy-1-methylethyl)-2-propylimidazole-5-carboxylate (prepared as described in Preparation 32), 0.736 g of 4-[2-(trityltetrazol-5-yl)phenyl]benzyl bromide and 0.366 g of potassium carbonate, 0.196 g of the title compound was obtained, melting at 118 - 120°C.

Nuclear Magnetic Resonance Spectrum (CDCl₃), δ ppm:
0.95 (3H, triplet, J = 7.5 Hz) ;
1.66 (6H, singlet);
1.65 - 1.80 (2H, multiplet);
2.60 (2H, triplet, J = 7.5 Hz);
5.09 (2H, singlet);
6.92 - 7.56 (27H, multiplet);
7.93 (1H, doublet of doublets, J = 1 & 8 Hz).

### 63(b) Phthalidyl 4-(1-hydroxy-1-methylethyl)-2-propyl-1-{4-[2-(tetrazol-5-yl)phenyl]phenyl}methyl-imidazole-5-carboxylate

Following a procedure similar to that described in Example 61 (b), 0.196 g of phthalidyl 4-(1-hydroxy-1-methylethyl)-2-propyl-1-{4-[2-(trityltetrazol-5-yl)-phenyl]phenyl}methylimidazole-5-carboxylate [prepared as described in step (a) above] was detritylated by heating it with 75% v/v aqueous acetic acid to give 0.110 g of the title compound, melting at 168 - 170°C.

Nuclear Magnetic Resonance Spectrum (CDCl₃), δ ppm:
0.92 (3H, triplet, J = 7.5 Hz);
1.57 (6H, singlet);
1.60 - 1.77 (2H, multiplet);
2.65 (2H, triplet, J = 7.5 Hz);
5.13 (2H, singlet);
6.91 - 7.57 (12H, multiplet);
7.80 (1H, doublet, J = 7.5 Hz).

### EXAMPLE 64

### Isopropoxycarbonyloxymethyl 4-(1-hydroxy-1-methylethyl)-2-propyl-1-{4-[2-(tetrazol-5-yl)phenyl]-phenyl} methylimidazole-5-carboxylate (Compound No. 2-21)

### 64(a) Isopropoxycarbonyloxymethyl 4-(1-hydroxy-1-methylethyl)-2-propyl-1-{4-[2-(trityltetrazol-5-yl)-phenyl]phenyl} methylimidazole-5-carboxylate

Following a procedure similar to that described in Example 61 (a), but using 656 mg of isopropoxycarbonyloxymethyl 4-(1-hydroxy-1-methylethyl)-2-propylimidazole-5-carboxylate (prepared as described in Preparation 33), 1.20 g of 4-[2-(trityltetrazol-5-yl)phenyl]benzyl bromide and 0.51 g of potassium carbonate, 0.78 g of the title compound was obtained as a viscous liquid.

Nuclear Magnetic Resonance Spectrum (CDCl₃), δ ppm:
0.87 (3H, triplet, J = 7.5 Hz);
1.24 (6H, doublet, J = 6 Hz);
1.63 (6H, singlet);
1.65 - 1.80 (2H, multiplet);
2.52 (2H, triplet, J = 7.5 Hz);
4.87 (1H, quintet, J = 6 Hz);
5.35 (2H, singlet);
5.42 (1H, singlet);
5.66 (2H, singlet);
6.74 - 7.87 (22H, multiplet);
7.87 - 7.96 (1H, multiplet).

### 64(b) Isopropoxycarbonyloxymethyl 4-(1-hydroxy-1-methylethyl)-2-propyl-1-{4-[2-(tetrazol-5-yl)phenyl]-phenyl} methylimidazole-5-carboxylate

Following a procedure similar to that described in Example 61 (b), 0.78 g of isopropoxycarbonyloxymethyl 4-(1-hydroxy-1-methylethyl)-2-propyl-1-{4-[2-(trityl-tetrazol-5-yl)phenyl]phenyl}methylimidazole-5- carboxylate [prepared as described in step (a) above] was detritylated by heating it with 75% v/v aqueous acetic acid, to give 0.48 g of the title compound as an amorphous solid.

Nuclear Magnetic Resonance Spectrum (CDCl₃), δ ppm:
0.96 (3H, triplet, J = 7.5 Hz);
1.21 (6H, doublet, J = 6 Hz);
1.63 (6H, singlet);
1.72 (2H, sextet, J = 7.5 Hz);
2.60 (2H, triplet, J = 7.5 Hz);
4.72 (1H, quintet, J = 6.5 Hz);
5.33 (2H, singlet);
5.76 (2H, singlet);
6.77 (2H, doublet, J = 7.5 Hz);
6.92 (2H, doublet, J = 7.5 Hz);
7.37 - 7.60 (3H, multiplet);
7.87 (1H, doublet, J = 7.5 Hz).

### EXAMPLE 65

### Ethyl 1-[(2'-t-butoxycarbonylbiphenyl-4-yl)methyl]-2-ethyl-4-(1-hydroxy-1-methylethyl)imidazole-5-carboxylate (Compound No. 1-130)

0.337 g of potassium t-butoxide was added to a solution of 0.68 g of ethyl 2-ethyl-4-(1-hydroxy-1-methylethyl) imidazole-5-carboxylate (prepared as described in Preparation 37) in 7 ml of N,N-dimethyl-acetamide, and the resulting mixture was stirred at room temperature for 10 minutes. 1.04 g of t-butyl 4'-bromomethylbiphenyl-2-carboxylate was then added to the resulting solution, and the reaction mixture was stirred at room temperature for 4 hours. At the end of this time, it was mixed with ethyl acetate and water. The ethyl acetate layer was separated, dried over anhydrous magnesium sulphate and concentrated by evaporation under reduced pressure. The residue was purified by column chromatography through silica gel, using a 1 : 1 by volume mixture of ethyl acetate and hexane as the eluent, to give 1.32 g of the title compound as a gum.

Nuclear Magnetic Resonance Spectrum (CDCl₃) δ ppm:
1.23 (9H, singlet);
1.23 (3H, triplet, J = 7.5 Hz);
1.29 (3H, triplet, J = 7.5 Hz);
1.63 (6H, singlet);
2.73 (2H, quartet, J = 7.5 Hz);
4.26 (2H, quartet, J = 7.5 Hz);
5.54 (2H, singlet);
5.73 (1H, singlet);
6.98 (2H, doublet, J = 8.5 Hz);
7.5 - 7.9 (6H, multiplet).

### EXAMPLE 66

### Ethyl 1-[(2'-carboxybiphenyl-4-yl)methyl]-2-ethyl-4-(1-hydroxy-1-methylethyl)imidazole-5-carboxylate (Compound No. 1-131)

Following a procedure similar to that described in Preparation 44, but using 1.32 g of ethyl 1-[(2'-t-butoxycarbonylbiphenyl-4-yl)methyl]-2-ethyl-4-(1-hydroxy-1-methylethyl)imidazole-5-carboxylate (prepared as described in Example 65) and a 4 N solution of hydrogen chloride in dioxane, 0.94 g of the hydrochloride of the title compound was obtained as an amorphous powder.

Nuclear Magnetic Resonance Spectrum (hexadeuterated dimethyl sulphoxide) δ ppm:
1.09 (3H, triplet, J = 7.5 Hz);
1.15 (3H, triplet, J = 7.5 Hz);
1.61 (6H, singlet);
3.03 (2H, quartet, J = 7.5 Hz);
4.22 (2H, quartet, J = 7.5 Hz);
5.64 (2H, singlet);
7.16 (2H, doublet, J = 8:5 Hz);
7.32 - 7.75 (6H, multiplet).

### EXAMPLE 67

### 1-[(2'-Carboxybiphenyl-4-yl)methyl]-2-ethyl-4-(1-hydroxy-1-methylethyl)imidazole-5-carboxylic acid (Compound No. 1-132)

Following a procedure similar to that described in Example 17, but using 0.40 g of the hydrochloride of ethyl 1-[2'-carboxybiphenyl-4-yl)methyl]-2-ethyl-4-(1- hydroxy-1-methylethyl)imidazole-5-carboxylate (prepared as described in Example 66) and 0.18 g of lithium hydroxide monohydrate, 0.25 g of the title compound was obtained as an amorphous powder.

Nuclear Magnetic Resonance Spectrum (hexadeuterated dimethyl sulphoxide) δ ppm:
1.17 (3H, triplet, J = 7.5 Hz);
1.64 (6H, singlet);
2.85 (2H, quartet, J = 7.5 Hz);
5.74 (2H, singlet);
7.10 (2H, doublet, J = 8 Hz);
7.30 - 7.76 (6H, multiplet).

### EXAMPLE 68

### Ethyl 2-ethyl-4-(1-hydroxy-1-methylethyl)-1-{4-[2-(tetrazol-5-yl)phenyl]phenyl}methylimidazole-5-carboxylate (Compound No. 2-72)

### 68(a) Ethyl 2-ethyl-4-(1-hydroxy-1-methylethyl)-1-{4-[2-(trityltetrazol-5-yl)phenyl]phenyl}methyl-imidazole-5-carboxylate

0.52 g of potassium t-butoxide was added to a solution of 1.00 g of ethyl 2-ethyl-4-(1-hydroxy-1-methylethyl) imidazole-5-carboxylate (prepared as described in Preparation 37) in 26 ml of N,N-dimethyl-acetamide, and the resulting mixture was stirred at room temperature for 1 minutes. A solution of 2.71 g of 4-[2-(trityltetrazol-5-yl)phenyl] benzyl bromide in 35 ml of N,N-dimethylacetamide was then added dropwise t the resulting solution, after which the reaction mixture wa stirred at 50°C for 4 hours. At the end of this time, the reaction mixture was worked up in a similar manner to that described in Example 18(a), to give 2.01 g of the title compound as crystals, melting at 150 - 152°C.

Magnetic Resonance Spectrum (CDCl₃) δ ppm:
1.10 (3H, triplet, J = 7.5 Hz);
1.18 (3H, triplet, J = 7.5 Hz);
1.65 (6H, singlet);
2.52 (2H, quartet, J = 7.5 Hz);
4.14 (2H, quartet, J = 7.5 Hz);
5.35 (2H, singlet);
5.80 (1H, singlet);
6.73 (2H, doublet, J = 8.5 Hz);
6.93 - 7.52 (20H, multiplet);
7.87 (1H, doublet, J = 7.5 Hz).

### 68(b) Ethyl 2-ethyl-4-(1-hydroxy-1-methylethyl)-1-{4-[2-(tetrazol-5-yl)phenyl]phenyl}methylimidazole-5-carboxylate

A solution of 1.9 g of ethyl 2-ethyl-4-(1-hydroxy-1-methylethyl)-1-{4-[2-(trityltetrazol-5-yl)phenyl]-phenyl}methylimidazole-5-carboxylate [prepared as described in step (a) above] in 28 ml of 75% v/v aqueous acetic acid was stirred at 60°C for 2 hours. At the end of this time, the reaction mixture was diluted with 7 ml of water and cooled to room temperature. Precipitated trityl alcohol was removed by filtration, and the filtrate was concentrated by evaporation under reduced pressure. The syrupy residue was crystallized in diisopropyl ether, to give 1.21 g of the title compound, melting at 166 - 167°C.

Nuclear Magnetic Resonance Spectrum (CDCl₃) δ ppm:
1.14 (3H, triplet, J = 7.5 Hz);
1.20 (3H, triplet, J = 7.5 Hz);
1.48 (6H, singlet);
2.52 (2H, quartet, J = 7.5 Hz);
4.19 (2H, quartet, J = 7.5 Hz);
5.41 (2H, singlet);
6.79 (2H, doublet, J = 8.5 Hz);
7.09 (2H, doublet, J = 8.5 Hz);
7.41 - 7.62 (3H, multiplet);
7.85 (1H, doublet, J = 7.5 Hz).

### EXAMPLE 69

### 2-Ethyl-4-(1-hydroxy-1-methylethyl)-1-{4-[2-tetrazol-5-yl)phenyl]phenyl}methylimidazole-5-carboxylic acid (Compound No. 2-68)

A solution of 0.54 g of lithium hydroxide monohydrate in 10 ml of water was added to a solution of ethyl 2-ethyl-4-(1-hydroxy-1-methylethyl)-1-{4-[2-(tetrazol-5-yl)phenyl]phenyl}methylimidazole-5- carboxylate [prepared as described in Example 68(b)] in 10 ml of dioxane, and the resulting mixture was stirred at room temperature for 4 hours. At the end of this time, the dioxane was removed by evaporation under reduced pressure, and 12.6 ml of IN aqueous hydrochloric acid were added to the resulting aqueous residue. Collection of precipitated crystals by filtration gave 0.93 g of the title compound, melting at 179 - 181°C.

Nuclear Magnetic Resonance Spectrum (hexadeuterated dimethyl sulphoxide) δ ppm:
1.09 (3H, triplet, J = 7.5 Hz);
1.55 (6H, singlet);
2.63 (2H, quartet, J = 7.5 Hz);
5.65 (2H, singlet);
6.96 (2H, doublet, J = 8.5 Hz);
7.03 (2H, doublet, .J = 8.5 Hz);
7.08 - 7.64 (4H, multiplet).

### EXAMPLE 70

### Ethyl 4-(1-hydroxy-1-methylethyl)-2-propyl-1-{4-[2-(tetrazol-5-yl)phenyl]phenyl}methylimidazole-5-carboxylate (Compound No. 2-7)

### 70(a) Ethyl 1-(2'-cyanobiphenyl-4-yl)methyl-4-(1-hydroxy-1-methylethyl)-2-propylimidazole-5- carboxylate

Following a procedure similar to that as described in Example 68(a), but using 4.01 g of ethyl 4-(1-hydroxy-1-methylethyl)-2-propylimidazole-5- carboxylate (prepared as described in Preparation 9), 5.0 g of 4'-bromomethylbiphenyl-2-carbonitrile and 1.97 g of potassium t-butoxide, 6.86 g of the title compound were obtained as crystals, melting at 92 - 93°C.

Nuclear Magnetic Resonance Spectrum (CDCl₃) δ ppm:
0.97 (3H, triplet, J = 7.5 Hz);
1.16 (3H, triplet, J = 7 Hz);
1.65 (6H, singlet);
1.74 (2H, sextet, J = 7.5 Hz);
2.67 (2H, triplet, J = 7.5 Hz);
4.24 (2H, quartet, J = 7 Hz);
5.52 (2H, singlet);
5.77 (1H, singlet);
7.05 (2H, doublet, J = 8.5 Hz);
7.42 - 7.67 (5H, multiplet);
7.76 (1H, doublet, J = 8 Hz).

### 70(b) Ethyl 4-(1-hydroxy-1-methylethyl)-2-propyl-1-{4-[2-(tetrazol-5-yl)phenyl]phenyl}methyl-imidazole-5-carboxylate

A solution of 2.00 g of ethyl 1-(2'-cyanobiphenyl-4-yl)methyl-4-(1-hydroxy-1-methylethyl)-2-propyl-imidazole-5-carboxylate [prepared as described in step (a) above] and 2.00 g of tributyltin azide in 15 ml of toluene was stirred at 100°C for 5 days. At the end of this time, the reaction mixture was concentrated by evaporation under reduced pressure, and the residue was dissolved in 30 ml of a 4 N solution of hydrogen chloride in dioxane. The solution was allowed to stand at room temperature for 16 hours, after which it was concentrated by evaporation under reduced pressure. The residue was triturated in diisopropyl ether, to give 2.00 g of the hydrochloride of the title compound.

The Nuclear Magnetic Resonance Spectrum of this compound was identical with that of the compound obtained as described in Example 18(b).

### EXAMPLE 71

### Ethyl 4-(1-hydroxy-1-methylethyl)-2-propyl-1-{4-[2-(tetrazol-5-yl)phenyl]phenyl]methylimidazole-5-carboxylate (Compound No. 2-7)

### 71(a) Ethyl 1-{4-[2-(t-butylaminocarbonyl)phenyl]-phenyl}methyl-4-(1-hydroxy-1-methylethyl)-2-propylimidazole-5-carboxylate

Following a procedure similar to that described in Example 68(a), but using 4.16 g of ethyl 4-(1-hydroxy-1-methylethyl)-2-propylimidazole-5-carboxylate (prepared as described in Preparation 9), 6.00 g of N-t-butyl-4'- bromomethylbiphenyl-2-carboxamide (prepared as described in Preparation 38) and 2.14 g of potassium t-butoxide, 5.87 g of the title compound was obtained as crystals, melting at 145 - 146°C.

Nuclear Magnetic Resonance Spectrum (CDCl₃) δ ppm:
0.97 (3H, triplet, J = 7.5 Hz);
1.12 (9H, singlet);
1.24 (3H, triplet, J = 7 Hz);
1.64 (6H, singlet);
1.75 (2H, sextet, J = 7.5 Hz);
2.66 (2H, triplet, J = 7.5 Hz);
4.25 (2H, quartet, J = 7 Hz);
5.03 (1H, singlet);
5.52 (2H, singlet);
5.69 (1H, singlet);
6.98 (2H, doublet, J = 8.5 Hz);
7.28 - 7.47 (5H, multiplet);
7.65 (1H, doublet, J = 7 Hz).

### 71 (b) Ethyl 1-(2'-cyanobiphenyl-4-yl)methyl-4-(1-hydroxy-1-methylethyl)-2-propylimidazole-5-carboxylate

0.345 ml of oxalyl chloride was added dropwise, whilst ice-cooling, to a solution of 1.00 g of ethyl 1-{4-[2-(t-butylaminocarbonyl)phenyl]phenyl}methyl-4-(1-hydroxy-1-methylethyl)-2-propylimidazole-5-carboxylate [prepared as described in step (a) above] in 10 ml of methylene chloride, and the mixture was stirred at the same temperature for 2 hours. At the end of this time, the reaction mixture was diluted with an aqueous solution of sodium hydrogencarbonate and ethyl acetate, and the ethyl acetate layer was separated, dried over anhydrous magnesium sulphate and concentrated by evaporation under reduced pressure. The residue was purified by column chromatography through silica gel, using a 1 : 1 by volume mixture of ethyl acetate and hexane as the eluent, to give 0.69 g of the title compound as crystals.

The melting point and Nuclear Magnetic Resonance Spectrum of this compound were identical with those of the compound obtained as described in Example 70 (a).

### 71(c) Ethyl 4-(1-hydroxy-1-methylethyl)-2-propyl-1-{4-[2-(tetrazol-5-yl)phenyl]phenyl}methyl-imidazole-5-carboxylate

Following a procedure similar to that described in Example 70(b), but using ethyl 1-(2'-cyanobiphenyl-4-yl) methyl-4-(1-hydroxy-1-methylethyl)-2-propylimidazole-5-carboxylate [prepared as described in step (b) above], the title compound was obtained in a 91% yield.

The Nuclear Magnetic Resonance Spectrum of this compound was identical with that of the compound obtained as described in Example 18(b).

### EXAMPLE 72

### Ethyl 4-(1-hydroxy-1-methylethyl)-2-propyl-1-{4-[2-(tetrazol-5-yl)phenyl]phenyl}methylimidazole-5-carboxylate (Compound No. 2-7)

### 72(a) Ethyl 1-[(2'-t-butoxycarbonylbiphenyl-4-yl)- methyl]-4-(1-hydroxy-1-methylethyl)-2-propyl-imidazole-5-carboxylate

Following a procedure similar to that described in Example 68(a), but using 4.80 g of ethyl 4-(1-hydroxy-1-methylethyl)-2-propylimidazole-5-carboxylate (prepared as described in Preparation 9), 6.94 g of t-butyl 4'-bromomethylbiphenyl-2-carboxylate and 2.28 g of potassium t-butoxide, 7.50 g of the title compound were obtained as crystals, melting at 90 - 91 °C.

Nuclear Magnetic Resonance Spectrum (CDCl₃) δ ppm:
0.97 (3H, triplet, J = 7 Hz);
1.23 (3H, triplet, J = 7 Hz);
1.25 (9H, singlet);
1.60 (6H, singlet);
1.82 (2H, sextet; J = 7 Hz);
2.67 (2H, triplet, J = 7 Hz);
4.24 (2H, quartet, J = 7 Hz);
5.51 (2H, singlet).;
5.72 (1H, singlet);
6.87 - 7.85 (8H, multiplet).

### 72(b) Ethyl 1-[(2'-carboxybiphenyl-4-yl)methyl]-4-(1-hydroxy-1-methylethyl)-2-propylimidazole-5-carboxylate

Following a procedure similar to that described in Example 18(b), but using 0.80 g of ethyl 1-[(2'-t-butoxycarbonylbiphenyl-4-yl)methyl]-4-(1-hydroxy-1-methylethyl)-2-propylimidazole-5-carboxylate [prepared as described in step (a) above] and a 4 N solution of hydrogen chloride in dioxane, 0.67 g of the hydrochloride of title compound was obtained as an amorphous powder.

Nuclear Magnetic Resonance Spectrum (hexadeuterated dimethyl sulphoxide) δ ppm:
0.88 (3H, triplet, J = 7 Hz);
1.14 (3H, triplet, J = 7 Hz);
1.50 - 1.65 (2H, multiplet);
1.60 (6H, singlet);
3.00 (2H, triplet, J = 7 Hz);
4.20 (2H, quartet, J = 7 Hz);
5.63 (2H, singlet);
7.13-7.75 (8H, multiplet).

### 72(c) Ethyl 1-[(2'-carbamoylbiphenyl-4-yl)methyl]-4- (1-hydroxy-1-methylethyl)-2-propylimidazole-5-carboxylate

3 ml of oxalyl chloride were added dropwise, whilst ice-cooling, to a solution of 4.00 g of the hydrochloride of ethyl 1-[(2'-carboxybiphenyl-4-yl)- methyl]-4-(1-hydroxy-1-methylethyl)-2-propylimidazole-5-carboxylate [prepared as described in step (b) above] in 40 ml of methylene chloride, and the resulting mixture was stirred at room temperature for 2 hours. At the end of this time, the reaction mixture was concentrated by evaporation under reduced pressure. Benzene was then added to the residue, and the mixture was concentrated again by evaporation under reduced pressure, to remove the remaining oxalyl chloride. The crystalline residue was suspended in 100 ml of ethyl acetate and mixed with 15 ml of concentrated aqueous ammonia, whilst ice-cooling, and then the mixture was stirred at room temperature for 10 minutes. The ethyl acetate layer was separated, washed with water, dried over anhydrous magnesium sulphate and concentrated by evaporation under reduced pressure. The crystalline residue was then washed with diisopropyl ether, to give 2.97 g of the title compound, melting at 148 - 151 °C.

Nuclear Magnetic Resonance Spectrum (CDCl₃) δ ppm:
0.96 (3H, triplet, J = 7.5 Hz);
1.19 (3H, triplet, J = 7 Hz);
1.64 (6H, singlet);
1.73 (2H, sextet, J = 7.5 Hz);
2.65 (2H, triplet, J = 7.5 Hz);
4.24 (2H, quartet, J = 7 Hz);
5.36 (1H, broad singlet);
5.49 (2H, singlet);
5.66 (1H, broad singlet);
5.76 (1H, singlet);
6.99 (2H, doublet, J = 8 Hz);
7.32 - 7.53 (5H, multiplet);
7.71 (1H, doublet, J = 6 Hz).

### 72(d) Ethyl 1-(2'-cyanobiphenyl-4-yl)methyl)-4-(1-hydroxy1-methylethyl)-2-propylimidazole-5- carboxylate

264 mP of trifluoroacetic anhydride were added, whilst cooling on a bath containing a mixture of ice and sodium chloride, to a solution of 0.70 g of ethyl 1-[(2'-carbamoylbiphenyl-4-yl)methyl]-4-(1-hydroxy-1-methylethyl)-2-propylimidazole-5-carboxylate [prepared as described in step (c) above] and 0.43 ml of triethylamine in 7 ml of methylene chloride, and the resulting mixture was stirred at the same temperature for 30 minutes. At the end of this time, the reaction mixture was diluted with an aqueous solution of sodium hydrogencarbonate and ethyl acetate, and the ethyl acetate layer was separated, dried over anhydrous magnesium sulphate and concentrated by evaporation under reduced pressure. The residue was purified by column chromatography through silica gel, using a 1 : 1 by volume mixture of ethyl acetate and hexane as the eluent, to give 0.60 g of the title compound as crystals.

The melting point and Nuclear Magnetic Resonance Spectrum of this compound were identical with those of the compound obtained as described in Example 70 (a).

### 72(e) Ethyl 4-(1-hydroxy-1-methylethyl)-2-propyl-1- {4-[2-(tetrazol-5-yl)phenyl]phenyl}methyl-imidazole-5-carboxylate

Following a procedure similar to that described in Example 70(b), but using ethyl 1-(2'-cyanobiphenyl-4-yl) methyl-4-{1-hydroxy-1-methylethyl)-2-propylimidazole-5-carboxylate [prepared as described in step (d) above] the title compound was obtained in a 90% yield.

The Nuclear Magnetic Resonance Spectrum of this compound was identical with that of the compound obtained as described in Example 18(b).

### EXAMPLE 78

### (5-Methyl-2-oxo-1,3-dioxolen-4-yl)methyl 4-(1-hydroxy-1-methylethyl)-2-propyl-1-{4-[2-(tetrazol-5-yl)-phenyl]phenyl} methylimidazole-5-carboxylate (Compound No. 2-17)

### 78(a) (5-Methyl-2-oxo-1,3-dioxolen-4-yl)methyl 4-(1-hydroxy-1-methylethyl)-2-propyl-1-{4-[2-(trityl-tetrazol-5-yl) phenyl]phenyl}methylimidazole-5-carboxylate

A solution of 2.65 g of lithium hydroxide monohydrate in 158 ml of water was added, whilst ice-cooling, to a solution of 30 g of ethyl 4-(1-hydroxy-1-methylethyl)-2-propyl-1-{4-[2-(trityl-tetrazol-5-yl)phenyl]phenyl}methylimidazole-5-carboxylate [prepared as described in Example 18(a)] in 344 ml of dioxane, and the resulting mixture was stirred at 5 - 10°C for 20 hours. At the end of this time, small pieces of dry ice were added to the mixture, which was then concentrated by evaporation under reduced pressure to a volume of about 100 ml. Ethyl acetate and sodium chloride were added to the concentrate, and the mixture was stirred. The ethyl acetate layer was separated, dried over anhydrous sodium sulphate and concentrated by evaporation under reduced pressure, to give lithium 4-(1-hydroxy-1-methylethyl)-2-propyl-1-{4-[2-(trityltetrazol-5-yl)phenyl]phenyl}methyl-imidazole-5-carboxylate as a glass. 6.08 g of potassium carbonate were added, whilst ice-cooling, to a solution of whole of this lithium carboxylate in 160 ml of N,N-dimethylacetamide, and then a solution of 11.2 g of 4-chloromethyl-5-methyl-2-oxo-1,3-dioxolene (74% purity) in 26 ml of N,N-dimethylacetamide was added dropwise, whilst ice-cooling, to the mixture. The resulting mixture was stirred at 50°C for 3 hours. At the end of this time, water and ethyl acetate were added to the reaction mixture, and the ethyl acetate layer was separated, dried over anhydrous magnesium sulphate and concentrated by evaporation under reduced pressure. The residue was crystallized in diisopropyl ether, to give 29.3 g of the title compound as crystals, melting at 98 - 100°C (with decomposition).

The Nuclear Magnetic Resonance Spectrum of this compound was identical with that of the compound obtained as described in Example 61 (a).

### 78(b) (5-Methyl-2-oxo-1,3-dioxolen-4-yl)methyl 4-(1-hydroxy-1-methylethyl)-2-propyl-1-{4-[2- (tetrazol-5-yl)phenyl] phenyl}methylimidazole-5-carboxylate

75 ml of water were added to a suspension of 29.3 g of (5-methyl-2-oxo-1,3-dioxolen-4-yl)methyl 4-(1- hydroxy-1-methylethyl)-2-propyl-1-{4-[2-(trityl-tetrazol-5-yl)phenyl]phenyl}methylimidazole-5- carboxylate [prepared as described in step (a) above] in 225 ml of acetic acid, and the resulting mixture was stirred at 60°C for 1.5 hours. At the end of this time, 75 ml of water were added to the mixture, which was then cooled. Precipitated trityl alcohol was removed by filtration, and the filtrate was concentrated by evaporation under reduced pressure. Toluene was added to the residue, and the mixture was again concentrated by evaporation under reduced pressure, to remove the remaining water and acetic acid. The residue was crystallized in ethyl acetate, to give 16.6 g of the title compound as crystals, melting at 177 - 180°C (with decomposition).

The Nuclear Magnetic Resonance Spectrum of this compound was identical with that of the compound obtained as described in Example 61 (b).

### EXAMPLE 79

### (5-Methyl-2-oxo-1,3-dioxolen-4-yl)methyl 4-(1-hydroxy-1-methylethyl)-2-propyl-1-{4-[2-(tetrazol-5-yl)-phenyl)phenyl} methylimidazole-5-carboxylate (Compound No. 2-17)

### 79(a) Ethyl 4-(1-hydroxy-1-methylethyl)-2-propyl-1- {4-[2-(trityltetrazol-5-yl)phenyl]phenyl}- methylimidazole-5-carboxylate

A solution of 1.00 g of ethyl 1-(2'-cyanobiphenyl-4-yl)methyl-4-(1-hydroxy-1-methylethyl)-2-propyl-imidazole-5-carboxylate [prepared as described in Example 71 (b)] and 1.00 g of tributyltin azide in 7.5 ml of toluene was stirred at 100°C for 5 days. 2.5 g of sodium hydrogencarbonate and 20 ml of water were then added to the mixture, and the resulting mixture was stirred at room temperature for 8 hours. At the end of this time, the mixture was diluted with ethyl acetate and acidified with 3 N aqueous hydrochloric acid to a pH value of 3. The ethyl acetate layer was separated, dried over anhydrous magnesium sulphate and concentrated by evaporation under reduced pressure, to give ethyl 4-(1-hydroxy-1-methylethyl)-2-propyl-1-{4-[2-(tetrazol-5-yl)phenyl]phenyl}methylimidazole-5- carboxylate as a syrup. 0.80 g of trityl chloride was added to a solution of the whole of this syrup in 15 ml of pyridine, and the mixture was stirred at 60°C for 4 hours. At the end of this time, the reaction mixture was concentrated by evaporation under reduced pressure, and the residue was purified by column chromatography through silica gel, using a 1 : 1 by volume mixture of ethyl acetate and hexane as the eluent; it was then crystallized in diisopropyl ether, to give 1.15 g of the title compound as crystals.

The Nuclear Magnetic Resonance Spectrum of this compound was identical with that of the compound obtained as described in Example 18(a).

### 79(b) (5-Methyl-2-oxo-1,3-dioxolen-4-yl)methyl4-(1-hydroxy-1-methylethyl)-2-propyl-1-{4-[2- (tetrazol-5-yl)phenyl] phenyl}methylimidazole-5-carboxylate

Following procedures similar to those described in Example 78(a) and 78(b), but using ethyl 4-(1-hydroxy-1-methylethyl)-2-propyl-{4-[2-(trityltetrazol-5-yl)-phenyl]phenyl}methylimidazole-5-carboxylate [prepared as described in step (a) above], the title compound was obtained in a 71% yield.

The Nuclear Magnetic Resonance Spectrum of this compound was identical with that of the compound obtained as described in Example 61 (b).

### EXAMPLE 80

### Pivaloyloxymethyl 2-ethyl-4-(1-hydroxy-1-methylethyl)-1-{4-[2-(tetrazole-5-yl)phenyl]phenyl}methylimidazole-5-carboxylate (Compound No. 2-69)

### 80(a) Pivaloyloxymethyl 2-ethyl-4-(1-hydroxy-1-methyl-ethyl)-1-{4-[2-(trityltetrazole-5-yl)phenyl]-phenyl} methylimidazole-5-carboxylate

Following a procedure similar to that described in Example 78(a), but using 2.25 g of ethyl 2-ethyl-4-(1-hydroxy-1-methylethyl)-1-{4-[2-(trityltetrazole-5-yl)-phenyl]phenyl}methylimidazole-5-carboxylate [prepared as described in Example 68 (a)] and using 203 mg of lithium hydroxide monohydrate for hydrolysis and 0.90 g of pivaloyloxymethylchloride for esterification, 2.53 g of the title compound were obtained as a glass (purified by column chromatography through silica gel, using a 1 : 1 by volume mixture of ethyl acetate and hexane as the eluent). Nuclear Magnetic Resonance Spectrum (CDCl₃) δ ppm:
1.14 (9H, singlet);
1.19 (3H, triplet, J = 7.5 Hz);
1.64 (6H, singlet);
2.50 (2H, quartet, J = 7.5 Hz);
5.34 (2H, singlet);
5.43 (1H, singlet);
5.72 (2H, singlet);
6.73 (2H, doublet, J = 8 Hz);
6.92 - 7.49 (20H, multiplet);
7.90 (1H, doublet, J= 8.5 Hz).

### 80(b) Pivaloyloxymethyl 2-ethyl-4-(1-hydroxy-1-methyl-ethyl)-1-{4-[2-(tetrazole-5-yl)phenyl]phenyl}-methylimidazole-5-carboxylate

Following a procedure similar to that described in Example 78(b), but using 2.53 g of pivaloyloxymethyl 2-ethyl-4-(1-hydroxy-1-methylethyl)-1-{4-[2-(trityl-tetrazole-5-yl)phenyl]phenyl}methylimidazole-5- carboxylate [prepared as described in step (a) above] and 28 ml of 75% v/v aqueous acetic acid, 1.70 g of the title compound was obtained as a glass.

Nuclear Magnetic Resonance Spectrum (CDCl₃) δ ppm:
1.09 (9H, singlet);
1.24 (3H, triplet, J = 7.5 Hz);
1.59 (6H, singlet);
2.64 (2H, quartet, J = 7.5 Hz);
5.41 (2H, singlet);
5.79 (2H, singlet);
6.86 (2H, doublet, J = 8.5 Hz);
7.11 (2H, doublet, J = 8.5 Hz);
7.42-7.62 (4H, multiplet).

### EXAMPLE 81

### (5-Methyl-2-oxo-1,3-dioxolen-4-yl)methyl 2-ethyl-4-(1-hydroxy-1-methylethyl)-1-{4-[2-(tetrazole-5-yl)phenyl]phenyl} methylimidazole-5-carboxylate (Compound No. 2-73)

### 81(a) (5-Methyl-2-oxo-1,3-dioxolen-4-yl)methyl 2-ethyl-4-(1-hydroxy-1-methylethyl)-1-{4-[2-(trityl-tetrazole-5-yl) phenyl]phenyl}methylimidazole-5- carboxylate

Following a procedure similar to that described in Example 78(a), but using 2.25 g of ethyl 2-ethyl-4-(1-hydroxy-1-methylethyl)-1-(4-[2-(trityltetrazole-5-yl)-phenyl]phenyl)methylimidazole-5-carboxylate [prepared as described in Example 68 (a)] and using 203 mg of lithium hydroxide monohydrate for hydrolysis and 0.95 g of 4-chloromethyl-5-methyl-2-oxo-1,3-dioxolene (74% purity) for esterification, 1.23 g of the title compound was obtained as crystals, melting at 145°C.

Nuclear Magnetic Resonance Spectrum (CDCl₃) δ ppm:
1.21 (3H, triplet, J = 7.5 Hz);
1.63 (6H, singlet);
1.98 (3H, singlet);
2.55 (2H, quartet, J = 7.5 Hz);
4.73 (2H, singlet);
5.30 (2H, singlet);
5.59 (1H, singlet);
6.69 (2H, doublet, J = 8 Hz);
6.90-7.53 (20H, multiplet);
7.87 (1H, doublet, J = 8 Hz).

### 81(b) (5-Methyl-2-oxo-1,3-dioxolen-4-yl)methyl 2-ethyl-4-(1-hydroxy-1-methylethyl)-1-{4-[2-(tetrazole-5-yl)phenyl] phenyl}methylimidazole-5-carboxylate

Following a procedure similar to that described in Example 78(b), but using 1.90 g of (5-methyl-2-oxo-1,3-dioxolen-4-yl)methyl 2-ethyl-4-(1-hydroxy-1-methyl-ethyl)-1-{4-[2-(trityltetrazole-5-yl)phenyl]phenyl}- methylimidazole-5-carboxylate [prepared as described in step (a) above] and 20 ml of 75% v/v aqueous acetic acid, 1.23 g of the title compound was obtained as a crystalline powder.

Nuclear Magnetic Resonance Spectrum (CDCl₃ and hexadeuterated dimethyl sulphoxide) δ ppm:
1.24 (3H, triplet, J = 7.5 Hz);
1.54 (6H, singlet);
2.10 (3H, singlet);
2.69 (2H, quintet, J=7.5 Hz);
4.99 (2H, singlet);
5.44 (2H, singlet);
6.86 (2H, doublet, J=8.5 Hz);
7.08 (2H, doublet, J=8.5 Hz);
7.50 - 7.65 (4H, multiplet).

### PREPARATION 1

### 2-Butylimidazole-4,5-dicarbonitrile

A suspension of 51.4 g of diaminomaleonitrile and 85.6 g of trimethyl orthovalerate in 300 ml of acetonitrile was stirred in an oil bath kept at 85°C for 6 hours. At the end of this time, the reaction mixture was concentrated by evaporation under reduced pressure, and the concentrate was purified by short column chromatography through silica gel, using a 1 : 1 by volume mixture of ethyl acetate and hexane as the eluent, to give 99 g of 1-amino-2-N-(1-methoxypentylidene)aminosuccinonitrile. The whole of this compound was dissolved in 300 ml of xylene, and the resulting solution was stirred in an oil bath kept at 150°C for 8 hours, after which the reaction mixture was concentrated to half its original volume and allowed to stand at room temperature. The crystals which precipitated were collected by filtration and washed with a small amount of xylene, to give 55.2 g of the title compound, melting at 109 - 111 °C.

### PREPARATION 2

### 2 -Butylimidazole -4,5-dicarboxylic acid

A solution of 100 g of 2-butylimidazole-4,5-dicarbonitrile (prepared as described in Preparation 1) in 1 litre of 6 N aqueous hydrochloric acid was heated under reflux for 7 hours, and then the reaction mixture was allowed to stand overnight at room temperature. At the end of this time, the crystals which precipitated were collected by filtration and washed with water and with a small amount of acetone, to give 84 g of the title compound, melting at 261 - 263°C.

### PREPARATION 3

### Diethyl 2-butylimidazole-4,5-dicarboxylate

Dry hydrogen chloride was bubbled through a suspension of 40 g of 2-butylimidazole-4,5-dicarboxylic acid (prepared as described in Preparation 2) in 600 ml of ethanol at room temperature, whilst stirring, for 2 hours to yield a solution. This solution was allowed to stand at room temperature for 18 hours, after which the reaction mixture was concentrated by evaporation under reduced pressure. The concentrate was then mixed with ethyl acetate and with an aqueous solution of sodium hydrogencarbonate and neutralized by adding powdery sodium hydrogencarbonate. The ethyl acetate layer was separated and dried over anhydrous magnesium sulphate, and the solvent was removed by distillation under reduced pressure. The resulting crystalline residue was triturated with a mixture of diisopropyl ether and hexane, and collected by filtration, to give 43 g of the title compound, melting at 82 - 84°C.

### PREPARATION 4

### Dimethyl 2-butylimidazole-4,5-dicarboxylate

A procedure similar to that described in Preparation 3 was repeated, using 40 g of 2-butyl-imidazole-4,5-dicarboxylic acid, and except that methanol was used instead of ethanol, to give 41.6 g of the title compound as crystals, melting at 88°C.

### PREPARATION 5

### 4-Acetyl-2-butyl-5-cyanoimidazole

### 5(i) 2-Butyl-1-tritylimidazole-4,5-dicarbonitrile

1.25 g of sodium hydride (as a 55% w/w dispersion in mineral oil) were added, whilst ice-cooling, to a solution of 5 g of 2-butylimidazole-4,5-dicarbonitrile (prepared as described in Preparation 1) in 50 ml of N,N-dimethylformamide, and the resulting mixture was stirred for 15 minutes. 10 g of trityl chloride were then added, and the reaction mixture was stirred at 50°C for 6 hours. At the end of this time, it was mixed with ethyl acetate and water, and the product was extracted with ethyl acetate. The extract was dried over anhydrous magnesium sulphate, and the solvent was removed by distillation under reduced pressure. The resulting residue was purified by column chromatography through silica gel, using a 1 : 5 by volume mixture of ethyl acetate and hexane as the eluent, to give 9.83 g of the title compound as a syrup, which solidified on being allowed to stand. The solid melted at 144 - 147°C (with decomposition and colouration at 94 - 98°C) .

Nuclear Magnetic Resonance Spectrum (CDCl₃), δ ppm:
0.60 (3H, triplet, J = 7 Hz);
0.5 - 1.4 (4H, multiplet) ;
2.03 (2H, triplet, J = 7 Hz);
7.0 - 7.6 (15H, multiplet).

### 5 (ii) 4-Acetyl-2-butyl-5-cyano-1-tritylimidazole

11.1 ml of a 2 M solution of methylmagnesium iodide in diethyl ether was slowly added dropwise at room temperature, in an atmosphere of nitrogen, to a solution of 4.5 g of 2-butyl-1-tritylimidazole-4,5-dicarbonitrile [prepared as described in step (i) above] in 45 ml of tetrahydrofuran., and the resulting mixture was stirred at room temperature for 3 hours. At the end of this time, a saturated aqueous solution of ammonium chloride was added dropwise, whilst ice-cooling, to the mixture. The tetrahydofuran layer was separated, washed with a saturated aqueous solution of sodium chloride and concentrated by evaporation under reduced pressure to give a concentrate. The aqueous layer was once again extracted with a small amount of ethyl acetate. The extract was washed with a saturated aqueous solution of sodium chloride, dried and concentrated by evaporation under reduced pressure. The resulting extract was combined with the above concentrate, and the resulting crude product was purified by column chromatography through silica gel, using a 3 : 1 by volume mixture of hexane and ethyl acetate as the eluent, and the product was crystallized from a mixture of ethyl acetate and hexane, to give 1.46 g of the title compound, melting at 159 - 160°C (with decomposition):

Nuclear Magnetic Resonance Spectrum (CDCl₃), δ ppm:
0.60 (3H, triplet, J = 7 Hz);
0.5 - 1.5 (4H, multiplet);
2.08 (2H, triplet, J = 7 Hz);
2.58 (3H, singlet);
7.1 - 7.6 (15H, multiplet).

### 5(iii) 4-Acetyl-2-butyl-5-cyanoimidazole

A suspension of 1.78 g of 4-acetyl-2-butyl-5-cyano-1-tritylimidazole [prepared as described in step (ii) above] in 80% v/v aqueous acetic acid was stirred at 60°C for 1 hour. The solution thus obtained was concentrated to dryness by evaporation under reduced pressure. The resulting residue was purified by column chromatography through silica gel, using a 3 : 1 by volume mixture of hexane and ethyl acetate as the eluent, to give 0.66 g of the title compound as a colourless solid, melting at 77 - 78°C.

Nuclear Magnetic Resonance Spectrum (CDCl₃), δ ppm:
0.93 (3H, triplet, J = 7 Hz);
1.0-2.1 (4H, multiplet);
2.72 (3H, singlet);
2.89 (2H, triplet, J = 7 Hz).

### PREPARATION 6

### 4-Benzoyl-2-butyl-5-cyanoimidazole

### 6(i) 4-Benzoyl-2-butyl-5-cyano-1-tritylimidazole

Following a procedure similar to that described in Preparation 5(ii), 10.3 g of the title compound were obtained as an amorphous solid by reacting a solution of 10 g of 2-butyl-1-tritylimidazole-4,5-dinitrile [prepared as described in Preparation 5(i)] in 100 ml of tetrahydrofuran with 25 ml of a 2 M solution of phenyl-magnesium iodide in diethyl ether.

Nuclear Magnetic Resonance Spectrum (CDCl₃), δ ppm:
0.67 (3H, triplet, J = 7 Hz);
0.5 - 1.5 (4H, multiplet);
2.11 (2H, triplet, J = 7 Hz);
7.1 - 8.0 (20H, multiplet).

### 6(ii) 4-Benzoyl-2-butyl-5-cyanoimidazole

A suspension of 10.3 g of 4-benzoyl-2-butyl-5- cyano-1-tritylimidazole [prepared as described in step (i) above] in 80% v/v aqueous acetic acid was stirred at 60°C for 5 hours. At the end of this time, the solution thus obtained was concentrated by evaporation under reduced pressure, and the concentrate was purified by column chromatography through silica gel, using a 2 : 1 by volume mixture of hexane and ethyl acetate as the eluent. The resulting oily product was dissolved in carbon tetrachloride and the solution was allowed to stand at room temperature, to precipitate crystals, which were collected by filtration to give 4.46 g of the title compound, melting at 121 - 122°C.

Nuclear Magnetic Resonance Spectrum (CDCl₃), δ ppm:
0.90 (3H, triplet, J = 7 Hz);
1.0 - 2.3 -(4H, multiplet);
2.85 (2H, triplet, J = 7 Hz);
7.2 - 8.0 (5H, multiplet);
11.0-12.1 (1H, broad).

### PREPARATION 7

### 2-Butyl-5-cyano-4-(1-hydroxy-1-methylethyl)imidazole

### 7(i) 2-Butyl-5-cyano-4-(1-hydroxy-1-methylethyl)-1-tritylimidazole

1 ml of a 2 M solution of methylmagnesium iodide in tetrahydrofuran was added dropwise at room temperature, whilst stirring, to a solution of 840 mg of 4-acetyl-2-butyl-5-cyano-1 tritylimidazole [prepared as described in Preparation 5(ii)] in 15 ml of tetrahydrofuran, and the resulting mixture was stirred at 40°C for 1 hour. The mixture was cooled, and then a saturate aqueous solution of ammonium chloride was added to it dropwise. The tetrahydrofuran layer-was separated and concentrated by evaporation under reduced pressure. The concentrate was purified by column chromatography through silica gel, using a 2 : 1 by volume mixture of hexane and ethyl acetate as the eluent, to give 539 mg of the title compound as a colourless solid, melting at 151 - 152°C.

Nuclear Magnetic Resonance Spectrum (CDCl₃), δ ppm:
0.60 (3H, triplet, J = 7 Hz);
0.6 - 1.5 (4H, multiplet);
1.59 (6H, singlet);
2.01 (2H, triplet, J = 7 Hz);
3.78 (1H, singlet);
7.0 - 7.6 (15H, multiplet).

### 7(ii) 2-Butyl-5-cyano-4-(1-hydroxy-1-methylethyl)-imidazole

A mixture of 1.3 g of 2-butyl-5-cyano-4-(1-hydroxy-1-methylethyl)-1-tritylimidazole [prepared as described in step (i) above] and 26 ml of 75% v/v aqueous acetic acid was stirred at 50° C for 3 hours, and then the solvent was removed by distillation under reduced pressure. The resulting residue was washed with carbon tetrachloride and purified by column chromatography through silica gel, using a 10 : 1 by volume mixture of methylenechloride and methanol as the eluent, and the product was crystallized in carbon tetrachloride, to give 0.6 g of the title compound as colourless crystals, melting at 171 - 172°C.

Nuclear Magnetic Resonance Spectrum (CDCl₃ + CD₃OD), δ ppm:
0.90 (3H, triplet, J = 7 Hz);
1.0 - 2.0 (4H, multiplet);
1.62 (6H, singlet);
2.68 (2H, triplet, J = 7 Hz).

### PREPARATION 8

### Ethyl 2-butyl-4-(1-hydroxy-1-methylethyl) imidazole-5-carboxylate

A solution of 5.36 g of diethyl 2-butylimidazole-4,5-dicarboxylate (prepared as described in Preparation 3) in 100 ml of tetrahydrofuran was cooled to -30°C in an atmosphere of nitrogen, and 32 ml of a methyl-magnesium bromide solution (2.5 M in tetrahydrofuran) were added dropwise at -30 to -20°C to the cooled solution. The reaction mixture was then stirred at 0°C for 1.5 hours and subsequently mixed with ethyl acetate and with an aqueous solution of ammonium chloride. The ethyl acetate layer was separated and dried over anhydrous magnesium sulphate, and the solvent was removed by distillation under reduced pressure. The residue was purified by column chromatography through silica gel, using a 1 : 20 by volume mixture of methanol and methylene chloride as the eluent, to give 5.01 g of the title compound as an oil.

Nuclear Magnetic Resonance Spectrum (CDCl₃), δ ppm:
0.90 (3H, triplet, J = 7 Hz);
1.32 (3H, triplet, J = 7 Hz);
1.2 - 2.0 (4H, multiplet);
1.64 (6H, singlet);
2.70 (2H, triplet, J = 7 Hz);
4.33 (2H, quartet, J = 7 Hz) ;
5.97 (1H, broad singlet);
10.2 (1H, broad singlet).

### PREPARATION 9

### Ethyl 4-(1-hydroxy-1-methylethyl)-2-propylimidazole-5-carboxylate

Following a procedure similar to that described in Preparation 8, 2.34 g of the title compound were obtained as an oil by reacting 3.01 g of diethyl 2-propylimidazole-4,5-dicarboxylate (prepared as described in Preparation 12) with 16 ml of a 2.5 M solution of methylmagnesium bromide in tetrahydrofuran. The compound was crystallized by allowing it to stand at room temperature, to give a product melting at 69 - 71 °C, and was then recrystallized from diisopropyl ether, to give a product melting at 101 - 102°C.

Nuclear Magnetic Resonance Spectrum (CDCl₃), δ ppm:
0.96 (3H, triplet, J = 7 Hz);
1.35 (3H, triplet, J = 7 Hz);
1.64 (6H, singlet);
1.81 (2H, sextet, J = 7 Hz);
2.68 (2H, triplet, J = 7 Hz);
4.35 (2H, quartet, J = 7 Hz);
5.81 (1H, singlet);
9.9 (1H, broad singlet).

### PREPARATION 10

### 2 -Propylimidazole-4,5-dicarbonitrile

Following a procedure similar to that described in Preparation 1, but using 16.0 g of diaminomaleonitrile and 24 g of trimethyl orthobutyrate, 18.7 g of the title compound were obtained as crystals, melting at 141 - 144°C.

### PREPARATION 11

### 2-Propylimidazole-4,5-dicarboxylic acid

Following a procedure similar to that described in Preparation 2, but using 18.2 g of 2-propylimidazole-4,5-dicarbonitrile (prepared as described in Preparation 10), 9.95 g of the title compound were obtained as crystals, melting at 261 - 263°C.

### PREPARATION 12

### Diethyl 2-propylimidazole-4,5-dicarboxylate

Following a procedure similar to that described in Preparation 3, but using 10.0 g of 2-propylimidazole-4,5-dicarboxylic acid (prepared as described in Preparation 11), 9.55 g of the title compound were obtained as crystals, melting at 81 - 83°C.

### PREPARATION 13

### Ethyl 2-butyl-4-(1-ethyl-1-hydroxypropyl) imidazole-5-carboxylate

Following a procedure similar to that described in Preparation 8, 2.68 g of the title compound, melting at 63 - 64°C, were obtained as crystals by reacting 2.68 g of diethyl 2-butylimidazole-4,5-dicarboxylate (prepared as described in Preparation 3) with a 3.0 M solution of ethylmagnesium bromide in diethyl ether.

Nuclear Magnetic Resonance Spectrum (CDCl₃), δ ppm:
0.82 (6H, triplet, J = 7 Hz);
0.93 (3H, triplet, J = 7 Hz);
1.38 (3H, triplet, J = 7 Hz);
1.31 - 1.45 (2H, multiplet);
1.65 - 1.76 (2H, multiplet);
1.79 - 1.89 (2H, multiplet);
1.97-2.11 (2H, multiplet);
2.76 (2H, triplet, J = 7.5 Hz);
4.36 (2H, quartet, J = 7 Hz);
5.70 (1H, broad singlet).

### PREPARATION 14

### 2-Propyl-1-tritylimidazole-4,5-dicarbonitrile

Following a procedure similar to that described in Preparation 5(i), but using 7.8 g of 2-propylimidazole-4,5-dicarbonitrile (prepared as described in Preparation 10), 2.14 g of sodium hydride (as a 55% w/w dispersion in mineral oil) and 17.1 g of trityl chloride, 14.6 g of the title compound were obtained as crystals, melting at 107°C (with decomposition and with a yellow colouration at 102°C) .

Nuclear Magnetic Resonance Spectrum (CDCl₃), δ ppm:
0.52 (3H, triplet, J = 7 Hz);
1.07-1.21 (2H, multiplet);
2.03 (2H, triplet, J = 8 Hz);
7.19 - 7.48 (15H, multiplet).

### PREPARATION 15

### 2-Butyl-5-cyano-4-propionyl-1-tritylimidazole

14 ml of a 3 M solution of ethylmagnesium bromide in diethyl ether were added dropwise at 10°C under an atmosphere of nitrogen to a solution of 8.33 g of 2-butyl-1-tritylimidazole-4,5-dicarbonitrile [prepared as described in Preparation 5(i)] in 83 ml of tetrahydrofuran, and the resulting mixture was stirred at room temperature for 3 hours. At the end of this time, a mixture of a saturated aqueous solution of ammonium chloride and ethyl acetate was added to the reaction mixture, whilst ice-cooling. The ethyl acetate layer was separated, washed with water and dried over anhydrous magnesium sulphate. The solvent was then removed by distillation under reduced pressure. The crystalline product thus obtained was washed with diisopropyl ether, to give 4.56 g of the title compound, melting at 140 - 143°C (softening at 83°C).

Nuclear Magnetic Resonance Spectrum (CDCl₃), δ ppm:
0.61 (3H, triplet, J = 7 Hz);
0.84 - 1.14 (4H, multiplet);
1.18 (3H, triplet, J = 8 Hz);
2.08 (2H, triplet, J = 7 Hz);
3.03 (2H, quartet, J = 7 Hz);
7.22-7.42 (15H, multiplet).

### PREPARATION 16

### 5-Cyano-4-propionyl-2-propyl-1-tritylimidazole

Following a procedure similar to that described in Preparation 15, but using 8.05 g of 2-propyl-1-trityl-imidazole-4,5-dicarbonitrile (prepared as described in Preparation 14) and 14 ml of a 3 M solution of ethylmagnesium bromide in diethyl ether, 7.03 g of the title compound were obtained as crystals, melting at 96°C (softening at 87°C).

Nuclear Magnetic Resonance Spectrum (CDCl₃), δ ppm:
0.52 (3H, triplet, J = 7 Hz);
1.05 - 1.3 (2H, multiplet);
1.18 (3H, triplet, J = 7 Hz);
2.05 (2H, triplet, J = 7 Hz);
3.03 (2H, quartet, J = 7 Hz);
7.20-7.40 (15H, multiplet).

### PREPARATION 17

### 2-Butyl-5-cyano-4-(1-hydroxy-1-methylpropyl)-1-tritylimidazole

5 ml of a 1 M solution of methylmagnesium bromide in tetrahydrofuran were added dropwise at 10°C under an atmosphere of nitrogen to a solution of 2 g of 2-butyl-5-cyano-4-propionyl-1-tritylimidazole (prepared as described in Preparation 15) in 36 ml of tetrahydrofuran, and the resulting mixture was stirred at 20°C for 1 hour and subsequently at 30°C for a further 1 hour. At the end of this time, a mixture of a saturated aqueous solution of ammonium chloride and ethyl acetate was added to the reaction mixture, which was then well shaken. The ethyl acetate layer was separated and dried over anhydrous magnesium sulphate. After the drying agent had been removed by filtration, the solvent was distilled off under reduced pressure. The residue was purified by column chromatography through silica gel, using a 1 : 2 by volume mixture of ethyl acetate and hexane as the eluent, to give 1.29 g of the title compound as crystals, melting at 90 - 93°C.

Nuclear Magnetic Resonance Spectrum (CDCl₃), δ ppm:
0.60 (3H, triplet, J = 7 Hz);
0.80 (3H, triplet, J = 7 Hz);
0.80-1.00(2H, multiplet);
1.00-1.13(2H, multiplet);
1.58 (3H, singlet);
1.75 - 2.05 (4H, multiplet);
3.90 (1H, broad singlet);
7.23 - 7.43 (15H, multiplet).

### PREPARATION 18

### 5-Cyano-4-(1-hydroxy-1-methylpropyl)-2-propyl-1-tritylimidazole

Following the procedure described in Preparation 17, but using 5.00 g of 5-cyano-4-propionyl-2-propyl-1-tritylimidazole (prepared as described in Preparation 16) and 12.5 ml of a 1 M solution of methylmagnesium bromide in tetrahydrofuran, 3.32 g of the title compound were obtained as a crystalline powder, melting at above 120°C (softening at 110°C).

Nuclear Magnetic Resonance Spectrum (CDCl₃), δ ppm:
0.50 (3H, triplet, J = 7 Hz);
0.80 (3H, triplet, J = 7 Hz);
1.07 - 1.12 (2H, multiplet);
1.58 (3H, singlet);
1.74 - 2.00 (4H, multiplet);
3.90 (1H, broad singlet);
7.24 - 7.37 (15H, multiplet).

### PREPARATION 19

### 2-Butyl-5-cyano-4-(1-hydroxy-1-methylpropyl)imidazole

A mixture of 1.21 g of 2-butyl-5-cyano-4-(1-hydroxy-1-methylpropyl)-1-tritylimidazole (prepared as described in Preparation 17) and 20 ml of 75% v/v aqueous acetic acid was stirred at 50°C for 1 hour, after which the mixture was cooled, and the deposited crystals of trityl alcohol were removed by filtration. The filtrate was concentrated by evaporation under reduced pressure, and the remaining water and acetic acid were distilled off as a toluene azeotrope under reduced pressure. The residue was purified by column chromatography through silica gel, using a 9 : 1 by volume mixture of methylene chloride and methanol as the eluent, to give 0.47 g of the title compound as a crystalline powder, melting at 139 - 142° C.

Nuclear Magnetic Resonance Spectrum (hexadeuterated dimethyl sulphoxide), δ ppm:
0.74 (3H, triplet, J = 7 Hz);
0.87 (3H, triplet, J = 7 Hz);
1.21 - 1.34 (2H, multiplet);
1.49 (3H, singlet);
1.53 - 1.64 (2H, multiplet);
1.72 (2H, quartet, J = 7.5 Hz);
2.56 (2H, triplet, J = 7 Hz);
5.45 (1H, singlet).

### PREPARATION 20

### 5-Cyano-4-(1-hydroxy-1-methylpropyl)-2-propylimidazole

Following the procedure described in Preparation 19, but using 1.20 g of 5-cyano-4-(1-hydroxy-1-methyl-propyl)-2-propyl-1-tritylimidazole (prepared as described in Preparation 18), 0.48 g of the title compound was obtained as a crystalline powder, melting at 157 - 159°C.

Nuclear Magnetic Resonance Spectrum (CDCl₃), δ ppm:
0.89 (3H, triplet, J = 7.5 Hz);
0.98 (3H, triplet, J = 7.5 Hz);
1.57 (3H, singlet);
1.76 (2H, quartet, J = 7.5 Hz);
1.83 - 2.08 (2H, multiplet);
2.00 (1H, singlet);
2.67 (2H, triplet, J = 7.5 Hz).

### PREPARATION 21

### Methyl 2-butyl-4-(1-hydroxy-1-methylethyl)imidazole-5-carboxylate

A solution of 9.73 g of dimethyl 2-butylimidazole-4,5-dicarboxylate (prepared as described in Preparation 4) in 100 ml of tetrahydrofuran was cooled to -30°C under an atmosphere of nitrogen, and 162 ml of a 1 M solution of methylmagnesium bromide in tetrahydrofuran were added dropwise to this solution at a temperature of -30°C to -20°C. The resulting mixture was stirred at 0°C for 2.5 hours, and then ethyl acetate and an aqueous solution of ammonium chloride were added to it. The ethyl acetate layer was separated and dried over anhydrous magnesium sulphate, after which the solvent was removed by distillation under reduced pressure. The resulting residue was purified by column chromatography through silica gel, using a 1 : 20 by volume mixture of methanol and methylene chloride as the eluent, to give 7.15 g of the title compound as an oil. The compound was crystallized by allowing it to stand at room temperature, to give a product melting at 60 - 65°C.

Nuclear Magnetic Resonance Spectrum (CDCl₃), δ ppm:
0.88 (3H, triplet, J = 7Hz);
1.0 - 2.0 (4H, multiplet);
1.64 (6H, singlet);
2.69 (2H, triplet, J = 7.5 Hz);
3.84 (3H, singlet);
7.35 (2H, broad singlet).

### PREPARATION 22

### Pivaloyloxymethyl 4-(1-hydroxy-1-methylethyl)-2-propylimidazole-5-carboxylate

### 22 (i) 4-(1-Hydroxy-1-methylethyl)-2-propylimidazole-5-carboxylic acid

A solution of 0.28 g of lithium hydroxide monohydrate in 5 ml of water was added to a solution of 0.48 g of ethyl 4-(1-hydroxy-1-methylethyl)-2-propyl-imidazole-5-carboxylate (prepared as described in Preparation 9) in 5 ml of methanol, and the resulting mixture was stirred at room temperature for 18 hours. At the end of this time, the pH of the reaction mixture was adjusted to a value of 2.3 by adding 6.67 ml of 1 N aqueous hydrochloric acid, and the mixture was concentrated by evaporation under reduced pressure to a volume of about 2 ml. The crystals which precipitated were collected by filtration to give 0.20 g of the title compound, melting at 232°C (with decomposition).

Nuclear Magnetic Resonance Spectrum (hexadeuterated dimethyl sulphoxide), δ ppm:
0.87 (3H, triplet, J = 7.5 Hz);
1.48 (6H, singlet);
1.65 (2H, sextet, J = 7.5 Hz);
2.62 (2H, triplet, J = 7.5 Hz).

### 22 (ii) Pivaloyloxymethyl 4-(1-hydroxy-1-methylethyl)-2-propylimidazole-5-carboxylate

1.76 ml of N,N-diisopropylethylamine were added to a suspension of 1.14 g of 4-(1-hydroxy-1- methylethyl)-2-propylimidazole-5-carboxylic acid [prepared as described in step (i) above] in 12 ml of N,N-dimethylacetamide, and the resulting mixture was stirred at room temperature for 10 minutes; 1.36 ml of pivaloyloxymethyl chloride was then added. The reaction mixture was stirred at 60°C for 4 hours, after which it was mixed with ethyl acetate and water. The ethyl acetate layer was separated and concentrated by evaporation under reduced pressure. The crystals which precipitated were triturated with diisopropyl ether and collected by filtration to give 1.53 g of the title compound, melting at 177°C.

Nuclear Magnetic Resonance Spectrum (CDCl₃), δ ppm:
0.99 (3H, triplet, J = 7.5 Hz);
1.22 (9H, singlet);
1.62 (6H, singlet);
1.76 (2H, sextet, J = 7.5 Hz);
2.70 (2H, triplet, J = 7.5 Hz);
5.15 (1H, broad singlet);
5.95 (2H, singlet).

### PREPARATION 23

### Ethyl 4-(1-hydroxyethyl)-2-propylimidazole-5-carboxylate

### 23 (i) 4-Acetyl-2-propylimidazole-5-carbonitrile

194 ml of a 1 M solution of methylmagnesium bromide in tetrahydrofuran were added dropwise at a temperature of 10°C to 15°C and under an atmosphere of nitrogen to a solution of 10 g of 2-propylimidazole-4,5-dicarbonitrile (prepared as described in Preparation 10) in 100 ml of tetrahydrofuran, and the resulting mixture was stirred at a temperature of 10° C to 15°C for 30 minutes. The reaction mixture was then cooled, and 200 ml of ethyl acetate and 100 ml of a. saturated aqueous solution of ammonium chloride were added to it. The mixture was then acidified by adding an aqueous solution of potassium bisulphate. The organic layer was separated and dried over anhydrous magnesium sulphate, and the solvent was removed by distillation under reduced pressure. The residue was subjected to column chromatography through silica gel, using a 1 : 1 by volume mixture of ethyl acetate and hexane as the eluent, to give 9.18 g of the title compound as crystals, melting at 93 - 95° C.

Nuclear Magnetic Resonance Spectrum (CDCl₃), δ ppm:
0.99 (3H, triplet, J = 7.5 Hz);
1.83 (2H, sextet, 7.5 Hz);
2.71 (3H, singlet);
2.82 (2H, triplet, J = 8 Hz).

### 23(ii) Ethyl 4-acetyl-2-propylimidazole-5-carboxylate

A mixture of 4.0 g of 4-acetyl-2-propylimidazole-5-carbonitrile [prepared as described in step (i) above] and 60 ml of 6 N aqueous hydrochloric acid was heated under reflux, with stirring, for 8 hours. The reaction mixture was then concentrated by evaporation under reduced pressure, and the concentrate was dissolved in ethanol, after which it was again concentrated in the same way. The residue was dissolved in ethanol and the solvent was again distilled off. After this sequence of dissolution and concentration had been carried out for a total of five times, the residue was dissolved in 60 ml of ethanol. A stream of hydrogen chloride was bubbled through the resulting solution at room temperature for 20 minutes, and then the solution was allowed to stand at room temperature for 16 hours. It was then concentrated by evaporation under reduced pressure. The concentrate was dissolved in a mixture of ethyl acetate and an aqueous solution of sodium hydrogencarbonate, and the solution was neutralized by adding sodium hydrogencarbonate. The ethyl acetate layer was separated and dried over anhydrous magnesium sulphate. The solvent was removed by distillation under reduced pressure, and the resulting residue was purified by column chromatography through silica gel, using a 1 : 1 by volume mixture of ethyl acetate and hexane as the eluent, to give 3.07 g of the title compound as crystals, melting at 76 - 78°C.

Nuclear Magnetic Resonance Spectrum (CDCl₃), δ ppm:
0.96 (3H, triplet, J = 7.5 Hz);
1.39 (3H, triplet, J = 7 Hz);
1.82 (2H, sextet, J = 7.5 Hz);
2.75 (3H, singlet);
2.80 (2H, triplet, J = 7.5 Hz);
4.44 (2H, quartet, J = 7 Hz).

### 23(iii) Ethyl 4-(1-hydroxyethyl)-2-propylimidazole-5-carboxylate

125 mg of sodium borohydride were added to a solution of 1.50 g of ethyl 4-acetyl-2-propylimidazole-5-carboxylate [prepared as described in step (ii) above] in 15 ml of ethanol, and the resulting mixture was stirred at room temperature for 30 minutes. 2 ml of acetone were added, and the mixture was stirred for a further 10 minutes. The reaction mixture was then concentrated by evaporation under reduced pressure, and the concentrate was dissolved in methanol. The solution was again concentrated by evaporation under reduced pressure, and the residue was purified by column chromatography through silica gel, using 1 : 20 and 1 : 10 by volume mixtures of methylene chloride and methanol as the eluent, to give 1.32 g of the title compound as crystals, melting at 151 - 153°C.

Nuclear Magnetic Resonance Spectrum (CDCl₃ + hexadeuterated dimethyl sulphoxide), δ ppm:
0.95 (3H, triplet, J = 7.5 Hz);
1.38 (3H, triplet, J = 7 Hz);
1.48 (3H, doublet, J = 6.5 Hz);
1.74 (2H, sextet, J = 7.5 Hz);
2.67 (2H, triplet, J = 8 Hz);
4.34 (2H, quartet, J=7Hz):
5.28 (1H, quartet, J = 6.5 Hz).

### PREPARATION 24

### Ethyl 2-butyl-4-(1-hydroxyethyl)imidazole-5-carboxylate

### 24 (i) 4-Acetyl-2-butylimidazole-5-carbonitrile

Following a procedure similar to that described in Preparation 23(i), but using 10 g of 2-butylimidazole-4,5-dicarbonitrile (prepared as described in Preparation 1), 9.15 g of the title compound were obtained as crystals, melting at 77 - 78°C.

Nuclear Magnetic Resonance Spectrum (CDCl₃), δ ppm:
0.93 (3H, triplet, J = 7Hz);
1.0-2.1 (4H, multiplet);
2.72 (3H, singlet);
2.89 (2H, triplet, J = 7Hz).

### 24(ii) Ethyl 4-acetyl-2-butylimidazole-5-carboxylate

Following a procedure similar to that described in Preparation 23(ii), but using 1.00 g of 4-acetyl-2-butylimidazole-5-carbonitrile [prepared as described in step (i) above], 0.92 g of the title compound was obtained as a viscous oil.

Nuclear Magnetic Resonance Spectrum (CDCl₃), δ ppm:
0.88 (3H, triplet, J = 7 Hz);
1.1 - 2.1 (4H, multiplet);
1.33 (3H, triplet, J = 7 Hz);
2.74 (3H, singlet);
2.82 (2H, triplet, J = 7.5 Hz);
4.38 (2H, quartet, J = 7 Hz).

### 24(iii) Ethyl 2-butyl-4-(1-hydroxyethyl)imidazole-5-carboxylate

Following a procedure similar to that described in Preparation 23(iii), but using 0.64 g of ethyl 4-acetyl-2-butylimidazole-5-carboxylate [prepared as described in step (ii) above], 0.55 g of the title compound was obtained as crystals, melting at 149°C.

Nuclear Magnetic Resonance Spectrum (CDCl₃ + hexadeuterated dimethyl sulphoxide), δ ppm:
0.91 (3H, triplet, J=7.5 Hz);
1.37 (3H, triplet, J = 7 Hz);
1.3-1.42(2H. multiplet);
1.50 (3H, doublet, J = 6.5 Hz);
1.69 (2H, quintet, J = 7.5 Hz);
2.69 (2H, triplet, J = 8 Hz);
4.34 (2H, quartet, J = 7 Hz);
5.26 (1H, quartet, J = 6.5 Hz).

### PREPARATION 25

### 2-Butyl-4-propionylimidazole-5-carbonitrile

Following a procedure similar to that described in Preparation 24(i), but using ethylmagnesium bromide instead of methylmagnesium bromide, the title compound, melting at 84 - 85°C, was obtained in a 51.9% yield.

Nuclear Magnetic Resonance Spectrum (CDCl₃), δ ppm:
0.95 (3H, triplet, J = 7 Hz);
1.0-2.2(4H. multiplet);
1.28 (3H, triplet, J = 7.0 Hz);
2.88 (2H, triplet, J = 7 Hz);
3.15 (2H, quartet, J = 7 Hz).

### PREPARATION 26

### 2-Butyl-4-butyrylimidazole-5-carbonitrile

Following a procedure similar to that described in Preparation 24(i), but using propylmagnesium bromide instead of methylmagnesium bromide, the title compound, melting at 91 - 92°C, was obtained in a 57.2% yield.

Nuclear Magnetic Resonance Spectrum (CDCl₃), δ ppm:
1.02 (3H, triplet, J = 7.5 Hz);
1.11 (3H, triplet, J = 7.5 Hz);
1.3 - 1.6 (2H, multiplet);
1.7 - 2.0 (4H, multiplet);
2.88 (2H, triplet, J = 8 Hz);
3.13 (2H, triplet, J = 7.5 Hz).

### PREPARATION 27

### 2-Butyl-4-isobutyrylimidazole-5-carbonitrile

Following a procedure similar to that described in Preparation 24(i), but using isopropylmagnesium bromide instead of methylmagnesium bromide, the title compound, melting at 88 - 89°C, was obtained in a 36.2% yield.

Nuclear Magnetic Resonance Spectrum (CDCl₃), δ ppm:
0.94 (3H, triplet, J = 7 Hz);
1.0-2.1 (4H, multiplet);
1.30 (6H, doublet, J = 7 Hz);
2.91 (2H, triplet, J = 7 Hz);
3.71 (1H, septet, J = 7 Hz).

### PREPARATION 28

### 4-Butyryl-2-propylimidazole-5-carbonitrile

Following a procedure similar to that described in Preparation 24(i), but using 2-propylimidazole-4,5-dicarbonitrile (prepared as described in Preparation 10) and propylmagnesium bromide, the title compound, melting at 94 - 95°C, was obtained in a 49.8% yield.

Nuclear Magnetic Resonance Spectrum (CDCl₃), δ ppm:
1.00 (3H, triplet, J = 7.5 Hz);
1.04 (3H, triplet, J = 7.5 Hz);
1.7 - 1.9 (4H, multiplet);
2.79 (2H, triplet, J = 7.5 Hz);
3.06 (2H, triplet, J = 7.5 Hz).

### PREPARATION 29

### 2-Butyl-4-(1-hydroxy-1-methylethyl)imidazole-5-carbonitrile

Following a procedure similar to that described in Preparation 23(i), but using 4-acetyl-2-butylimidazole-5-carbonitrile [prepared as described in Preparation 24(i)] and a solution of methylmagnesium bromide in tetrahydrofuran, the title compound, melting at 171 - 172°C, was obtained in a 66.3% yield.

Nuclear Magnetic Resonance Spectrum (CDCl₃ + CD₃OD), δ ppm:
0.91 (3H, triplet, J = 7 Hz);
1.0 - 2.1 (4H, multiplet);
1.62 (6H, singlet);
2.69 (2H, triplet, J = 7 Hz).

### PREPARATION 30

### 2-Butyl-4-[1-hydroxy-2-methyl-1-(1-methylethyl)-propyl]imidazole-5-carbonitrile

Following a procedure similar to that described in Preparation 23(i), but using 2-butyl-4-isobutyryl-imidazole-5-carbonitrile (prepared as described in Preparation 27) and a solution of isopropylmagnesium bromide in tetrahydrofuran, the title compound, melting at 63 - 65°C, was obtained in a 87% yield.

Nuclear Magnetic Resonance Spectrum (CDCl₃), δ ppm:
0.7 - 1.0 (3H, multiplet);
0.87 (6H, doublet, J = 7 Hz);
0.91 (6H, doublet, J = 7 Hz);
1.0-2.1 (4H, multiplet);
2.0 - 2.9 (2H, multiplet);
2.71 (2H, triplet, J = 7 Hz).

### PREPARATION 31

### (5-Methyl-2-oxo-1,3-dioxolen-4-yl)methyl 4-(1-hydroxy-1-methylethyl)-2-propylimidazole-5-carboxylate

1.76 ml of N,N-diisopropylethylamine were added to a suspension of 1.06 g of 4-(1-hydroxy-1-methylethyl)-2-propylimidazole-5-carboxylic acid [prepared as described in Preparation 22(i)] in 10 ml of N,N-dimethylacetamide, and the resulting mixture was stirred at room temperature for 10 minutes, after which 1.12 g of (5-methyl-2-oxo-1,3-dioxolen-4-yl)methyl chloride was added, and the mixture was stirred at 60°C for 4 hours. At the end of this time, the reaction mixture was mixed with ethyl acetate and water. The ethyl acetate layer was separated and concentrated by evaporation under reduced pressure, and the concentrate was purified by column chromatography through silica gel, using a 1 : 15 by volume mixture of methanol and methylene chloride as the eluent, to give 1.14 g of the title compound as a viscous oil.

Nuclear Magnetic Resonance Spectrum (CDCl₃), δ ppm:
0.94 (3H, triplet, J = 7.5 Hz);
1.62 (6H, singlet);
1.6 - 1.8 (2H, multiplet);
2.19 (3H, singlet);
2.67 (2H, triplet, J = 8 Hz);
5.03 (2H, singlet).

### PREPARATION 32

### Phthalidyl 4-(1-hydroxy-1-methylethyl)-2-propylimidazole-5-carboxylate

Following a procedure similar to that described in Preparation 31, but using 1.06 g of 4-(1-hydroxy-1-methylethyl)-2-propylimidazole-5-carboxylic acid [prepared as described in Preparation 22(i)] and 1.15 g of 3-bromophthalide, 1.63 g of the title compound were obtained as an amorphous solid.

Nuclear Magnetic Resonance Spectrum (CDCl₃), δ ppm:
0.92 (3H, triplet, J = 7.5 Hz);
1.64 (6H, singlet);
1.6-1.75 (2H, multiplet);
2.63 (2H, triplet, J = 7.5 Hz);
7.63-7.79 (4H, multiplet);
7.91 (1H,doublet, J=8.5 Hz).

### PREPARATION 33

### Isopropoxycarbonyloxymethyl 4-(1-hydroxy-1-methylethyl)-2-propylimidazole-5-carboxylate

Following a procedure similar to that described in Preparation 22(ii), but using 1.06 g of 4-(1-hydroxy-1-methylethyl)-2-propylimidazole-5-carboxylic acid [prepared as described in Preparation 22(i)] and 0.83 g of isopropoxycarbonyloxymethyl chloride, 1.22 g of the title compound, melting at 144 - 146°C, were obtained.

Nuclear Magnetic Resonance Spectrum (CDCl₃), δ ppm:
0.98 (3H, triplet, J=7.5 Hz);
1.32 (6H, doublet, J = 6.5 Hz);
1.62 (6H, singlet);
1.76 (2H, sextet, J = 7.5 Hz);
2.69 (2H, triplet, J = 7.5 Hz);
4.93 (1H, quintet, J = 6.5 Hz);
5.95 (2H, singlet).

### PREPARATION 34

### 2-Ethylimidazole-4,5-dicarbonitrile

Following a procedure similar to that described in Preparation 1, but using 53.3 g of diaminomaleonitrile and 91.3 g of triethyl orthopropionate, 59.5 g of the title compound were obtained as crystals, melting at 179 - 181°C.

### PREPARATION 35

### 2 -Ethylimidazole -4,5 -dicarboxylic acid

Following a procedure similar to that described in Preparation 2, but using 45.0 g of 2-ethylimidazole-4,5-dicarbonitrile (prepared as described in Preparation 34), 31.2 g of the title compound were obtained as crystals, melting at 265 - 268°C.

### PREPARATION: 36

### Diethyl 2-ethylimidazole-4,5-dicarboxylate

Following a procedure similar to that described in Preparation 3, but using 35.0 g of 2-ethylimidazole-4,5-dicarboxylic acid (prepared as described in Preparation 35), 38.7 g of the title compound were obtained as crystals, melting at 83 - 84°C.

### PREPARATION 37

### Ethyl 2-ethyl-4-(1-hydroxy-1-methylethyl)imidazole-5-carboxylate

Following a procedure similar to that described in Preparation 8, but using 3.60 g of diethyl 2-ethyl-imidazole-4,5-dicarboxylate (prepared as described in Preparation 36) and 60 ml of a 1 M solution of methylmagnesium bromide in tetrahydrofuran, 2.05 g of the title compound were obtained as crystals, melting at 181 - 184°C.

Nuclear Magnetic Resonance Spectrum (hexadeuterated dimethyl sulphoxide) δ ppm:
1.22 (3H, triplet, J = 7 Hz);
1.33 (3H, triplet, J = 7.5 Hz);
1.50 (6H, singlet);
2.65 (2H, quartet, J = 7.5 Hz);
3.30 (1H, broad singlet);
4.31 (2H, quartet, J = 7.5 Hz).

### PREPARATION 38

### N-t-Butyl-4'-bromomethylbiphenyl-2-carboxamide

### 38 (i) N-t-Butyl-4'-methylbiphenyl-2-carboxamide

5.7 ml of oxalyl chloride were added dropwise, whilst ice-cooling, to a solution of 6.91 g of 4'-methylbiphenyl-2-carboxylic acid in 70 ml of methylene chloride, and the mixture was stirred at room temperature for 2 hours. The mixture was then concentrated by evaporation under reduced pressure, and the residue was dissolved in 70 ml of tetrahydrofuran. A solution of 7.5 ml of t-butylamine in 50 ml of tetrahydrofuran was added dropwise, whilst ice-cooling to the solution, and the mixture was stirred at room temperature for 10 minutes. At the end of this time, the reaction mixture was diluted with water and ethyl acetate. The ethyl acetate layer was separated, washed with aqueous sodium hydrogencarbonate and then with aqueous sodium chloride, dried over anhydrous sodium sulphate and concentrated by evaporation under reduced pressure, to give 7.48 g of the title compound as crystals, melting at 105 - 106.5°C (after recrystallization from ethyl acetate and hexane).

Nuclear Magnetic Resonance Spectrum (CDCl₃) δ ppm:
1.12 (9H, singlet);
2.41 (3H, singlet);
5.04 (1H, broad singlet);
7.2 - 7.5 (7H, multiplet);
7.71 (1H, doublet, J = 8 Hz).

### 38 (ii) N-t-Butyl-4'-bromomethylbiphenyl-2-carboxamide

4.39 g of N-bromosuccinimide and 50 mg of benzoyl peroxide were added to a solution of 6.00 g of N-t-butyl-4'-methylbiphenyl-2-carboxamide [prepared as described in Preparation 38(i)] in 90 ml of carbon tetrachloride, and the mixture was heated under reflux for 4 hours. At the end of this time, the reaction mixture was cooled to room temperature, washed with water, dried over anhydrous magnesium sulphate and concentrated by evaporation under reduced pressure. The residue was purified by column chromatography through silica gel, using a 1 : 4 by volume mixture of ethyl acetate and hexane as the eluent, to give 7.04 g of the title compound as crystals, melting at 124 - 126°C.

Nuclear Magnetic Resonance Spectrum (CDCl₃) δ ppm:
1.14 (9H, singlet);
4.55 (2H, singlet);
4.99 (1H, broad singlet);
7.30 - 7.72 (8H, multiplet).

### PREPARATION 39

### 4-Isobutyryl-2-propylimidazole-5-carbonitrile

Following a procedure similar to that described in Preparation 23(i), but using 8.24 g of 2-propyl-imidazole-4,5-dicarbonitrile (prepared as described in Preparation 10) and 103 ml of a 2 M solution of isopropylmagnesium iodide in diethyl ether, 45.0 g of the title compound were obtained as crystals, melting at 90.5 - 91°C.

Nuclear Magnetic Resonance Spectrum (CDCl₃) δ ppm:
1.01 (3H, triplet, J = 7.5 Hz);
1.29 (6H, doublet, J = 6.5 Hz);
1.82 (2H, sextet, J = 7.5 Hz);
2.81 (2H, triplet, J = 7.5 Hz);
3.66 (1H, septet, J = 6.5 Hz).

### PREPARATION 40

### 2-Butyl-4-pivaloylimidazole-5-carbonitrile

A solution of 10.4 g of 2-butylimidazole-4,5-dicarbonitrile (prepared as described in Preparation 1) in 150 ml of methylene chloride was added dropwise in an atmosphere of nitrogen at 10 - 15°C to 100 ml of a 2 M solution of t-butylmagnesium chloride in diethyl ether, and the mixture was stirred at the same temperature for 1 hour. 200 ml of ethyl acetate and 100 ml of aqueous potassium hydrogensulphate were then added dropwise to the reaction mixture, and the mixture was stirred at room temperature for 20 minutes. At end of this time, insoluble materials were removed by filtration, and the organic layer was separated, dried over anhydrous magnesium sulphate and concentrated by evaporation under reduced pressure. The residue was purified by column chromatography through silica gel, using a 1 : 3 by volume mixture of ethyl acetate and hexane as the eluent, to give 7.95 g of the title compound as crystals, melting at 135 - 137°C.

Nuclear Magnetic Resonance Spectrum (CDCl₃) δ ppm:
0.95 (3H, triplet, J = 7.5 Hz);
1.42 (2H, septet, J = 7.5 Hz);
1.46 (9H, singlet);
1.75 (2H, quintet, J = 7.5 Hz);
2.79 (2H, triplet, J = 7.5 Hz).

### PREPARATION 41

### 2-Propyl-4-pivaloylimidazole-5-carbonitrile

Following a procedure similar to that described in Preparation 40, but using 3.2 g of 2-propylimidazole-4,5-dicarbonitrile (prepared as described in Preparation 10) and 33 ml of a 2 M solution of t-butylmagnesium chloride in diethyl ether, 2.35 g of the title compound were obtained as crystals, melting at 176 - 178°C.

Nuclear Magnetic Resonance Spectrum (CDCl₃) δ ppm:
0.93 (3H, triplet, J = 7.5 Hz);
1.36 (9H, singlet);
1.75 (2H, sextet, J = 7.5 Hz);
2.68 (2H, triplet, J = 7.5 Hz).

### Preparation 42

### 1-[(2'-t-Butoxycarbonylbiphenyl-4-yl)methyl]-2-butyl-4-hydroxymethylimidazole-5-carboxylic acid

A solution of 2.01 g of lithium hydroxide monohydrate in 97 ml of water was added to a solution of 4.78 g of methyl 1-[(2'-t-butoxycarbonylbiphenyl-4-yl)-methyl]-2-butyl-4-hydroxymethylimidazole-5-carboxylate (prepared as described in Preparation 46) in 48 ml of dioxane, and the resulting mixture was stirred at room temperature for 18 hours. At the end of this time, the reaction mixture was freed from dioxane by distillation under reduced pressure, and 47.6 ml of 1 N aqueous hydrochloric acid were added to the aqueous residue. The crystals which precipitated were collected by filtration and then washed with water and with diethyl ether, in that order, to give 4.26 g of the title compound, melting at 187°C (with decomposition).

Nuclear Magnetic Resonance Spectrum (CDCl₃) δ ppm:
0.85 (3H, triplet, J = 7 Hz);
1.24 (9H, singlet);
1.1 - 1.9 (4H, multiplet);
2.80 (2H, triplet, J = 7 Hz);
5.05 (2H, singlet);
5.93 (2H, singlet);
7.0 - 7.85 (8H, multiplet).

### Preparation 43

### Pivaloyloxymethyl 1-[(2'-t-butoxycarbonylbiphenyl-4-yl)methyl]-2-butyl-4-hydroxymethylimidazole-5-carboxylate

350 mg of potassium carbonate were added to a solution of 552 mg of 1-[(2'-t-butoxycarbonylbiphenyl-4-yl) methyl]-2-butyl-4-hydroxymethylimidazole-5- carboxylic acid (prepared as described in Preparation 42) and 220 mg of pivaloyloxymethyl chloride in 7 ml of N,N-dimethylacetamide, and the resulting mixture was stirred at room temperature for 5 hours. At the end of this time, the reaction mixture was mixed with ethyl acetate and water, and the ethyl acetate layer was separated and dried over anhydrous magnesium sulphate; the solvent was then removed by distillation under reduced pressure. The resulting residue was purified by column chromatography through silica gel, using ethyl acetate as the eluent, to give 0.62 g of the title compound as a syrup.

Nuclear Magnetic Resonance Spectrum (CDCl₃) δ ppm:
0.91 (3H, triplet, J = 7 Hz);
1.18 (9H, singlet);
1.21 (9H, singlet);
1.1 - 2.0 (4H, multiplet);
2.72 (2H, triplet, J = 7 Hz);
3.35 (1H, broad);
4.85 (2H, doublet, J = 5 Hz);
5.61 (2H, singlet);
5.90 (2H, singlet);
6.95 - 7.9 (8H, multiplet).

### Preparation 44

### Pivaloyloxymethyl 2-butyl-1-[(2'-carboxybiphenyl-4-yl)methyl]-4-hydroxymethylimidazole-5-carboxylate

A solution of 0.62 g of pivaloyloxymethyl 1-[(2'-t-butoxycarbonylbiphenyl-4-yl)methyl]-2-butyl-4-hydroxymethylimidazole-5-carboxylate (prepared as described in Preparation 43) in 10 ml of a 4 N solution of hydrogen chloride in dioxane was allowed to stand at room temperature for 4 hours, after which it was concentrated by evaporation under reduced pressure. The syrupy residue was stirred in diethyl ether, and then the solvent was removed by decantation and the residue was dried in vacuo, to give 0.46 g of the hydrochloride of the title compound as a powder.

Nuclear Magnetic Resonance Spectrum (CDCl₃) δ ppm:
0.85 (3H, triplet, J = 7 Hz);
1.19 (9H, singlet);
1.25 - 1.45 (2H, multiplet);
1.65 - 1.80 (2H, multiplet);
2.99 (2H, triplet, J = 7 Hz);
5.01 (2H, singlet);
5.70 (2H, singlet);
5.89 (2H, singlet);
7.05 - 7.97 (8H, multiplet).

### Preparation 45

### 2-Butyl-1-[(2'-carboxybiphenyl-4-yl)methyl]-4-(1-hydroxyethyl)imidazol-5-carboxamide

### 45(a) 4-Acetyl-1-[(2'-t-butoxycarbonylbiphenyl-4-yl)-methyl]-2-butylimidazole-5-carbonitrile

0.192 g of sodium hydride (as a 55% w/w dispersion in mineral oil) was added to a solution of 0.843 g of 4-acetyl-2-butylimidazole-5-carbonitrile [prepared as described in Preparation 24(i)] in 17 ml of N,N-dimethylacetamide, and the resulting mixture was stirred at room temperature for 20 minutes. 1.68 g of t-butyl 4'-(bromomethyl)biphenyl-2-carboxylate was then added, and the resulting mixture was stirred at 55°C for 2.5 hours. At the end of this time, an aqueous solution of sodium chloride was added to the mixture, which was then extracted with ethyl acetate. The extract was washed with water and dried over anhydrous magnesium sulphate, and then the solvent was removed by distillation under reduced pressure. The resulting oily residue was purified by column chromatography through silica gel, using 4 : 1 and 2 : 1 by volume mixtures of hexane and ethyl acetate as the eluent, to afford 1.14 g of the title compound as a viscous oil.

Nuclear Magnetic Resonance Spectrum (CDCl₃) δ ppm:
0.93 (3H, triplet, J = 7 Hz);
1.23 (9H, singlet);
1.3 - 2.1 (4H, multiplet);
2.58 (3H, singlet);
2.75 (2H, triplet, J = 7 Hz);
5.32 (2H, singlet);
7.0 - 8.0 (8H, multiplet).

### 45(b) 1-[(2'-t-Butoxycarbonylbiphenyl-4-yl)methyl]-2-butyl-4-(1-hydroxyethyl)imidazole-5-carbonitrile

0.098 g of sodium borohydride was added to a solution of 1.18 g of 4-acetyl-1-[(2'-t-butoxycarbonyl-biphenyl-4-yl)methyl]-2-butylimidazole-5-carbonitrile [prepared as described in step' (a) above] in 30 ml of ethanol, and the resulting mixture was stirred at room temperature for 1 hour. The excess sodium borohydride was decomposed by adding acetone, and then the reaction mixture was concentrated by evaporation under reduced pressure. The residue was diluted with an aqueous solution of sodium chloride and extracted with ethyl acetate. The extract was dried and concentrated by evaporation under reduced pressure. The oily residue was purified by column chromatography through silica gel, using a 3 : 2 by volume mixture of ethyl acetate and hexane as the eluent, to afford 1.18 g of the title compound as a viscous oil.

Nuclear Magnetic Resonance Spectrum (CDCl₃) δ ppm:
0.92 (3H, triplet, J = 7.5 Hz);
1.25 (9H, singlet);
1.3 - 1.5 (2H, multiplet);
1.60 (3H, doublet, J = 6.5 Hz);
1.6 - 1.8 (2H, multiplet);
2.6 - 2.8 (2H, multiplet);
5.00 (1H, quartet, J = 6.5 Hz);
5.22 (2H, singlet);
7.1 - 7.9 (8H, multiplet).

### 45(c) 1-[(2'-t-Butoxycarbonylbiphenyl-4-yl)methyl]-2-butyl-4-(1-hydroxyethyl)imidazole-5-carboxamide

12 ml of a 1 N aqueous solution of sodium hydroxide were added to a. solution of 0.52 g of 1-[(2'-t-butoxycarbonylbiphenyl-4-yl)methyl]-2-butyl-4-(1-hydroxyethyl)-imidazole-5-carbonitrile [prepared as described in step (b) above] in 3 ml of ethanol, and the resulting mixture was heated under reflux for 3 hours. At the end of this time, the reaction mixture was neutralized by the addition of dilute aqueous hydrochloric acid and extracted with ethyl acetate. The extract was washed with water and dried over anhydrous magnesium sulphate. The solvent was then removed by distillation.under reduced pressure. The resulting residue was purified by column chromatography through silica gel, using a 4 : 1 by volume mixture of ethyl acetate and hexane, followed by ethyl acetate alone, as the eluent, to afford 0.14 g of the title compound as an amorphous solid.

Nuclear Magnetic Resonance Spectrum (CDCl₃) δ ppm:
0.90 (3H, triplet, J = 7.5 Hz);
1.23 (9H, singlet);
1.2 - 1.5 (2H, multiplet);
1.6 - 1.8 (2H, multiplet);
1.66 (3H, doublet, J = 6.5 Hz);
2.63 (2H, triplet, J = 8 Hz);
5.11 (1H, quartet, J = 6.5 Hz);
5.59 & 5.74 (each 1H, AB-quartet, J = 16 Hz);
7.0 - 7.9 (8H, multiplet).

### 45(d) 2-Butyl-1-[(2'-carboxybiphenyl-4-yl)methyl]-4-(1-hydroxyethyl)imidazole-5-carboxamide

A solution of 0.15 g of 1-[(2'-t-butoxycarbonylbiphenyl-4-yl)methyl]-2-butyl-4-(1-hydroxyethyl)-imidazole-5-carboxamide [prepared as described in step (c) above] dissolved in 3 ml of a 4 N solution of hydrogen chloride in dioxane was allowed to stand overnight at room temperature. The solution was then concentrated by evaporation under reduced pressure. The resulting residue was triturated in hexane and the powder thus obtained was collected by filtration, to afford 0.105 g of the hydrochloride of the title compound as an amorphous solid, melting at 212 - 214°C (with decomposition).

Nuclear Magnetic Resonance Spectrum (CDCl₃) δ ppm:
0.94 (3H, triplet, J = 7.5 Hz);
1.3 - 1.6 (2H, multiplet);
1.59 (3H, doublet, J = 6.5 Hz);
1.6 - 2.0 (2H, multiplet);
3.0 - 3.4 (2H, multiplet);
5.16 (1H, quartet, J = 6.5 Hz);
5.41 & 5.58 (each 1H, AB-quartet, J = 15 Hz);
7.1 - 7.9 (8H, multiplet).

### Preparation 46

### Methyl 1-[(2'-t-butoxycarbonylbiphenyl-4-yl)methyl]-2-butyl-4-hydroxymethylimidazole-5-carboxylate

### 46(a) Dimethyl 1-[(2'-t-butoxycarbonylbiphenyl-4-yl)-methyl]-2-butylimidazole-4,5-dicarboxylate

A sodium methoxide solution prepared from 0.69 g of sodium and 40 ml of methanol was added to a solution of 7.2 g of dimethyl 2-butylimidazole-4,5-dicarboxylate (prepared as described in Preparation 4) in 40 ml of methanol, and the resulting mixture was concentrated by evaporation under reduced pressure. The resulting residue was mixed with benzene, and the mixture was concentrated by distillation under reduced pressure. After this operation had been repeated three times, the solid thus obtained was dissolved in 72 ml of N,N-dimethylacetamide. A solution of 10.41 g of t-butyl 4'-bromomethylbiphenyl-2-carboxylate in 100 ml of N,N-dimethylacetamide was then added dropwise to the resulting solution. The reaction mixture was then stirred at room temperature for 1 hour and at 50 - 55°C for 2 hours. At the end of this time, it was mixed with ethyl acetate and water, and the ethyl acetate layer was separated, and dried over anhydrous magnesium sulphate; the solvent was then removed by distillation under reduced pressure. The residue was purified by column chromatography through silica gel, using a 1 : 1 by volume mixture of ethyl acetate and hexane as the eluent, to give 15.1 g of the title compound as a gum.

Nuclear Magnetic Resonance Spectrum (CDCl₃) δ ppm:
0.90 (3H, triplet, J = 7 Hz);
1.26 (9H, triplet);
1.1 - 2.0 (4H, multiplet);
2.70 (2H, triplet, J = 7 Hz);
3.81 (3H, singlet);
3.90 (3H, singlet);
5.47 (2H, singlet);
6.95 - 7.85 (8H, multiplet).

### 46(b) Methyl1-[(2'-t-butoxycarbonylbiphenyl-4-yl)-methyl]-2-butyl-4-hydroxymethylimidazole-5-carboxylate

42 ml of diisobutylaluminium hydride (as a 1.5 M solution in in toluene) were added dropwise at a temperature between -20°C and -15°C to a solution of 16.0 g of dimethyl 1-[(2'-t-butoxycarbonylbiphenyl-4- yl)methyl]-2-butylimidazole-4,5-dicarboxylate [prepared as described in step (a) above] in 200 ml of tetrahydrofuran, and the resulting mixture was allowed to stand at 0 - 5°C for 16 hours. At the end of this time, the reaction mixture was mixed with an aqueous solution of ammonium chloride and ethyl acetate and was then stirred for 1 hour. After this, precipitates were removed by filtration. The ethyl acetate layer was then separated and dried over anhydrous magnesium sulphate, and the solvent was removed by distillation under reduced pressure. The residue was then purified by column chromatography through silica gel, using ethyl acetate as the eluent, to give 12.0 g of the title compound as crystals, melting at 99°C.

Nuclear Magnetic Resonance Spectrum (CDCl₃) δ ppm:
0.90 (3H, triplet, J = 7 Hz);
1.20 (9H, singlet);
1.1 - 2.0 (4H, multiplet);
2.69 (2H, triplet, J = 7Hz);
3.55 (1H, broad singlet);
3.78 (3H, singlet);
4.84 (2H, doublet, J = 5 Hz);
5.60 (2H, singlet);
6.95 - 7.9 (8H, multiplet).

## Claims (Claims for the following Contracting State(s): GB, DE, FR, IT, CH, DK, BE, NL, SE, LU, AT, MC, PT)

1. A compound of formula (I): 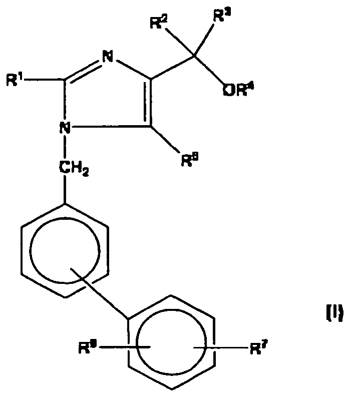 in which:
R¹ represents a propyl group;
R² and R³ each represent a methyl group;
R⁴ represents a hydrogen atom;
R⁵ represents a carboxy group;
R⁶ represents a hydrogen atom;
R⁷ represents a tetrazol-5-yl group at the 2- position of the benzene ring; and
the benzene ring which bears the substituents represented by R⁶ and R⁷ is at the 4-position of the benzyl group to which it is attached;
and pharmaceutically acceptable salts and esters thereof.

2. A compound according to Claim 1, which is an ester in which R⁵ is a group of formula -COOR^{5a}, where R^{5a} represents a (5-methyl-2-oxo-1,3-dioxolen-4-yl)methyl group.

3. A compound according to Claim 1, which is:
4-(1-hydroxy-1-methylethyl)-2-propyl-1-{4-[2-(tetrazol-5-yl)phenyl]phenyl}methylimidazole-5-carboxylic acid;
or a pharmaceutically acceptable salt thereof.

4. A compound according to Claim 1, which is:
(5-methyl-2-oxo-1,3-dioxolen-4-yl)methyl 4-(1-hydroxy-1-methylethyl)-2-propyl-1-{4-[2-(tetrazol-5-yl)phenyl]-phenyl}methylimidazole-5-carboxylate;
or a pharmaceutically acceptable salt thereof.

5. A pharmaceutical composition for the treatment or prophylaxis of hypertension, which comprises an antihypertensive agent in admixture with a pharmaceutically acceptable carrier or diluent, in which the antihypertensive agent is at least one compound of formula (I) or a pharmaceutically acceptable salt or ester thereof, as claimed in any one of Claims 1 to 4.

6. Compounds of formula (I) and pharmaceutically acceptable salts and esters thereof, as claimed in any one of Claims 1 to 4, for use in a method of therapy.

7. The use of compounds of formula (I) and pharmaceutically acceptable salts and esters thereof, as claimed in any one of Claims 1 to 4, for the manufacture of a medicament for the treatment or prophylaxis of hypertension.

8. A process for preparing a compound according to any one of Claims 1 to 4, which comprises the steps:
reacting a compound of formula (II): 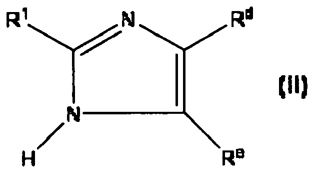 in which:
R¹ is as defined in Claim 1 and R^{d} represents a group of formula 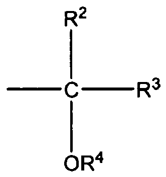 wherein R², R³ and R⁴ are as defined in Claim 1,
or R^{d} represents a group of formula -COOR^{f} wherein R^{f} represents a carboxy-protecting group, R^{d} represents a group of formula -COR², wherein R² is as defined above, or R^{d} represents a cyano group; and
R^{e} represents a cyano group, a carboxy group or a group of formula -COOR^{f}, wherein R^{f} is as defined above,
with a compound of formula (III): 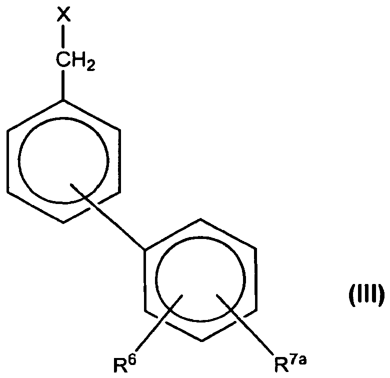 in which: R⁶ is as defined above; R^{7a} represents a protected carboxy group, a cyano group, a protected tetrazol-5-yl group, a carbamoyl group or an alkylcarbamoyl group; and X represents a halogen atom;
to give a compound of formula (IV): 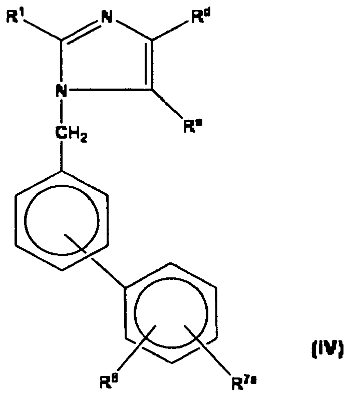
wherein R^{d}, R^{e}, R¹, R⁶ and R^{7a} are as defined above; and
in any order, removing protecting groups, and, if necessary, converting said group R^{d} to a group of formula 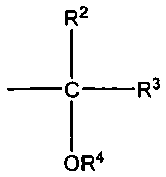 wherein R², R³ and R⁴ are as defined above,
and, if necessary, converting said group R^{e} to a group R⁵, or converting said group R^{7a} to a group R⁷, to give a compound of formula (I); and
optionally salifying or esterifying the product.

## Claims (Claims for the following Contracting State(s): ES, GR)

1. A process for preparing a compound of formula (I): 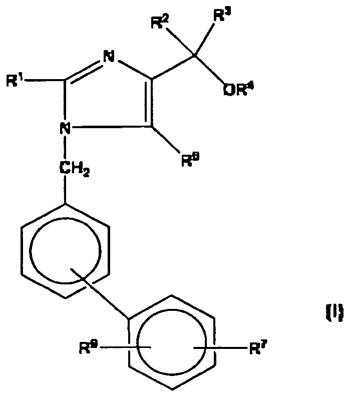 in which:
R¹ represents a propyl group;
R² and R³ each represent a methyl group;
R⁴ represents a hydrogen atom;
R⁵ represents a carboxy group;
R⁶ represents a hydrogen atom;
R⁷ represents a tetrazol-5-yl group at the 2- position of the benzene ring; and
the benzene ring which bears the substituents represented by R⁶ and R⁷ is at the 4-position of the benzyl group to which it is attached;
or a pharmaceutically acceptable salt or ester thereof,
which process comprises the steps:
reacting a compound of formula (II): 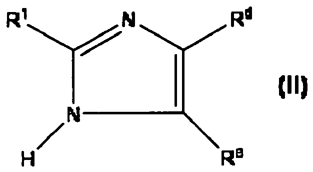 in which:
R¹ is as defined above and R^{d} represents a group of formula 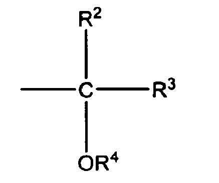 wherein R², R³ and R⁴ are as defined above,
or R^{d} represents a group of formula -COOR^{f} wherein R^{f} represents a carboxy-protecting group, R^{d} represents a group of formula -COR², wherein R² is as defined above, or R^{d} represents a cyano group; and
R^{e} represents a cyano group, a carboxy group or a group of formula -COOR^{f}, wherein R^{f} is as defined above,
with a compound of formula (III): 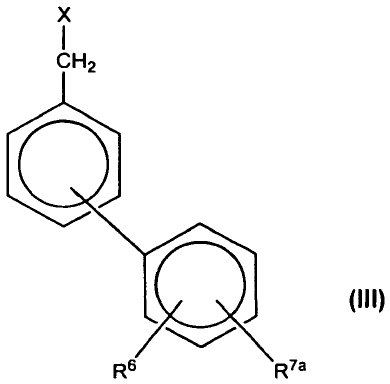 in which: R⁶ is as defined above; R^{7a} represents a protected carboxy group, a cyano group, a protected tetrazol-5-yl group, a carbamoyl group or an alkylcarbamoyl group; and X represents a halogen atom;
to give a compound of formula (IV): 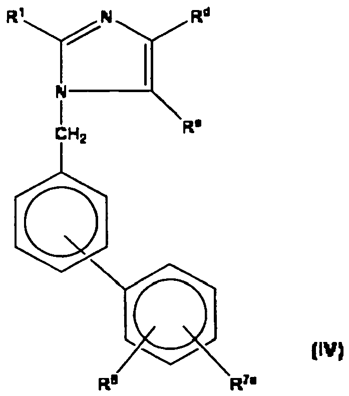 wherein R^{d}, R^{e}, R¹, R⁶ and R^{7a} are as defined above; and
in any order, removing protecting groups, and, if necessary, converting said group R^{d} to a group of formula 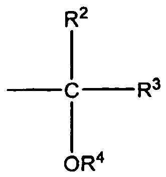 wherein R², R³ and R⁴ are as defined above,
and, if necessary, converting said group R^{e} to a group R⁵, or converting said group R^{7a} to a group R⁷, to give a compound of formula (I); and
optionally salifying or esterifying the product.

2. A process according to Claim 1, in which the reagents and conditions are so chosen as to prepare an ester of said compound of formula (I), in which R⁵ is a group of formula -COOR^{5a}, where R^{5a} represents a (5-methyl-2-oxo-1,3-dioxolen-4-yl)methyl group.

3. A process according to Claim 1, in which the reagents and conditions are so chosen as to prepare:
4-(1-hydroxy-1-methylethyl)-2-propyl-1-{4-[2-(tetrazol-5-yl)phenyl]phenyl}methylimidazole-5-carboxylic acid;
or a pharmaceutically acceptable salt thereof.

4. A process according to Claim 1, in which the reagents and conditions are so chosen as to prepare:
(5-methyl-2-oxo-1,3-dioxolen-4-yl)methyl 4-(1-hydroxy-1-methylethyl)-2-propyl-1-{4-[2-(tetrazol-5-yl)phenyl]-phenyl}methylimidazole-5-carboxylate;
or a pharmaceutically acceptable salt thereof.

5. A process for preparing a pharmaceutical composition for the treatment or prophylaxis of hypertension, which comprises mixing an anti-hypertensive agent with a pharmaceutically acceptable carrier or diluent, in which the anti-hypertensive agent is at least one compound of formula (I) or a pharmaceutically acceptable salt or ester thereof, as defined in any one of Claims 1 to 4.

6. The use of compounds of formula (I) and pharmaceutically acceptable salts and esters thereof, as defined in any one of Claims 1 to 4, for the manufacture of a medicament for the treatment or prophylaxis of hypertension.

## Patentansprüche (Patentansprüche für folgende(n) Vertragsstaat(en): GB, DE, FR, IT, CH, DK, BE, NL, SE, LU, AT, MC, PT)

1. Verbindung der Formel (I): 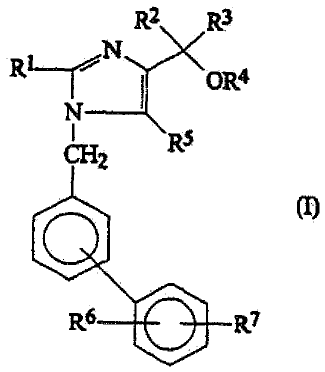 in der
R¹ eine Propylgruppe darstellt;
R² und R³ jeweils eine Methylgruppe darstellen;
R⁴ ein Wasserstoffatom darstellt;
R⁵ eine Carboxygruppe darstellt;
R⁶ ein Wasserstoffatom darstellt;
R⁷ eine Tetrazol-5-yl-Gruppe in 2-Stellung des Benzolrings darstellt, und
der Benzolring, welcher die durch R⁶ und R⁷ verkörperten Substituenten trägt, in 4-Stellung der Benzylgruppe ist, an die er gebunden ist,
und pharmazeutisch geeignete Salze und Ester dieser Verbindung.

2. Verbindung nach Anspruch 1, die ein Ester ist, in welchem
R⁵ eine Gruppe der Formel -COOR^{5a} ist, wobei R^{5a} eine (5-Methyl-2-oxo-1,3-dioxolen-4-yl)methylgruppe darstellt.

3. Verbindung nach Anspruch 1, die Folgendes ist:
4-(1-Hydroxy-1-methylethyl)-2-propyl-1-{4-[2-(tetrazol-5-yl)phenyl]phenyl} methylimidazol-5-carbonsäure;
oder ein pharmazeutisch geeignetes Salz davon.

4. Verbindung nach Anspruch 1, die Folgendes ist:
(5-Methyl-2-oxo-1,3-dioxolan-4-yl)-methyl-4-(1-hydroxy-1-methylethyl)-2-propyl-1-{4-[2-(tetrazol-5-yl)phenyl]phenyl}methylimidazol-5-carboxylat;
oder ein pharmazeutisch geeignetes Salz davon.

5. Arzneimittelzusammensetzung zur Behandlung oder Prophylaxe von Bluthochdruck, die ein antihypertensives Mittel im Gemisch mit einem pharmazeutisch geeigneten Träger oder Verdünnungsmittel enthält, wobei das antihypertensive Mittel mindestens eine Verbindung der Formel (I) oder ein pharmazeutisch geeignetes Salz oder Ester davon gemäß einem der Ansprüche 1 bis 4 ist.

6. Verbindungen der Formel (I) und pharmazeutisch geeignete Salze und Ester davon gemäß einem der Ansprüche 1 bis 4 zur Verwendung in einer therapeutischen Methode.

7. Verwendung von Verbindungen der Formel (I) und pharmazeutisch geeigneter Salze und Ester davon gemäß einem der Ansprüche 1 bis 4 zur Herstellung eines Arzneimittels zur Behandlung oder Prophylaxe von Bluthochdruck.

8. Verfahren zur Herstellung einer Verbindung nach einem der Ansprüche 1 bis 4, welches folgende Stufen umfaßt:
Umsetzen einer Verbindung der Formel (II): 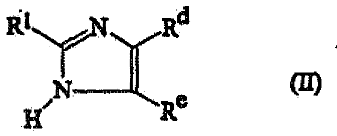 in der:
R¹ wie in Anspruch 1 definiert ist und R^{d} eine Gruppe der Formel 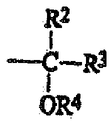
darstellt, worin R², R³ und R⁴ wie in Anspruch 1 definiert sind,
oder R^{d} eine Gruppe der Formel -COOR^{f} darstellt, worin R^{f} eine CarboxySchutzgruppe darstellt, R^{d} eine Gruppe der Formel -COR² darstellt, worin R² wie vorstehend definiert ist oder R^{d} eine Cyangruppe darstellt; und
R^{e} eine Cyangruppe, eine Carboxygruppe oder eine Gruppe der Formel -COOR^{f} darstellt, worin R^{f} wie vorstehend definiert ist,
mit einer Verbindung der Formel (III): 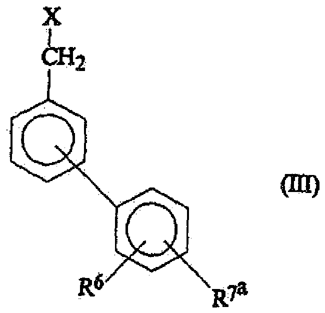 worin R⁶ wie vorstehend definiert ist, R^{7a} eine geschützte Carboxygruppe, eine Cyangruppe, eine geschützte Tetrazol-5-yl-Gruppe, eine Carbamoylgruppe oder eine Alkylcarbamoylgruppe darstellt und X ein Halogenatom bedeutet,
unter Bildung einer Verbindung der Formel (IV) : 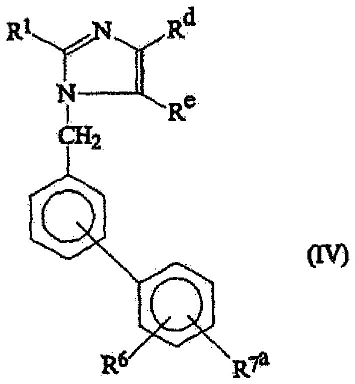
worin R^{d}, R^{e}, R¹, R⁶ und R^{7a} wie vorstehend definiert sind, und
in beliebiger Reihenfolge, Entfernen der Schutzgruppen und erforderlichenfalls die Umwandlung der Gruppe R^{d} in eine Gruppe der Formel 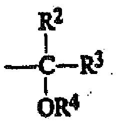
worin R², R³ und R⁴ wie vorstehend definiert sind,
und erforderlichenfalls Umwandeln der Gruppe R^{e} in eine Gruppe R⁵ oder Umwandeln der Gruppe R^{7a} in eine Gruppe R⁷ unter Bildung einer Verbindung der Formel (I), und
gegebenenfalls Salzbildung oder Verestern des Produkts.

## Patentansprüche (Patentansprüche für folgende(n) Vertragsstaat(en): GR, ES)

1. Verfahren zur Herstellung einer Verbindung der Formel (I): 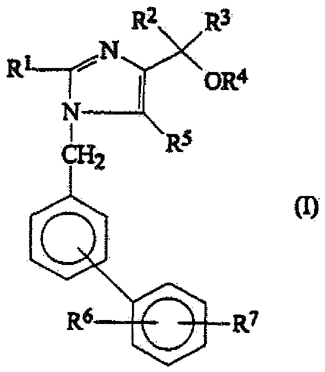 in der
R¹ eine Propylgruppe darstellt;
R² und R³ jeweils eine Methylgruppe darstellen;
R⁴ ein Wasserstoffatom darstellt;
R⁵ eine Carboxygruppe darstellt;
R⁶ ein Wasserstoffatom darstellt;
R⁷ eine Tetrazol-5-yl-Gruppe in 2-Stellung des Benzolrings darstellt; und der Benzolring, welcher die durch R⁶ und R⁷ verkörperten Substituenten trägt, in 4-Stellung der Benzylgruppe ist, an die er gebunden ist;
oder eines pharmazeutisch geeigneten Salzes oder Esters dieser Verbindung, welches folgende Stufen umfaßt:
Umsetzen einer Verbindung der Formel (II) : 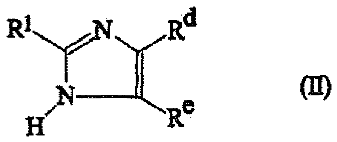
in der:
R¹ wie vorstehend definiert ist und R^{d} eine Gruppe der Formel 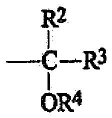
darstellt, worin R², R³ und R⁴ wie vorstehend definiert sind,
oder R^{d} eine Gruppe der Formel -COOR^{f} darstellt, worin R^{f} eine CarboxySchutzgruppe darstellt, R^{d} eine Gruppe der Formel -COR² darstellt, worin R² wie vorstehend definiert ist oder R^{d} eine Cyangruppe darstellt; und
R^{e} eine Cyangruppe, eine Carboxygruppe oder eine Gruppe der Formel -COOR^{f} darstellt, worin R^{f} wie vorstehend definiert ist,
mit einer Verbindung der Formel (III) 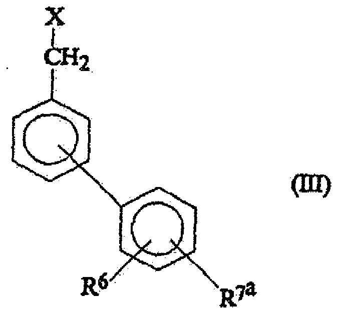
worin R⁶ wie vorstehend definiert ist, R^{7a} eine geschützte Carboxygruppe, eine Cyangruppe, eine geschützte Tetrazol-5-yl-Gruppe, eine Carbamoylgruppe oder eine Alkylcarbamoylgruppe darstellt und X ein Halogenatom darstellt,
unter Bildung einer Verbindung der Formel (IV): 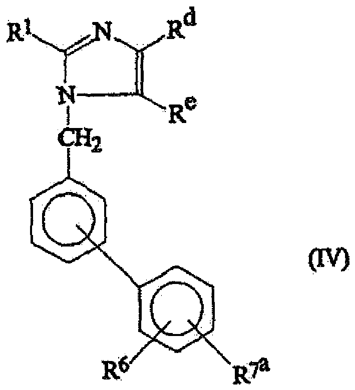
worin R^{d}, R^{e}, R¹, R⁶ und R^{7a} wie vorstehend definiert sind, und
in beliebiger Reihenfolge, Entfernen der Schutzgruppen und erforderlichenfalls die Umwandlung der Gruppe R^{d} in eine Gruppe der Formel 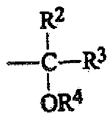 worin R², R³ und R⁴ wie vorstehend definiert sind,
und erforderlichenfalls Umwandeln der Gruppe R^{e} in eine Gruppe R⁵ oder Umwandeln der Gruppe R^{7a} in eine Gruppe R⁷ unter Bildung einer Verbindung der Formel (I), und
gegebenenfalls Salzbildung oder Verestern des Produkts.

2. Verfahren nach Anspruch 1, bei dem die Reagenzien und Reaktionsbedingungen so gewählt werden, dass ein Ester der Verbindung der Formel (I) gebildet wird, worin R⁵ eine Gruppe der Formel -COOR^{5a} ist, wobei R^{5a} eine (5-Methyl-2-oxo-1,3-dioxolen-4-yl)methylgruppe darstellt.

3. Verfahren nach Anspruch 1, bei dem die Reagenzien und Reaktionsbedingungen so gewählt werden, dass hergestellt wird:
4-(1-Hydroxy-1-methylethyl)-2-propyl-1-{4-[2-(tetrazol-5-yl)phenyl]phenyl}-methylimidazol-5-carbonsäure;
oder ein pharmazeutisch geeignetes Salz davon.

4. Verfahren nach Anspruch 1, bei dem die Reagenzien und Reaktionsbedingungen so gewählt werden, dass hergestellt wird:
(5-Methyl-2-oxo-1,3-dioxolen-4-yl)-methyl-4-(1-hydroxy-1-methylethyl)-2-propyl-1-{4-[2-(tetrazol-5-yl)phenyl]phenyl}methylimidazol-5-carboxylat;
oder ein pharmazeutisch geeignetes Salz davon.

5. Verfahren zur Herstellung einer Arzneimittelzusammensetzung zur Behandlung oder Prophylaxe von Bluthochdruck, welches das Vermischen eines antihypertensiven Mittels mit einem pharmazeutisch geeigneten Träger oder Verdünnungsmittel umfaßt, wobei das antihypertensive Mittel mindestens eine Verbindung der Formel (I) oder ein pharmazeutisch geeignetes Salz oder Ester davon gemäß der Definition in einem der Ansprüche 1 bis 4 ist.

6. Verwendung von Verbindungen der Formel (I) und pharmazeutisch geeigneter Salze und Ester davon gemäß der Definition in einem der Ansprüche 1 bis 4 zur Herstellung eines Arzneimittels zur Behandlung oder Prophylaxe von Bluthochdruck.

## Revendications (Revendications pour l'(les) Etat(s) contractant(s) suivant(s): GB, DE, FR, IT, CH, DK, BE, NL, SE, LU, AT, MC, PT)

1. Composé de formule (I): 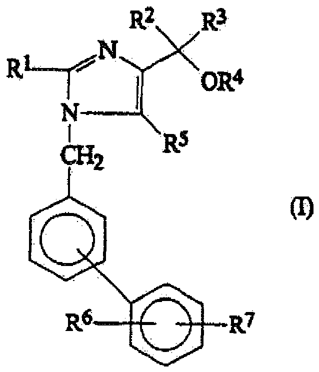 dans laquelle
R¹ représente un groupe propyle;
R² et R³ représentent chacun un groupe méthyle;
R⁴ représente un atome d'hydrogène;
R⁵ représente un groupe carboxy;
R⁶ représente un atome d'hydrogéne;
R⁷ représente un groupe tétrazol-5-yle en position 2 du cycle benzène; et
le cycle benzène, qui porte les substituants représentés par R⁶ et R⁷ est en position 4 du groupe benzyle auquel il est rattaché;
et ses sels et esters acceptables en pharmacie.

2. Composé selon la revendication 1, qui est un ester dans lequel R⁵ est un groupe de formule -COOR^{5a}, où :
R^{5a} représente un groupe (5-méthyl-2-oxo-1,3-dioxolén-4-yl)méthyle.

3. Composé selon la revendication 1, qui est:
acide 4-(1-hydroxy-1-méthyléthyl)-2-propyl-1-{4-[2-(tétrazol-5-yl)phényl]phényl}méthylimidazole-5-carboxylique;
ou un sel acceptable en pharmacie de celui-ci.

4. Composé selon la revendication 1, qui est:
4-(1-hydroxy-1-méthyléthyl)-2-propyl-1-{4-[2-(tétrazol-5-yl)phényl]phényl}-méthylimidazole-5-carboxylate de (5-méthyl-2-oxo-1,3-dioxolén-4-yl)méthyle;
ou un sel acceptable en pharmacie de celui-ci.

5. Composition pharmaceutique pour le traitement ou la prophylaxie de l'hypertension, qui comprend un agent antihypertenseur en mélange avec un véhicule ou diluant acceptable en pharmacie, dans laquelle l'agent antihypertenseur est au moins un composé de formule (I) ou un de ses sels ou esters acceptables en pharmacie, tel que revendiqué dans l'une quelconque des revendications 1 à 4.

6. Composés de formule (I) et leurs sels et esters acceptables en pharmacie, tels que revendiqués dans l'une quelconque des revendications 1 à 4, à utiliser dans une méthode de traitement.

7. Utilisation des composés de formule (I) et de leurs sels et esters acceptables en pharmacie, tels que revendiqués dans l'une quelconque des revendications 1 à 4, pour la fabrication d'un médicament destiné au traitement ou à la prophylaxie de l'hypertension.

8. Procédé pour préparer un composé selon l'une quelconque des revendications 1 à 4, qui comprend les étapes consistant à:
faire réagir un composé de formule (II): 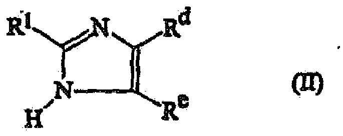 dans laquelle:
R¹ est tel que défini dans la revendication 1 et R^{d} représente un groupe de formule : 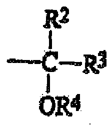
dans laquelle R², R³ et R⁴ sont tels que définis dans la revendication 1,
ou bien R^{d} représente un groupe de formule -COOR^{f}, dans laquelle R^{f} représente un groupe carboxy-protecteur, R^{d} représente un groupe de formule -COR², où R² est tel que défini ci-dessus, ou bien R^{d} représente un groupe cyano; et
R^{e} représente une groupe cyano, un groupe carboxy ou un groupe de formule -COOR^{f}, dans laquelle R^{f} est tel que défini ci-dessus,
avec un composé de formule (III): 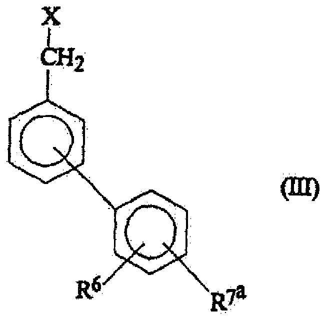 dans laquelle: R⁶ est tel que défini ci-dessus ; R^{7a} représente un groupe carboxy protégé, un groupe cyano, un groupe tétrazol-5-yle protégé, un groupe carbamoyle ou un groupe alkylcarbamoyle; et X représente un atome d'halogène;
pour donner un composé de formule (IV) : 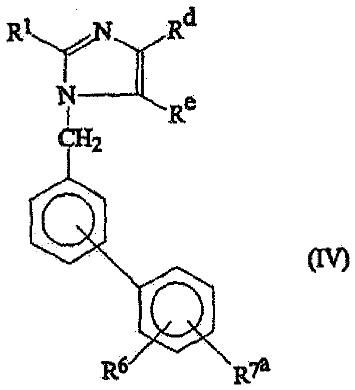 dans laquelle R^{d}, R^{e}, R¹, R⁶ et R^{7a} sont tels que définis ci-dessus, et
dans un ordre quelconque, éliminer les groupes protecteurs et, si nécessaire, convertir ledit groupe R^{d} en un groupe de formule 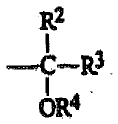 dans laquelle R², R³ et R⁴ sont tels que définis ci-dessus, et, si nécessaire, convertir ledit groupe R^{e} en un groupe R⁵, ou convertir ledit groupe R^{7a} en un groupe R⁷ pour donner un composé de formule (I), et
éventuellement, salifier ou estérifier le produit.

## Revendications (Revendications pour l'(les) Etat(s) contractant(s) suivant(s): GR, ES)

1. Procédé pour préparer un composé de formule (I): 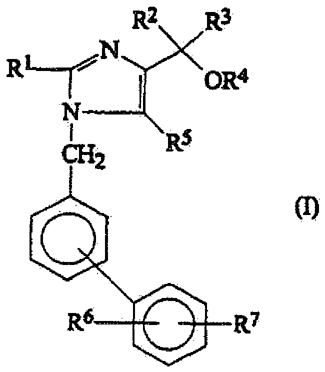 dans laquelle :
R¹ représente un groupe propyle;
R² et R³ représentent chacun un groupe méthyle;
R⁴ représente un atome d'hydrogène;
R⁵ représente un groupe carboxy;
R⁶ représente un atome d'hydrogène;
R⁷ représente un groupe tétrazol-5-yle en position 2 du cycle benzène; et
le cycle benzène qui porte les substituants représentés par R⁶ et R⁷ est en position 4 du groupe benzyle auquel il est rattaché;
ou de ses sels ou esters acceptables en pharmacie;
lequel procédé comprend les étapes consistant à :
faire réagir un composé de formule (II) : 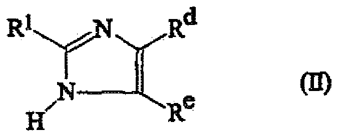 dans laquelle:
R¹ est tel que défini ci-dessus et R^{d} représente un groupe de formule : 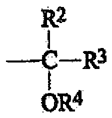 dans laquelle R², R³ et R⁴ sont tels que définis ci-dessus,
ou bien R^{d} représente un groupe de formule -COOR^{f}, dans laquelle R^{f} représente un groupe carboxy-protecteur, R^{d} représente un groupe de formule -COR², où R² est tel que défini ci-dessus, ou bien R^{d} représente un groupe cyano; et
R^{e} représente un groupe cyano, un groupe carboxy ou un groupe de formule -COOR^{f}, dans laquelle R^{f} est tel que défini ci-dessus,
avec un composé de formule (III) : 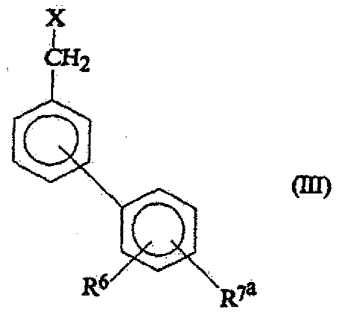 dans laquelle : R⁶ est tel que défini ci-dessus ; R^{7a} représente un groupe carboxy protégé, un groupe cyano, un groupe tétrazol-5-yle protégé, un groupe carbamoyle ou un groupe alkylcarbamoyle; et X représente un atome d'halogène;
pour donner un composé de formule (IV) : 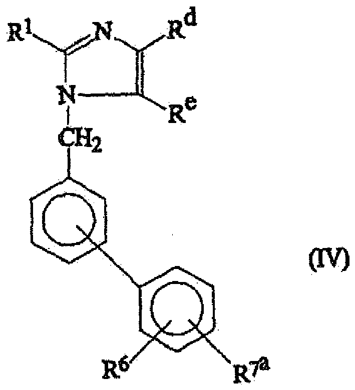 dans laquelle R^{d}, R^{e}, R¹, R⁶ et R^{7a} sont tels que définis ci-dessus ; et
dans un ordre quelconque, éliminer les groupes protecteurs et, si nécessaire, convertir ledit groupe R^{d} en un groupe de formule : 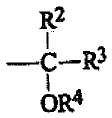 dans laquelle R², R³ et R⁴ sont tels que définis ci-dessus,
et, si nécessaire, convertir ledit groupe R^{e} en un groupe R⁵, ou convertir ledit groupe R^{7a} en un groupe R⁷, pour donner un composé de formule (I) ; et
éventuellement, salifier ou estérifier le produit.

2. Procédé selon la revendication 1, dans lequel les réactifs et les conditions réactionnelles sont choisis de façon à préparer un ester dudit composé de formule (I), dans lequel :
R⁵ est un groupe de formule -COOR^{5a}, où :
R^{5a} représente un groupe (5-méthyl-2-oxa-1,3-dioxolén-4-yl)méthyle.

3. Procédé selon la revendication 1, dans lequel les réactifs et les conditions réactionnelles sont choisis de façon à préparer:
acide 4-(1-hydroxy-1-méthyléthyl)-2-pxopyl-1-{4-[2-(tétrazol-5-yl)phényl]phényl}méthylimidazole-5-carboxylique;
ou un sel acceptable en pharmacie de celui-ci.

4. Procédé selon la revendication 1, dans lequel les réactifs et les conditions réactionnelles sont choisis de façon à préparer:
4-(1-hydroxy-1-méthyléthyl)-2-propyl-1-{4-[2-(tétrazol-5-yl)phényl]phényl}-méthylimidazole-5-carboxylate de (5-méthyl-2-oxo-1,3-dioxolén-4-yl)méthyle;
ou un sel acceptable en pharmacie de celui-ci.

5. Procédé pour préparer une composition pharmaceutique pour le traitement ou la prophylaxie de l'hypertension, qui comprend le mélange d'un agent antihypertenseur avec un véhicule ou diluant acceptable en pharmacie, dans laquelle l'agent antihypertenseur est au moins un composé de formule (I) ou un de ses sels ou esters acceptables en pharmacie, tel que revendiqué dans l'une quelconque des revendications 1 à 4.

6. Utilisation de composés de formule (I) et de leurs sels et esters acceptables en pharmacie, tels que revendiqués dans l'une quelconque des revendications 1 à 4, pour la fabrication d'un médicament destiné au traitement ou à la prophylaxie de l'hypertension.
